# EUROPEAN PATENT SPECIFICATION

(11) **EP 0 681 575 B1**
(45) Date of publication and mention of the grant of the patent: **22.05.2002**
(21) Application number: 94909517.8
(22) Date of filing: 31.01.1994
(51) Int. Cl.: C07D 305/14, C07D 317/70, C07D 493/10

(54) **Process for the preparation of Baccatin III analogs bearing new C2 and C4 functional groups**
Verfahren zur Herstell ung von Baccatin III-Analogen, Verbindungen mit neuen funktionellen Gruppen an C2 und C4
Procédé de préparation d'analogues de Baccatine III portant de nouveaux groupes fonctionnels C2 et C4

(30) Priority: 29.01.1993 US 10798; 22.03.1993 US 34852; 20.07.1993 US 94715
(43) Date of publication of application: 15.11.1995
(73) Proprietor: FLORIDA STATE UNIVERSITY, Tallahassee, Florida 32310 (US)
(72) Inventor: HOLTON, Robert A., 2035 East Paul Dirac Drive, Tallahassee, FL 32310 (US); KIM, Seocchan, 2035 East Paul Dirac Drive, Tallahassee, FL 32310 (US); SUZUKI, Yukio, 2035 East Paul Dirac Drive, Tallahassee, FL 32310 (US)
(74) Representative: Eyles, Christopher Thomas
(86) International application number: US9401099
(87) International publication number: WO9417051

(56) References cited:
- TETRAHEDRON, (INCL. TETRAHEDRON REPORTS), vol.48, no.34, 1992, OXFORD GB pages 6965 - 6973 A. WAHL ET AL. 'REARRANGEMENT REACTIONSOF TAXANES:STRUCTURAL MODIFICATIONS OF 10-DESACETYLBACCATIN III.'
- Tetrahedron Letters, Volume 34, No. 13, issued 1993, LARRY L. KLEIN: "Synthesis of 9-Dihydrotaxol: A Novel Bioactive Taxane", pages 2047-2050.

## Description

### BACKGROUND OF THE INVENTION

The present invention relates to a process for the preparation of baccatin III and 10-desacetylbaccatin III analogs having new C2 and/or C4 functional groups.

Taxol is a natural product extracted from the bark of yew trees. It has been shown to have excellent antitumor activity in in vivo animal models, and recent studies have elucidated its unique mode of action, which involves abnormal polymerization of tubulin and disruption of mitosis. It is currently undergoing clinical trials against ovarian, breast and other types of cancer in the United States and France and preliminary results have confirmed it as a most promising chemotherapeutic agent. The structure of taxol and the numbering system conventionally used is shown below; this numbering system is also applicable to compounds used in the process of the present invention.

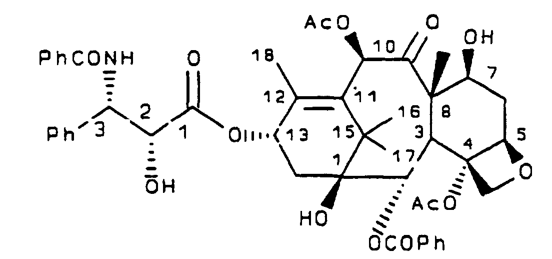

In Colin U.S. Patent No. 4,814,470, it was reported that a taxol derivative, commonly referred to as taxotere, has an activity significantly greater than taxol. Taxotere has the following structure:

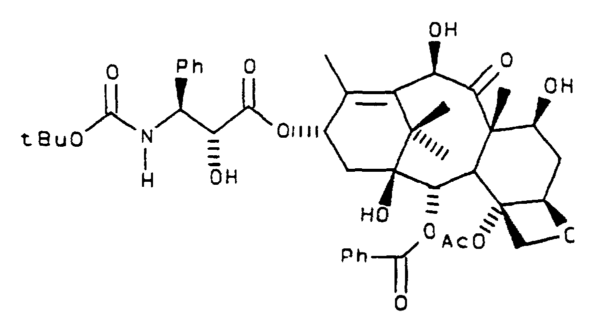

Taxol, taxotere and other biologically active tetracyclic taxanes may be prepared semisynthetically from baccatin III and 10-desacetyl baccatin III as set forth in U.S. Patent Nos. 4,924,011 and 4,924,012 or by the reaction of a β-lactam and a suitably protected baccatin III or 10-desacetylbaccatin III ("10-DAB") derivative as set forth in U. S. Patent No. 5,175,315 or copending U.S. Patent Application Serial No. 07/949,107. Baccatin III **1** and 10-DAB **2** can be separated from mixtures extracted from natural sources such as the needles, stems, bark or heartwood of numerous Taxus species and have the following structures.

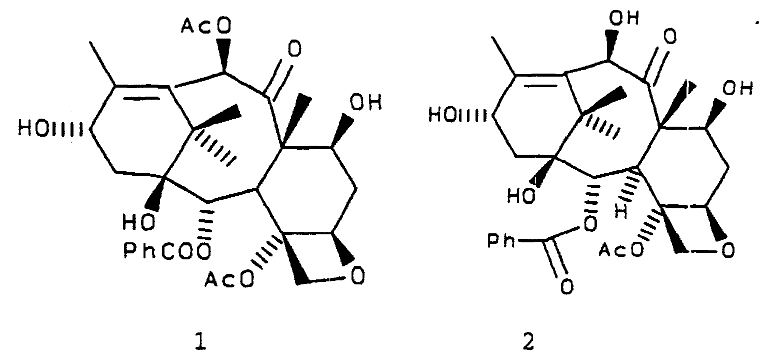

The tetracyclic core of taxol and taxotere bear six singly bonded oxygen substituents. Two of these (three in the case of taxotere) are present as hydroxyl groups, and the others are esters of three different carboxylic acids. Selective manipulation of these groups presents a formidable problem which must be overcome before, a series of taxol analogs can be prepared by a rational synthetic sequence. Hydrolytic and solvolytic methods have previously encountered complications. For example, it has been reported that hydrolysis of taxol under mildly basic conditions yields a complex mixture of products. Miller et al., J. Org. Chem. 1981, 46, 1469. Recently it has been found that solvolysis of baccatin (III) derivatives leads to rearrangement of the tetracyclic core. Farina, et al., Tetrahedron Lett. 1992, 33, 3979.

The compound 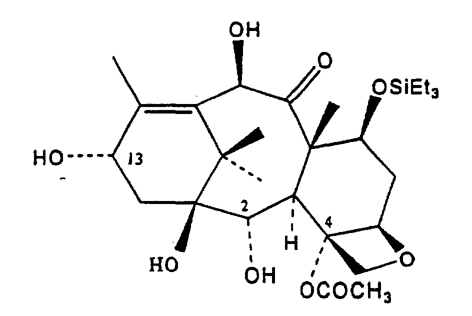 is disclosed in the literature. A. Wahl, et al., Tetrahedron **1992**, Vol. 48, No. 34, 6965.

### SUMMARY OF THE INVENTION

Among the objects of the present invention, therefore, is the provision of a process for selectively attaching different functional groups to the C2 and/or C4 oxygens of baccatin III and analogs or derivatives thereof; the provision of such a process which is relatively straightforward; the provision of such a process in which the C2 benzoate substituent of baccatin III and analogs or derivatives thereof may be selectively reduced or hydrolyzed and the provision of such a process in which the C4 acetate substituent may be selectively reduced.

Briefly, therefore, the present invention is directed to a process for the preparation of analogs or derivatives of baccatin III or 10-desacetyl baccatin III in which the C2 substituent and/or the C4 acetate substituent of baccatin III or 10-desacetoxy baccatin III or an analog thereof is selectively converted to the corresponding hydroxy group(s).

According to the present invention there is provided a process for the preparation of a derivative or analog of baccatin III or 10-desacetyl baccatin III having a C2 substituent other than benzoate and/or a C4 substituent other than acetate from a derivative or analog of baccatin III of 10-desacetyl baccatin III which comprises a C2 benzoate and/or C4 acetate substituent, the process comprising selectively reducing the C2 benzoate substituent and/or the C4 acetate substituent with an aluminate to the corresponding hydroxy group(s) in a non-aqueous system and acylating the C2 hydroxy substituent and/or the C4 hydroxy substituent to convert the C2 hydroxy substituent to R₃₁COO- and/or convert the C4 hydroxy substituent to R₃₀COO- wherein R₃₁ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

In such a process the derivative or analog of baccatin III or 10-desacetyl derivative may have the formula 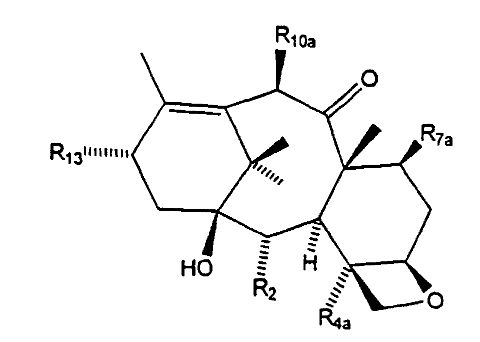 wherein
R₁₀ₐ is H, -OH, protected hydroxy, or -OCOR₂₉ₐ;
R₁₃ is -OH or protected hydroxy;
R₇ₐ is H, -OH or protected hydroxy;
R₂ is -OCOR₃₁;
R₄ₐ is -OCOR₃₀; and
R₂₉ₐ, R₃₀ and R₃₁ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

The C2 benzoate substituent and/or the C4 acetate substituent of the derivative of baccatin III or 10-desacetyl baccatin III may be selectively reduced with lithium aluminum hydride or sodium bis (2-methoxyethoxy) aluminum hydride.

In a preferred process the derivative or analog of baccatin III or 10-desacetyl derivative has the formula 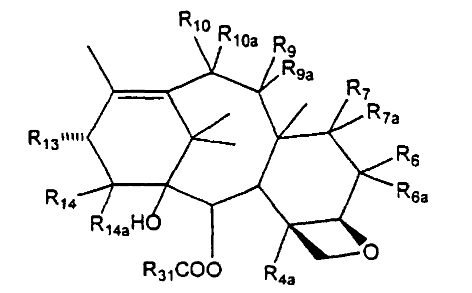 wherein
R₄ₐ is hydrogen, optionally substituted alkyl, optionally substituted alkenyl, optionally substituted alkynyl, optionally substituted aryl, optionally substituted heteroaryl, cyano, hydroxy, or -OCOR₃₀;
R₆ is hydrogen, optionally substituted alkyl, optionally substituted alkenyl, optionally substituted alkynyl, optionally substituted aryl, or optionally substituted heteroaryl, hydroxy, protected hydroxy or together with R₆ₐ forms an oxo;
R₆ₐ is hydrogen, optionally substituted alkyl, optionally substituted alkenyl, optionally substituted alkynyl, optionally substituted aryl, or optionally substituted heteroaryl, hydroxy, protected hydroxy or together with R₆ forms an oxo;
R₇ is hydrogen or together with R₇ₐ forms an oxo;
R₇ₐ is hydrogen, halogen, protected hydroxy, -OR₂₈, or together with R, forms an oxo;
R₉ is hydrogen or together with R₉ₐ forms an oxo;
R₉ₐ is hydrogen, hydroxy, protected hydroxy, or together with R₉ forms an oxo;
R₁₀ is hydrogen or together with R₁₀ₐ forms an oxo;
R₁₀ₐ is hydrogen, hydroxy, protected hydroxy, or together with R₁₀ forms an oxo;
R₁₃ is hydroxy or protected hydroxy;
R₁₄ is hydrogen, optionally substituted alkyl, optionally substituted alkenyl, optionally substituted alkynyl, optionally substituted aryl, or optionally substituted heteroaryl;
R₁₄ₐ is hydrogen, optionally substituted alkyl, optionally substituted alkenyl, optionally substituted alkynyl, optionally substituted aryl, or optionally substituted heteroaryl, hydroxy, protected hydroxy or together with R₁ forms a carbonate;
R₂₈ is hydrogen, hydroxy protecting group or a functional group which increases the solubility of the taxane derivative; and
R₃₀ and R₃₁ are independently hydrogen, optionally substituted alkyl, optionally substituted alkenyl, optionally substituted alkynyl, optionally substituted monocyclic aryl or optionally substituted monocyclic heteroaryl.

The derivative or analog of baccatin III or 10-desacetyl baccatin III may be prepared from a C1 hydroxy C2 benzoate derivative of baccatin III or 10-desacetyl baccatin III, and the C1 hydroxy C2 benzoate derivative may be reduced with sodium bis (2-methoxyethoxy) aluminum hydride to form a 1,2-diol derivative, the 1,2-diol then being reacted with Cl₂CO to form a 1,2-carbonate, and the 1,2-carbonate may then be reacted with a nucleophilic reagent to convert the C2 substituent to R₃₁COO- wherein R₃₁ is as defined above.

The invention further provides a process for the preparation of a derivative or analog of baccatin III or 10-desacetyl baccatin III from a 1,2 carbonate derivative of baccatin III or 10-desacetyl baccatin III, the process comprising reacting the 1,2 carbonate with a nucleophilic reagent to convert the C2 substituent to R₃₁COO- wherein R₃₁ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl and the 1,2 carbonate is a compound having the formula 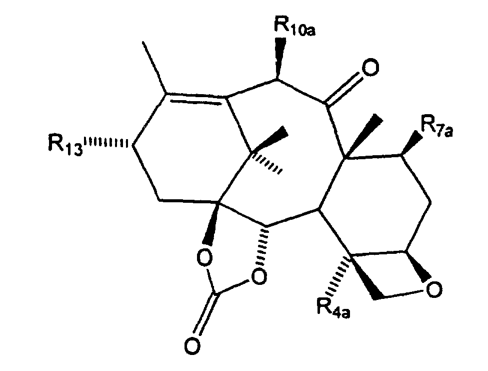 wherein
R₄ₐ is -OH or R₃₀COO-;
R₇ₐ is protected hydroxy;
R₁₀ₐ is H, -OH, protected hydroxy, or -OCOR₂₉;
R₂₃ is -OH or protected hydroxy;
R₂₉ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl; and
R₃₀ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

In another aspect of the invention there is provided a compound having the formula 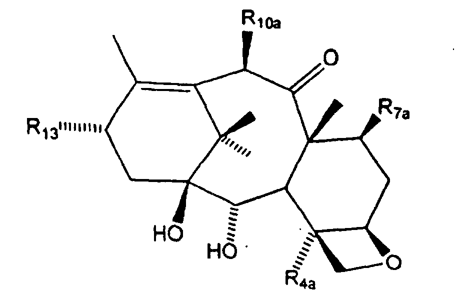 wherein
R₁₀ₐ is H, -OH, protected hydroxy, or -OCOR₂₉;
R₁₃ is -OH or protected hydroxy;
R₄ₐ is R₃₀COO-;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl with the exception that, when R₁₀ₐ and R₁₃ both represent -OH and R₄ₐ is acetoxy, then R₇ₐ is not triethylsilyloxy.

Also provided in accordance with the invention is a compound having the formula 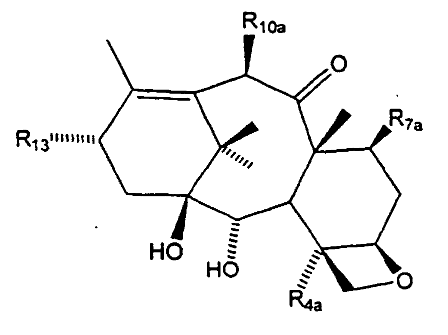 wherein
R₁₀ₐ is H, -OH, protected hydroxy, or -OCOR₂₉;
R₁₃ is -OH or protected hydroxy;
R₄ₐ is -OH or R₃₀COO-;
R₇ₐ is H; and
R₂₉ and R₃₀ are independently H, optionally substituted C₁-C₆ alkyl, optionally substituted C₂-C₅ alkenyl, optionally substituted C₂-C₆ alkynyl, optionally substituted monocyclic aryl, or optionally substituted monocyclic heteroaryl.

The invention further provides a compound having the formula 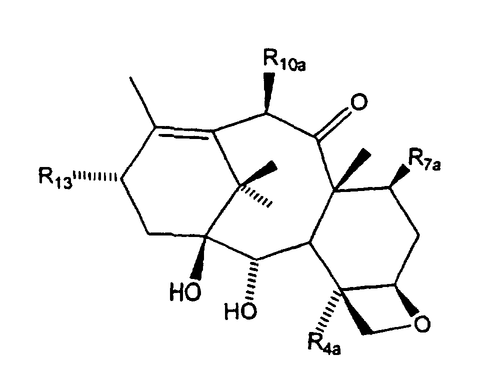 wherein
R₁₀ₐ is H or -OCOR₂₉;
R₁₃ is -OH or protected hydroxy;
R₄ₐ is -OH or R₃₀COO-;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

Also included within the scope of the invention is a compound having the formula 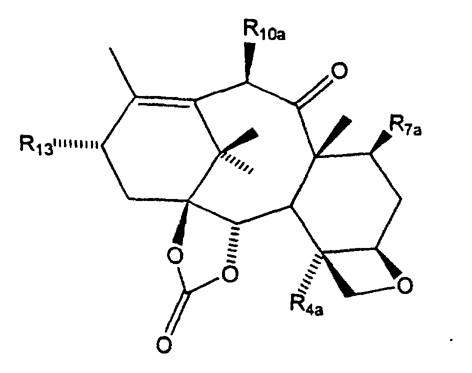 wherein
R₁₀ₐ is H, -OH, protected hydroxy, or -OCOR₂₉;
R₁₃ is -OH or protected hydroxy;
R₄ₐ is -OH or R₃₀COO-;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

Other objects and features of this invention will be in part apparent and in part pointed out hereinafter.

### DETAILED DESCRIPTION OF THE INVENTION

As used herein "Ar" means aryl; "Ph" means phenyl; "Me" means methyl; "Et" means ethyl; "iPr" means isopropyl; "tBu" means tert-butyl; "R" means lower alkyl unless otherwise defined; "Ac" means acetyl; "py" means pyridine; "TES" means triethylsilyl; "TMS" means trimethylsilyl; "TBS" means Me₂t-BuSi-; "Tf" means -SO₂CF₃; "BDMA". means BrMgNiPr₂; "Swern" means (COCl)₂, Et₃N; "LTMP" means lithium tetramethylpiperidide; "MOP" means 2-methoxy-2-propyl; "BOM" means benzyloxymethyl; "LDA" means lithium diisopropylamide; "LAH" means lithium aluminum hydride; "Red-Al" means sodium bis(2-methoxyethoxy) aluminum hydride; "Ms" means CH₃SO₂-; "TASF" means tris(diethylamino)sulfonium-difluorotrimethylsilicate; "Ts" means toluenesulfonyl;"TBAF" means tetrabutyl ammonium hydride; "TPAP" means tetrapropyl-ammonium perruthenate; "DBU" means diazabicycloundecane; "DMAP" means p-dimethylamino pyridine; "LHMDS" means lithium hexamethyldisilazide; "DMF" means dimethylformamide; "AIBN" means azo-(bis)-isobutyronitrile; "10-DAB" means 10-deacetylbaccatin III; "FAR" means 2-chloro-1,1,2-trifluorotriethylamine; "mCPBA" means metachloroperbenzoic acid; "DDQ" means dicyanodichloroquinone; "sulfhydryl protecting group" includes, but is not limited to, hemithioacetals such as 1-ethoxyethyl and methoxymethyl, thioesters, or thiocarbonates; "amine protecting group" includes, but is not limited to, carbamates, for example, 2,2,2-trichloroethylcarbamate or tertbutylcarbamate; "protected hydroxy" means -OP wherein P is a hydroxy protecting group; and "hydroxy protecting group" includes, but is not limited to, acetals having two to ten carbons, ketals having two to ten carbons, ethers such as methyl, t-butyl, benzyl, p-methoxybenzyl, p-nitrobenzyl, allyl, trityl, methoxymethyl, methoxyethoxymethyl, ethoxyethyl, tetrahydropyranyl, tetrahydrothiopyranyl, and trialkylsilyl ethers such as trimethylsilyl ether, triethylsilyl ether, dimethylarylsilyl ether, triisopropylsilyl ether and t-butyldimethylsilyl ether; esters such as benzoyl, acetyl, phenylacetyl, formyl, mono-, di-, and trihaloacetyl such as chloroacetyl, dichloroacetyl, trichloroacetyl, trifluoro-acetyl; and carbonates including but not limited to alkyl carbonates having from one to six carbon atoms such as methyl, ethyl, n-propyl, isopropyl, n-butyl, t-butyl; isobutyl, and n-pentyl; alkyl carbonates having from one to six carbon atoms and substituted with one or more halogen atoms such as 2,2,2-trichloroethoxymethyl and 2,2,2-tri-chloroethyl; alkenyl carbonates having from two to six carbon atoms such as vinyl and allyl; cycloalkyl carbonates having from three to six carbon atoms such as cyclopropyl, cyclobutyl, cyclopentyl and cyclohexyl; and phenyl or benzyl carbonates optionally substituted on the ring with one or more C₁₋₆ alkoxy, or nitro. Other hydroxyl, sulfhydryl and amine protecting groups may be found in "Protective Groups in Organic Synthesis" by T. W. Greene, John Wiley and Sons, 1981.

The alkyl groups described herein are preferably lower alkyl containing from one to six carbon atoms in the principal chain and up to 15 carbon atoms. They may be straight or branched chain and include methyl, ethyl, propyl, isopropyl, butyl, hexyl and the like. They may be heterosubstituted with the various substituents defined herein, including alkaryl.

The alkenyl groups described herein are preferably lower alkenyl containing from two to six carbon atoms in the principal chain and up to 15 carbon atoms. They may be straight or branched chain and include ethenyl, propenyl, isopropenyl, butenyl, isobutenyl, hexenyl, and the like. They may be heterosubstituted with the various substituents defined herein, including alkenaryl.

The alkynyl groups described herein are preferably lower alkynyl containing from two to six carbon atoms in the principal chain and up to 15 carbon atoms. They may be straight or branched chain and include ethynyl, propynyl, butynyl, isobutynyl, hexynyl, and the like. They may be heterosubstituted with the various substituents defined herein, including alkynaryl.

The aryl moieties described herein contain from 6 to 15 carbon atoms and include phenyl. They may be hydrocarbon or heterosubstituted with the various substituents defined hereinbelow. Phenyl is the more preferred aryl.

The heteroaryl moieties described herein contain from 5 to 15 atoms and include, furyl, thienyl, pyridyl and the like. They may be hydrocarbon or heterosubstituted with the various substituents defined hereinbelow.

The acyl moieties described herein contain alkyl, alkenyl, alkynyl, aryl or heteroaryl groups.

The alkoxycarbonyloxy moieties described herein comprise lower alkyl, alkenyl, alkynyl or aryl groups.

The hydrocarbon substituents described herein may be alkyl, alkenyl, alkynyl, or aryl, and the hetero-substituents of the heterosubstituted alkyl, alkenyl, alkynyl, aryl, and heteroaryl moieties described herein contain nitrogen, oxygen, sulfur, halogens and/or one to six carbons, and include lower alkoxy such as methoxy, ethoxy, butoxy, halogen such as chloro or fluoro, and nitro, heteroaryl such as furyl or thienyl, alkanoxy, hydroxy, protected hydroxy, acyl, acyloxy, nitro, amino, and amido.

Surprisingly, it has been discovered that the C2 ester of a suitably protected derivative of baccatin III or 10-DAB having the formula 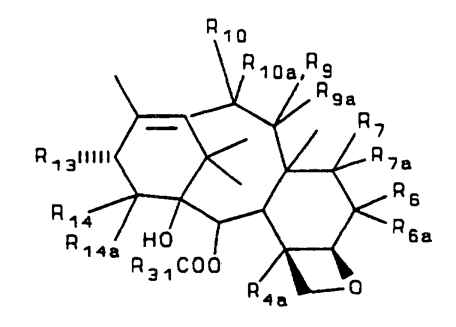 may be selectively reduced to form a 1,2 diol having the formula 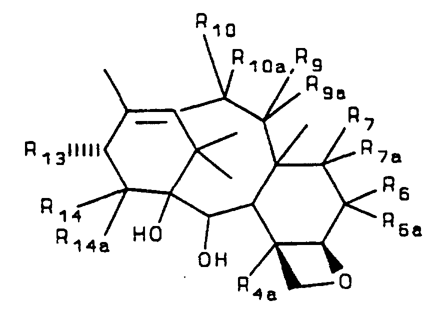 which, in turn, may be converted to a 1,2 carbonate intermediate which permits the selective formation of a variety of C2 esters through reaction with alkyl, alkenyl, alkynyl or aryl lithium reagents or Grignard reagents. The 1,2 carbonate intermediate has the formula 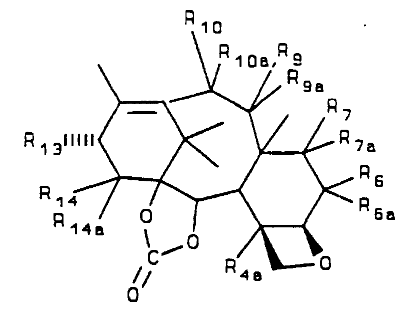 wherein
R₄ₐ is hydrogen, alkyl, alkenyl, alkynyl, aryl, heteroaryl, cyano, hydroxy, or -OCOR₃₀;
R₆ is hydrogen, alkyl, alkenyl, alkynyl, aryl, or heteroaryl, hydroxy, protected hydroxy or together with R₆ₐ forms an oxo;
R₆ₐ is hydrogen, alkyl, alkenyl, alkynyl, aryl, or heteroaryl, hydroxy, protected hydroxy or together with R₆ forms an oxo;
R₇ is hydrogen or together with R₇ₐ forms an oxo,
R₇ₐ is hydrogen, halogen, protected hydroxy, -OR₂₈, or together with R₇ forms an oxo;
R₉ is hydrogen or together with R₉ₐ forms an oxo,
R₉ₐ is hydrogen, hydroxy, protected hydroxy, or together with R₉ forms an oxo;
R₁₀ is hydrogen or together with R₁₀ₐ forms an oxo,
R₁₀ₐ is hydrogen, hydroxy, protected hydroxy, or together with R₁₀ forms an oxo;
R₁₃ is hydroxy or protected hydroxy;
R₁₄ is hydrogen, alkyl, alkenyl, alkynyl, aryl, or heteroaryl;
R₁₄ₐ is hydrogen, alkyl, alkenyl, alkynyl, aryl, or heteroaryl, hydroxy, protected hydroxy or together with R₁ forms a carbonate;
R₂₈ is hydrogen, hydroxy protecting group or a functional group which increases the solubility of the taxane derivative; and
R₃₀, and R₃₁ are independently hydrogen, alkyl, alkenyl, alkynyl, monocyclic aryl or monocyclic heteroaryl.

Any agent which selectively removes the C2 and/or C4 acyl groups and thereby converts the ester(s) to the corresponding alcohol(s) may be used. The agent may be a reducing agent, preferably a hydride of aluminum or boron, more preferably an alkyl substituted aluminum hydride or an alkyl substituted borohydride, and most preferably lithium aluminum hydride ("LAH"), sodium bis(2-methoxyethoxy) aluminum hydride ("Red-Al") or lithiumtriethylborohydride. Alternatively, the agent may be a base, preferably a tetraalkylammonium base and most preferably, tetrabutylammoniumhydroxide. The conversion of the ester to the corresponding alcohol is carried out in a single phase, non-aqueous system such as methylene chloride.

After the C2 and/or C4 esters are reduced to the corresponding alcohol(s), standard acylating agents such as anhydrides and acid chlorides in combination with an amine such as pyridine, triethylamine, DMAP, or diisopropyl ethyl amine can be used to form new esters at C2 and/or C4. Alternatively, the C2 and/or C4 alcohols may be converted to new C2 and/or C4 esters through formation of the corresponding alkoxide by treatment of the alcohol with a suitable base such as LDA followed by an acylating agent such as an acid chloride.

As will be discussed in greater detail below, baccatin III and 10-DAB derivatives having new C2 and/or C4 esters can be produced by several reaction schemes. To simplify the description, 10-DAB is used as the starting material in Reaction Schemes 1-6. Baccatin III derivatives or analogs, however, may be produced using the same reactions (except for the protection of the C10 hyaroxy group with TES) by simply replacing 10-DAB with baccatin III as the starting material.

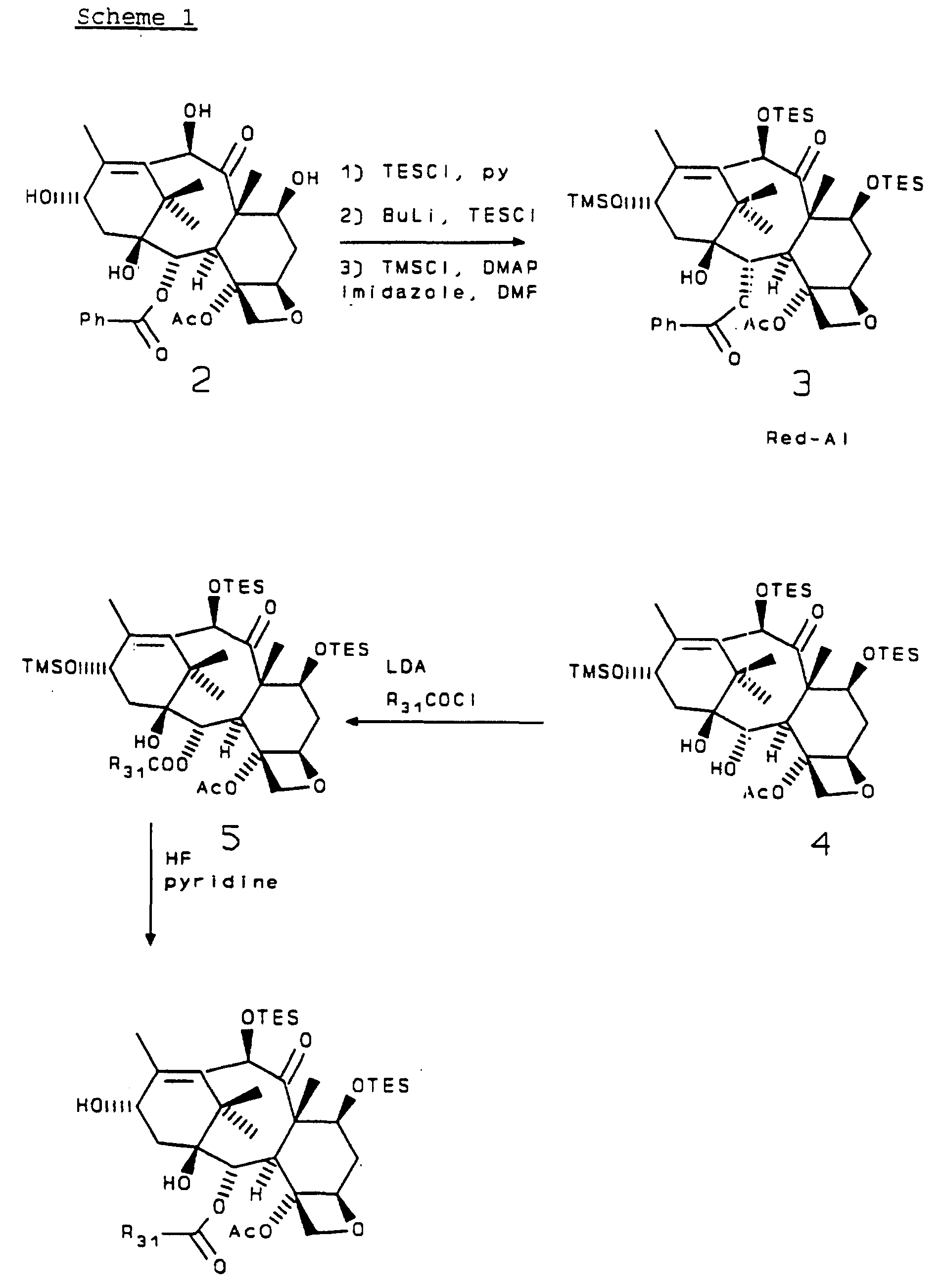

In Reaction Scheme 1, the C7 hydroxyl group of 10-deacetyl baccatin (III) was selectively protected as its triethylsilyl (TES) ether as described by Green, et al., JACS 110, 5917 (1988). The C10 hydroxyl group was then protected as the TES ether through the use of n-butyllithium and triethylsilyl chloride. The C13 hydroxyl group was subsequently protected as the trimethylsilyl (TMS) ether, which could be selectively removed at a later stage. The fully protected 13-O-trimethylsilyl-7,10-bis-O-triethylsilyl10-deacetyl baccatin (III) **3** underwent selective reduction with Red-Al to give the 2 hydroxy derivative **4.** 2 hydroxy derivative 4 may alternatively be obtained by selectively reducing the fully protected baccatin III 3 with tetrabutylammonium borohydride in ether or by hydrolyzing the C4 ester of fully protected baccatin III with tetrabutylammoniumhydroxide. Deprotonation of **4** with either n-butyllithium or a bulky amide base such as LDA was followed by the addition of an appropriate acid chloride to provide the C2 ester derivative **5.** The C13 TMS group may then be removed using HF.

As shown in Reaction Scheme 2, 1,2 diol **4** can be readily converted to the 1,2 carbonate **6** which can be transformed to the C2 formate **5** (R₃₁ = H) by treatment with Red-Al under mild conditions. In addition, carbonate **6** reacts selectively with nucleophilic agents (e.g., Grignard reagents or alkyllithium reagents) to provide the C2 ester derivative **5**. Again, the C13 TMS group may then be removed using HF.

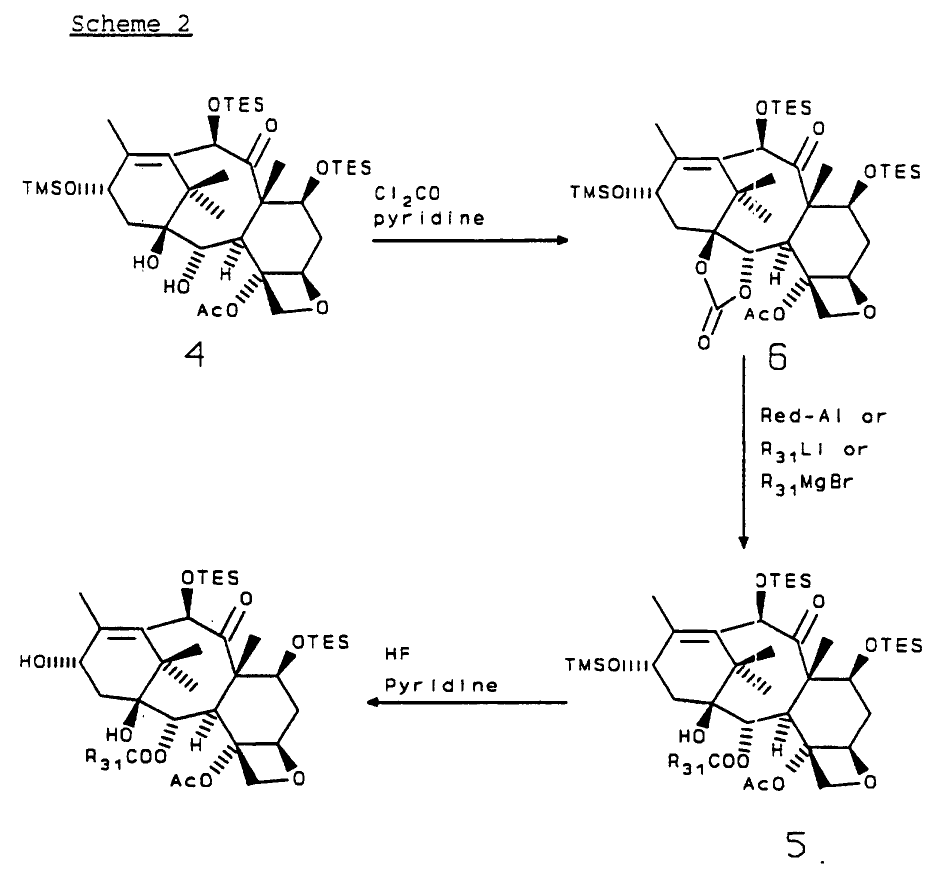

10-DAB analogs having different substituents only at C4, or at both C2 and C4 can be prepared as set forth in Reaction Schemes 3-6.

In Reaction Scheme 3, protected 10-DAB **3** is converted to the triol **7** with lithium aluminum hydride. Triol **7** is then converted to the corresponding C2 ester using Cl₂CO in pyridine followed by a nucleophilic agent (e.g., Grignard reagents or alkyllithium reagents).

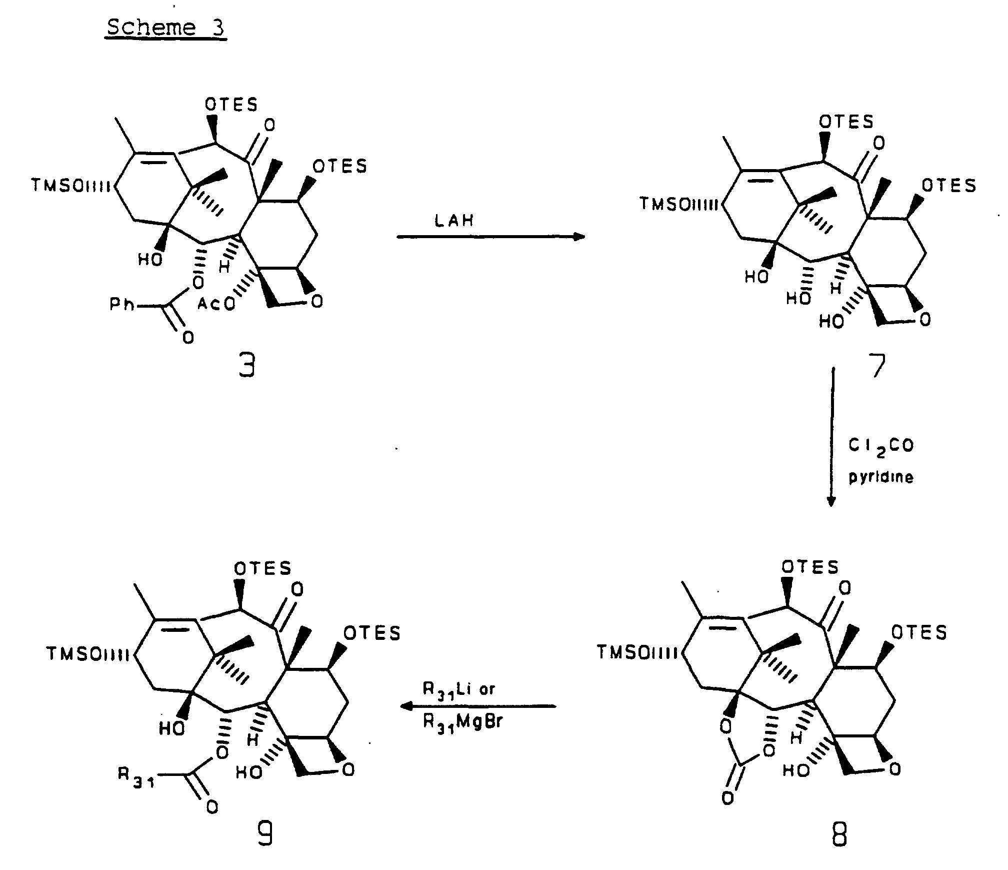

Alternatively, protected 10-DAB 3 may be converted directly to the C2 ester by treating 10-DAB 3 with lithiumtriethylborohydride which selectively cleaves the C4 acetyl group. Also, deprotonation of triol **7** with LDA followed by introduction of an acid chloride selectively gives the C4 ester. For example, when acetyl chloride was used, triol **7** was converted to 1,2 diol **4** as set forth in Reaction Scheme 4. 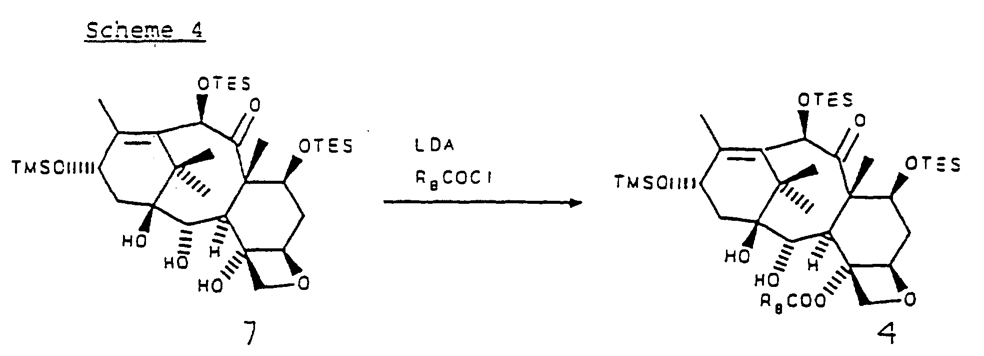 wherein R₈ is hydrogen, alkyl, alkenyl, alkynyl, monocyclic aryl or monocyclic heteroaryl.

Triol **7** can also readily be converted to the 1,2 carbonate **8**. Acetylation of carbonate **8** under vigorous standard conditions provides carbonate **6** as described in Reaction Scheme 5; addition of alkyllithiums or Grignard reagents to carbonate **6** provides the C2 ester having a free hydroxyl group at C4 as set forth in Reaction Scheme 2. As set forth in Reaction Scheme 6, other C4 substituents can be provided by reacting carbonate **8** with an acid chloride and a tertiary amine to yield carbonate **10** which is then reacted with alkyllithiums or Grignard reagents to provide 10-DAB derivatives having new substituents at C2 as set forth in Reaction Scheme 6.

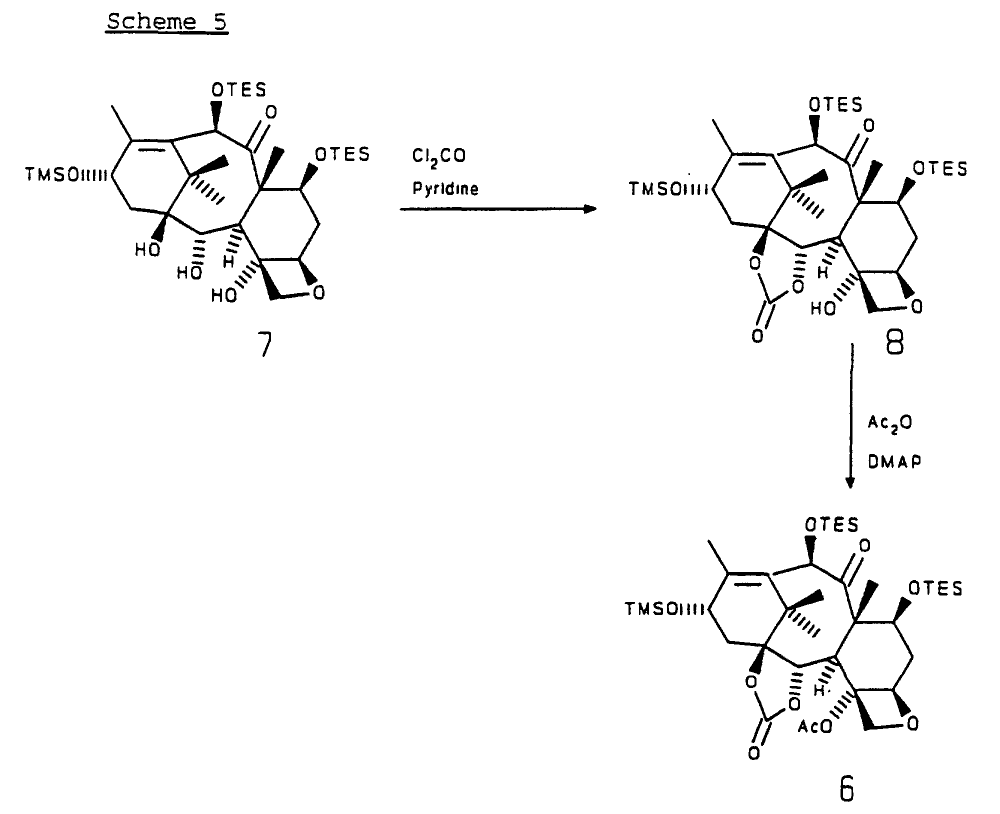

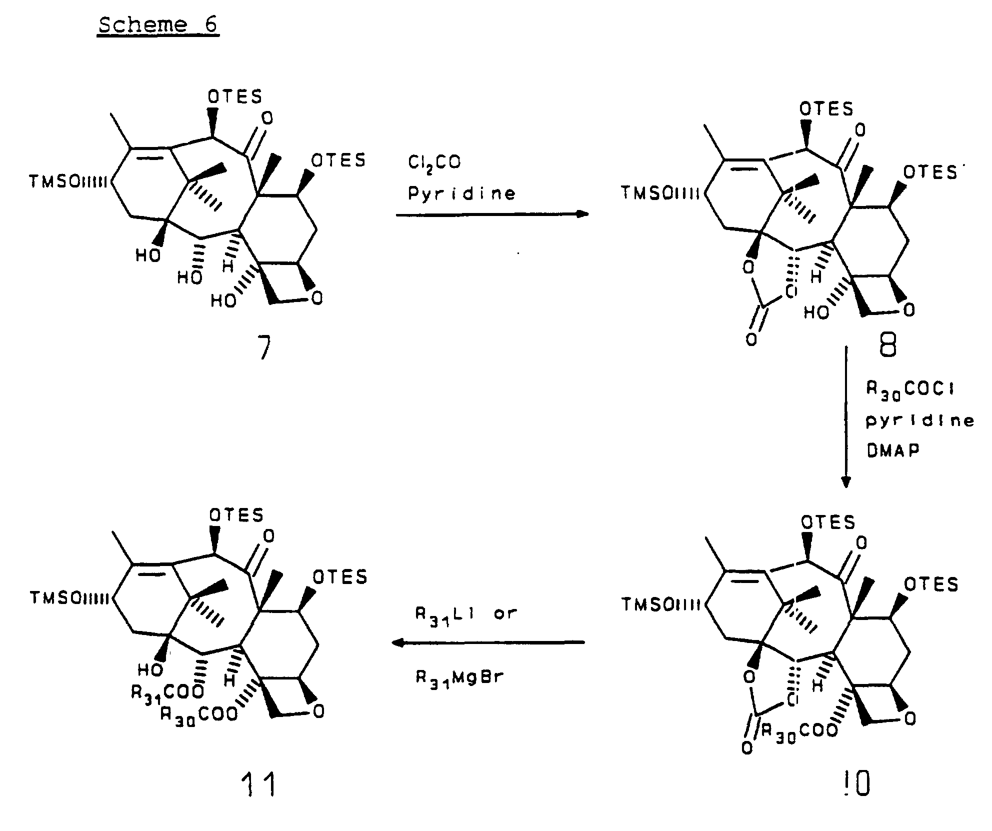

Taxanes having alternative acyloxy C2 and C10 substituents may be prepared as set forth in Reaction Scheme 7, using, for example, baccatin III as a starting material. After being protected at C7 and C13, baccatin III is reduced with LAH to produce 1,2,4,10 tetraol **12.** Tetraol **12** is converted to carbonate **13** using Cl₂CO and pyridine, and carbonate **13** is acylated at C10 with an acid chloride and pyridine to produce carbonate **14** (as shown) or with acetic anhydride and pyridine (not shown). Acetylation of carbonate **14** under vigorous standard conditions provides carbonate **15** which is then reacted with alkyl lithiums to provide the baccatin III derivatives having new substituents at C2 and C10. 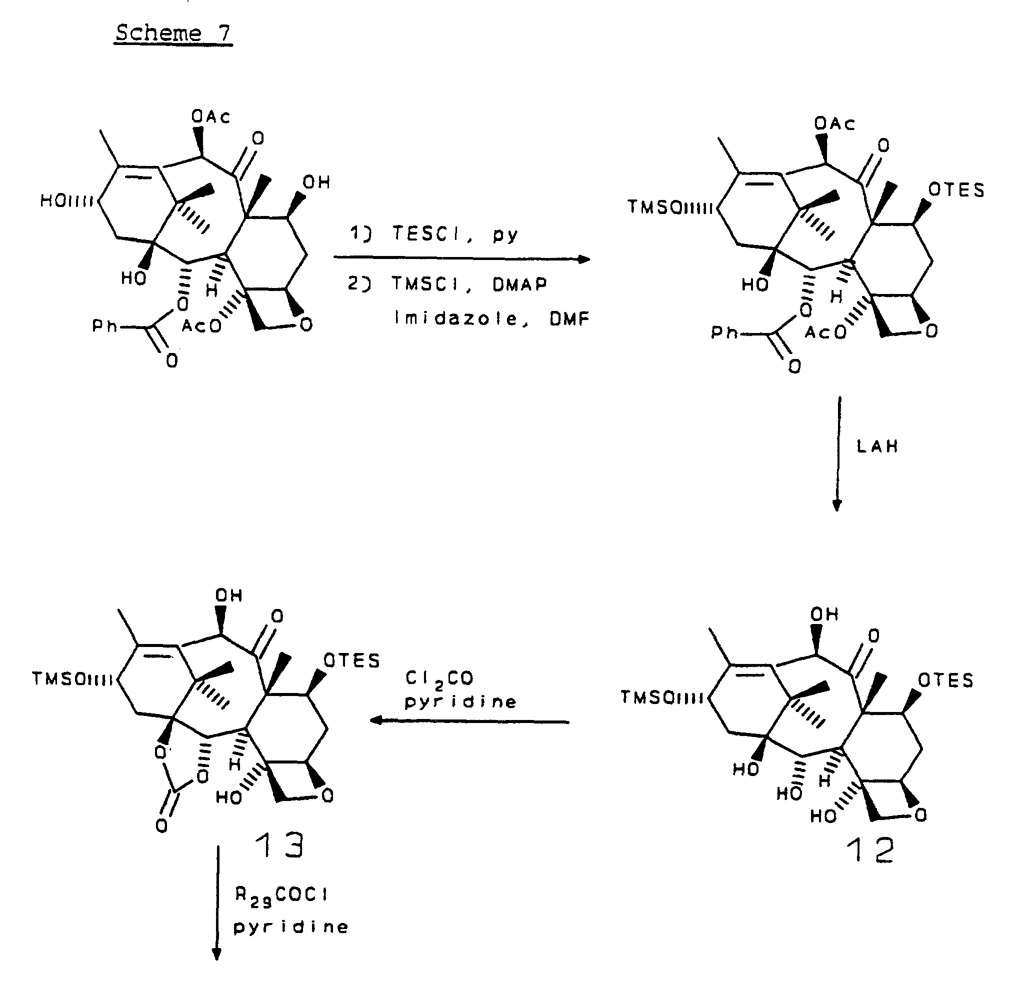 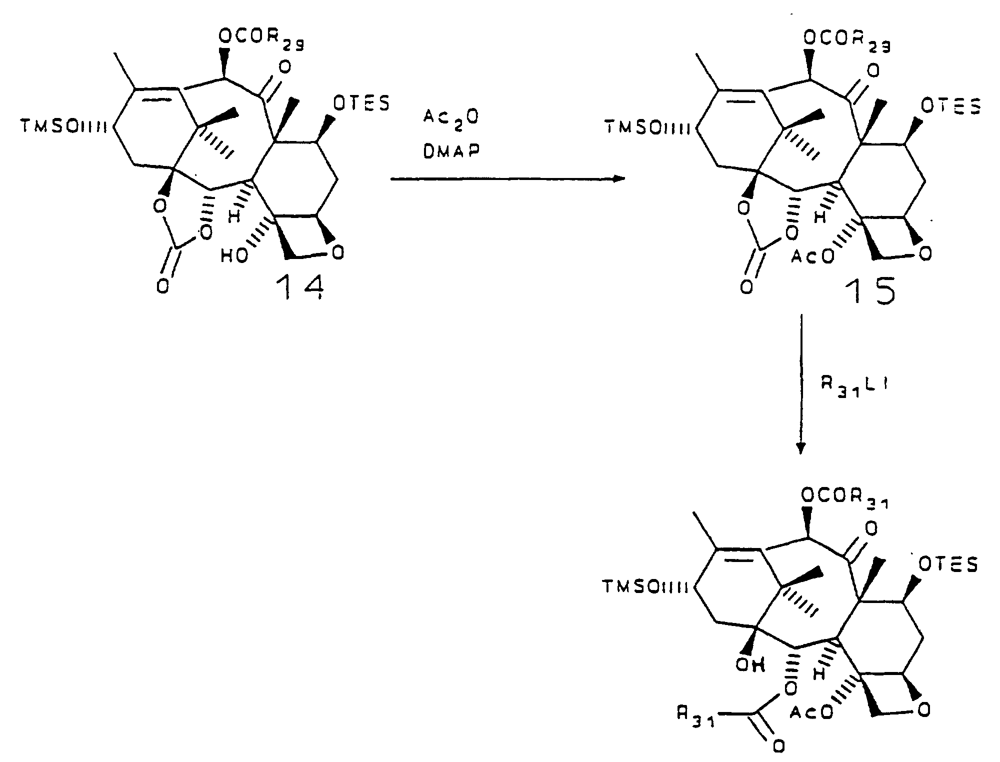 wherein R₂₉ in hydrogen, alkyl, alkenyl, alkynyl, monocyclic aryl or monocyclic heteroaryl.

10-desacetoxy derivatives of baccatin III and 10-desoxy derivatives of 10-DAB having alternative C2 and C4 substituents may be produced using the same reactions (except for the protection of the C10 hydroxy group with TES) by simply replacing 10-DAB with 10-desacetoxy baccatin III as the starting material in Reaction Schemes 1-6. Baccatin III and 10-DAB may be selectively and nearly quantitatively converted to the corresponding 10-desacetoxy or 10-desoxytaxane when they are reacted with samarium diiodide. Alternatively, the 10-DAB derivatives having alternative C2 and C4 substituents may themselves be reacted with samarium diiodide to yield the corresponding 10-deacetoxy compound.

As illustrated in Reaction Scheme 8, the reaction of baccatin III with Bu₄NBH₄ in methylene chloride yields 9-desoxo-9β-hydroxybaccatin III 5. After the C7 hydroxy group is protected with the triethylsilyl protecting group, for example 7-protected-9β-hydroxy derivative 6 may be used as a starting material in Reaction Schemes 1-7.

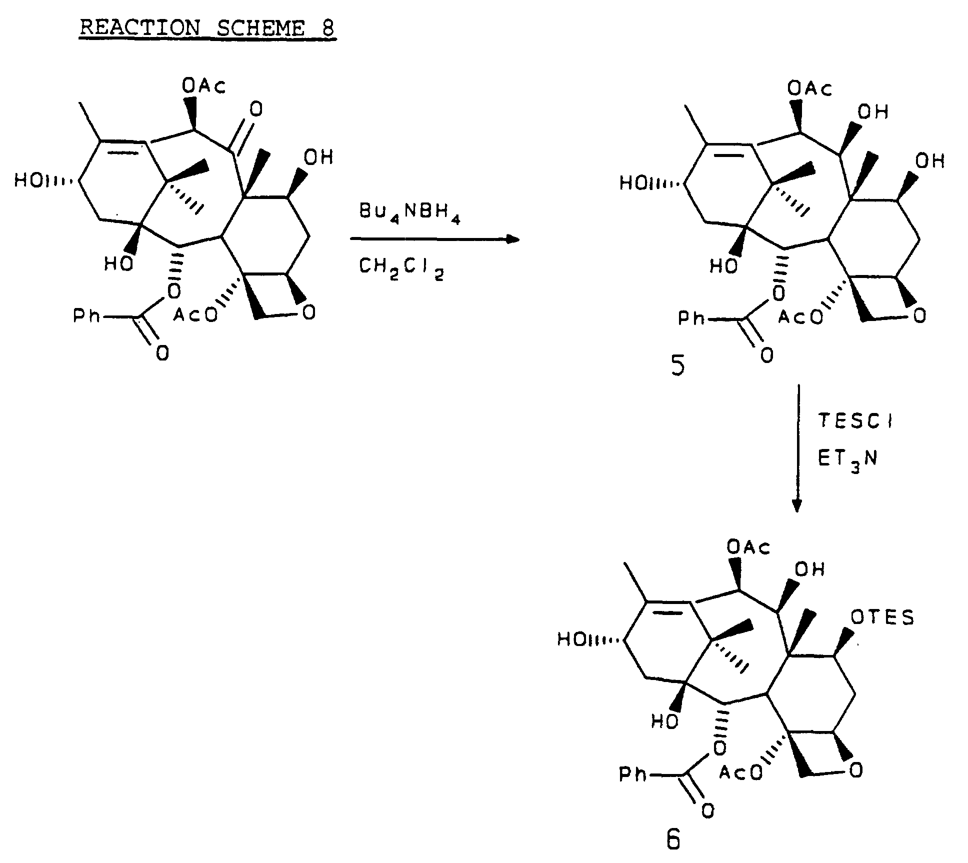

Alternatively, the C13 hydroxy group of 7-protected-9β-hydroxy derivative **6** may be protected with trimethylsilyl or other protecting group which can be selectively removed relative to the C7 hydroxy protecting group as illustrated in Reaction Scheme 9, to enable further selective manipulation of the various substituents of the taxane. For example, reaction of 7,13-protected-9β-hydroxy derivative **7** with KH causes the acetate group to migrate from C10 to C9 and the hydroxy group to migrate from C9 to C10, thereby yielding 10-desacetyl derivative **8.** Protection of the C10 hydroxy group of 10-desacetyl derivative **8** with triethylsilyl yields derivative **9.** Selective removal of the C13 hydroxy protecting group from derivative **9** yields derivative 10 which may be used as a starting material in Reaction Schemes 1-7.

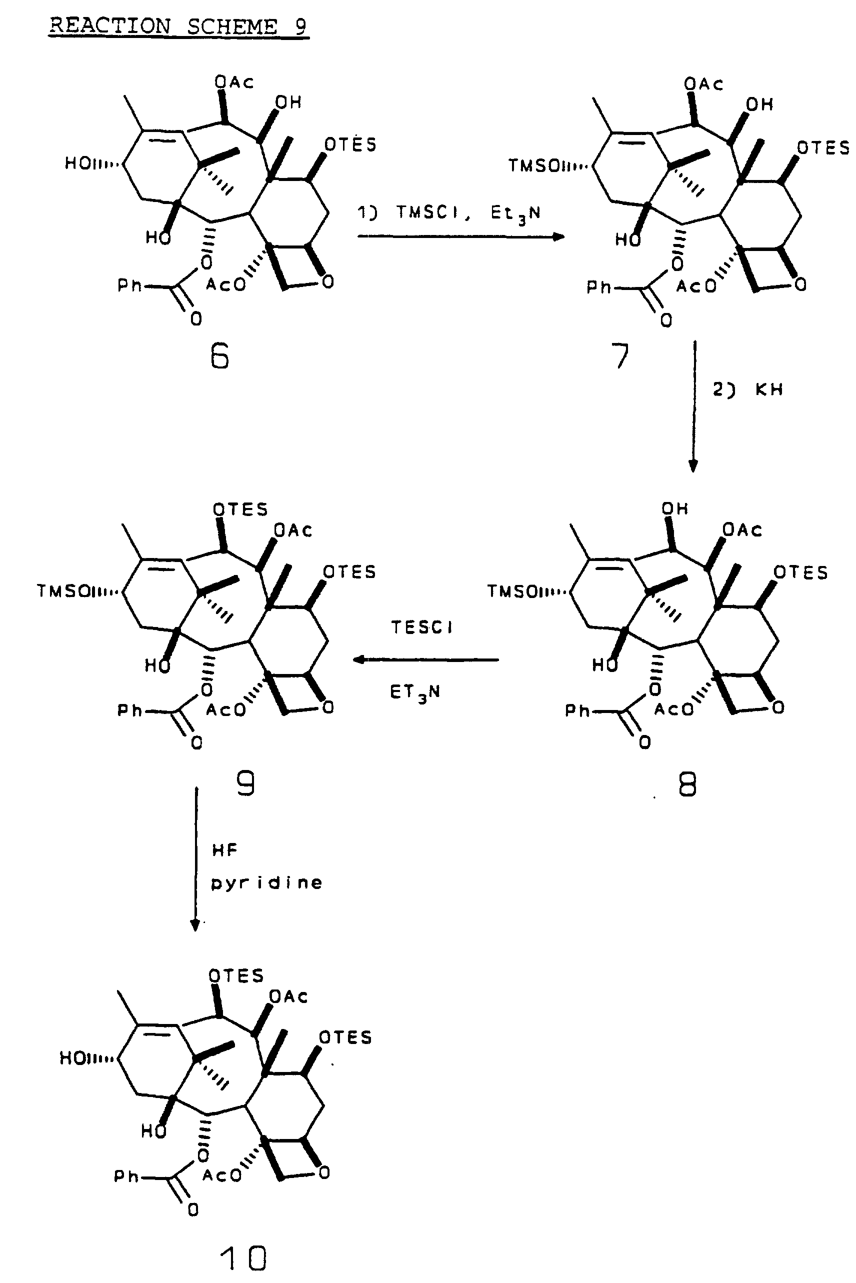

As shown in Reaction Scheme 10, 10-oxo derivative **11** can be provided by oxidation of 10-desacetyl derivative **8.** Thereafter, the C13 hydroxy protecting group can be selectively removed followed by attachment of a side chain as described above to yield 9-acetoxy-10-oxo-taxol or other 9-acetoxy-10-oxotetracyclic taxanes having a C13 side chain. Alternatively, the C9 acetate group can be selectively removed by reduction of 10-oxo derivative **11** with a reducing agent such as samarium diiodide to yield 9-desoxo-10-oxo derivative 12 which can be used as a starting material for Reaction Schemes 1-7.

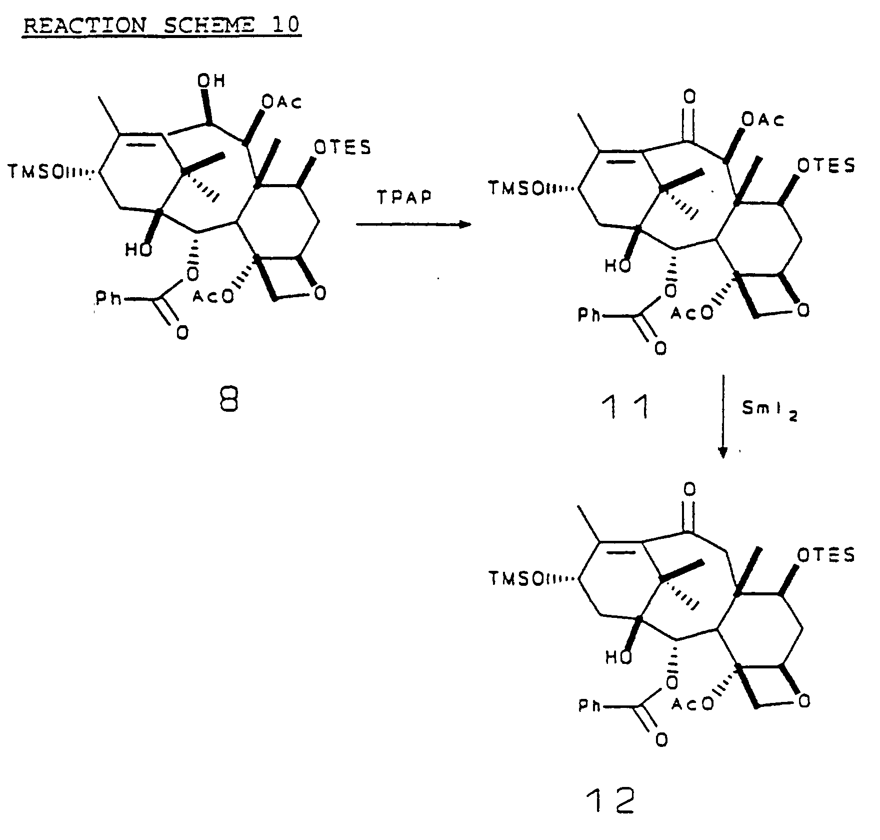

Reaction Scheme 11 illustrates a reaction in which 10-DAB is reduced to yield pentaol **13.** The C7 and C10 hydroxyl groups of pentaol **13** can then be selectively protected with the triethylsilyl or another protecting group to produce triol **14** which can be used as a starting material for Reaction Schemes 1-7 above.

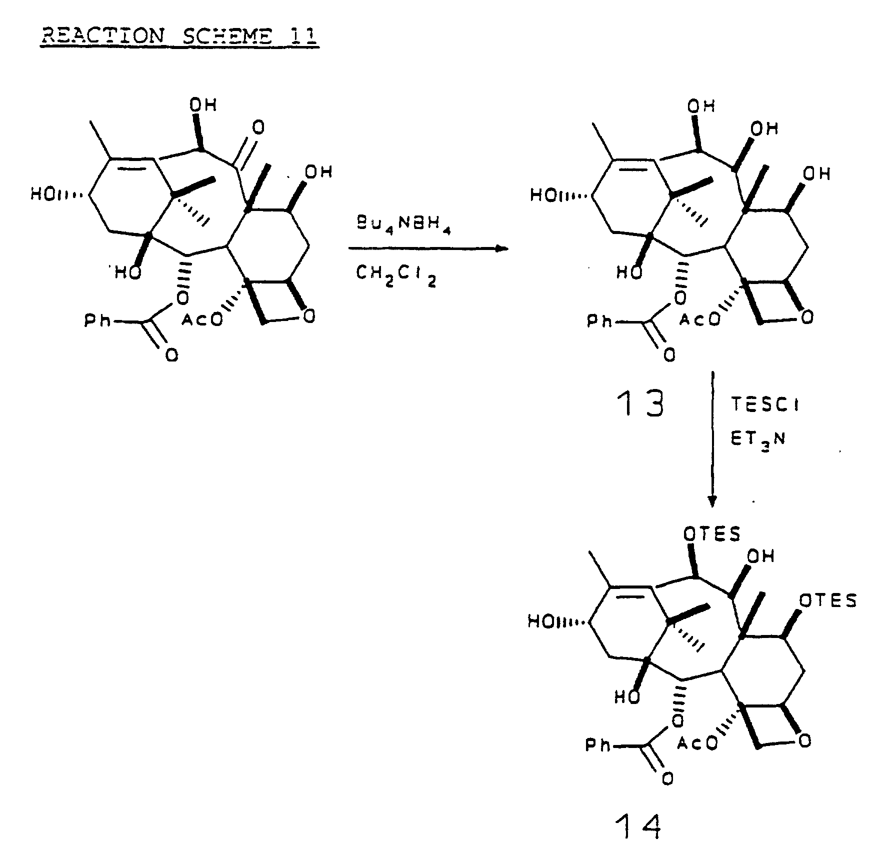

Taxanes having C9 and/or C10 acyloxy substituents other than acetate can be prepared using 10-DAB as a starting material as illustrated in Reaction Scheme 12. Reaction of 10-DAB with triethylsilyl chloride in pyridine yields 7-protected 10-DAB **15**. The C10 hydroxy substituent of 7-protected 10-DAB **15** may then be readily acylated with any standard acylating agent to yield derivative **16** having a new C10 acyloxy substituent. Selective reduction of the C9 keto substituent of derivative **16** yields 9β-hydroxy derivative **17** to which a C13 side chain may be attached. Alternatively, the C10 and C9 groups can be caused to migrate as set forth in Reaction Scheme **9,** above.

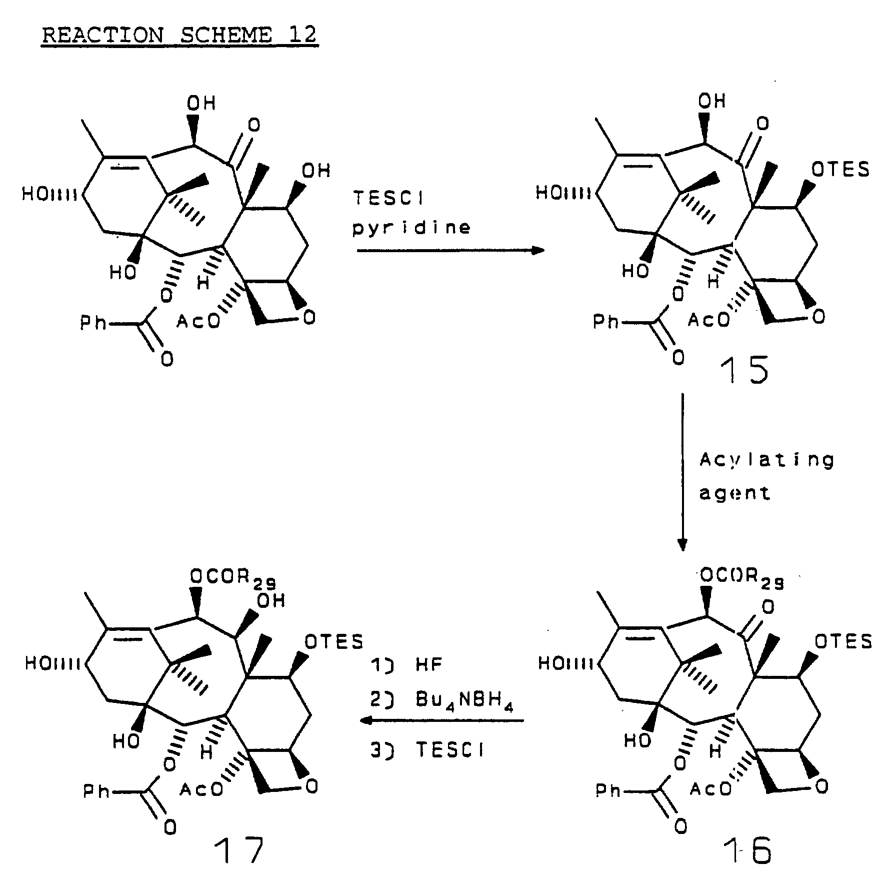

10-desacetoxy derivatives of baccatin III and 10-desoxy derivatives of 10-DAB may be prepared by reacting baccatin III or 10-DAB (or their derivatives) with samarium diiodide. Reaction between the tetracyclic taxane having a C10 leaving group and samarium diiodide may be carried out at 0°C in a solvent such as tetrahydrofuran. Advantageously, the samarium diiodide selectively abstracts the C10 leaving group; C13 side chains and other substituents on the tetracyclic nucleus remain undisturbed. Thereafter, the C9 keto substituent may be reduced to provide the corresponding 9-desoxo-9β-hydroxy-10-desacetoxy or 10-desoxy derivatives as otherwise described herein.

C7 dihydro and other C7 substituted taxanes can be prepared as set forth in Reaction Schemes 13, 14 and 14a.

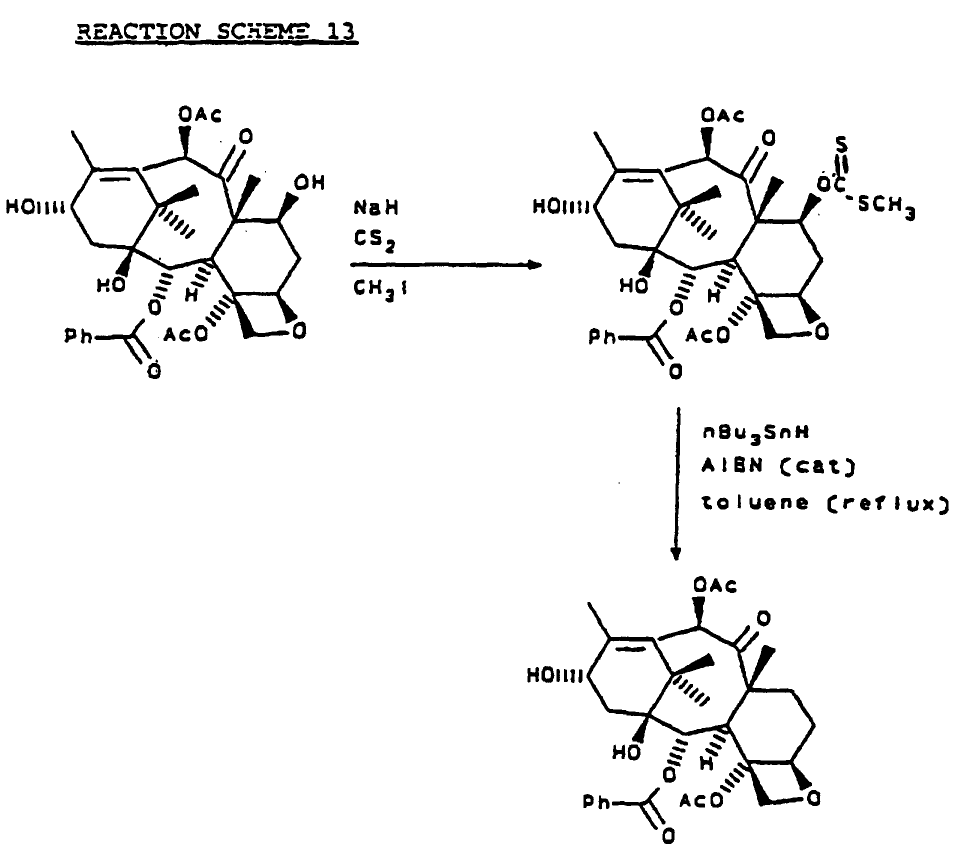

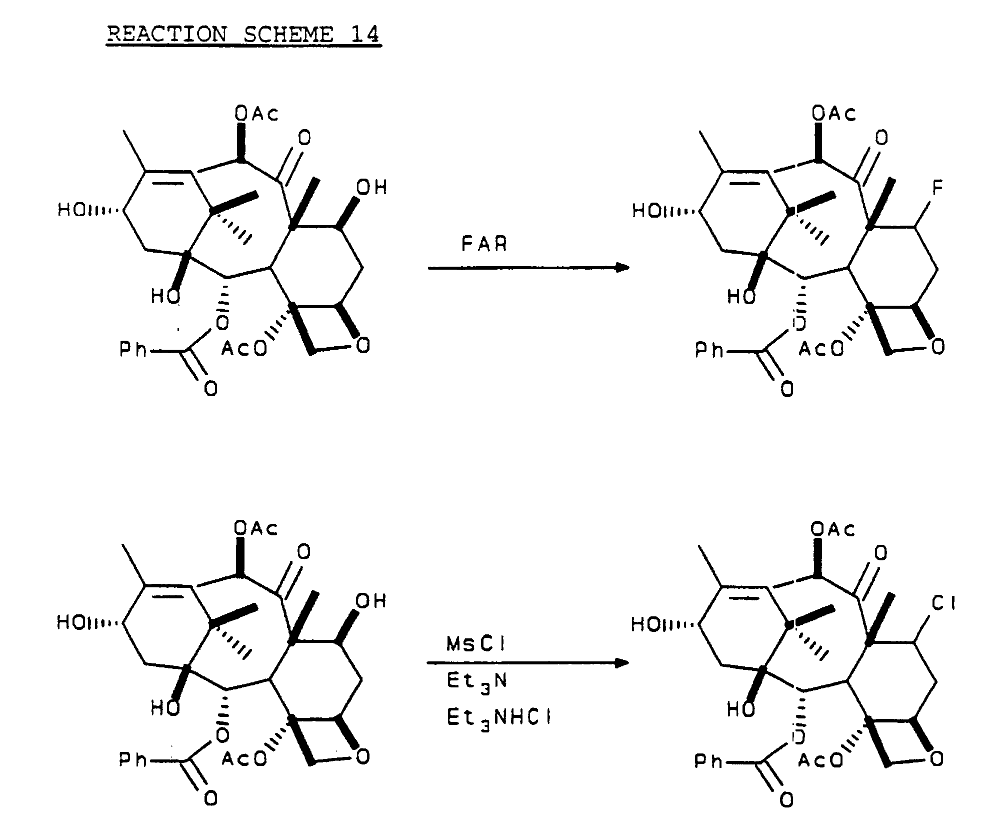

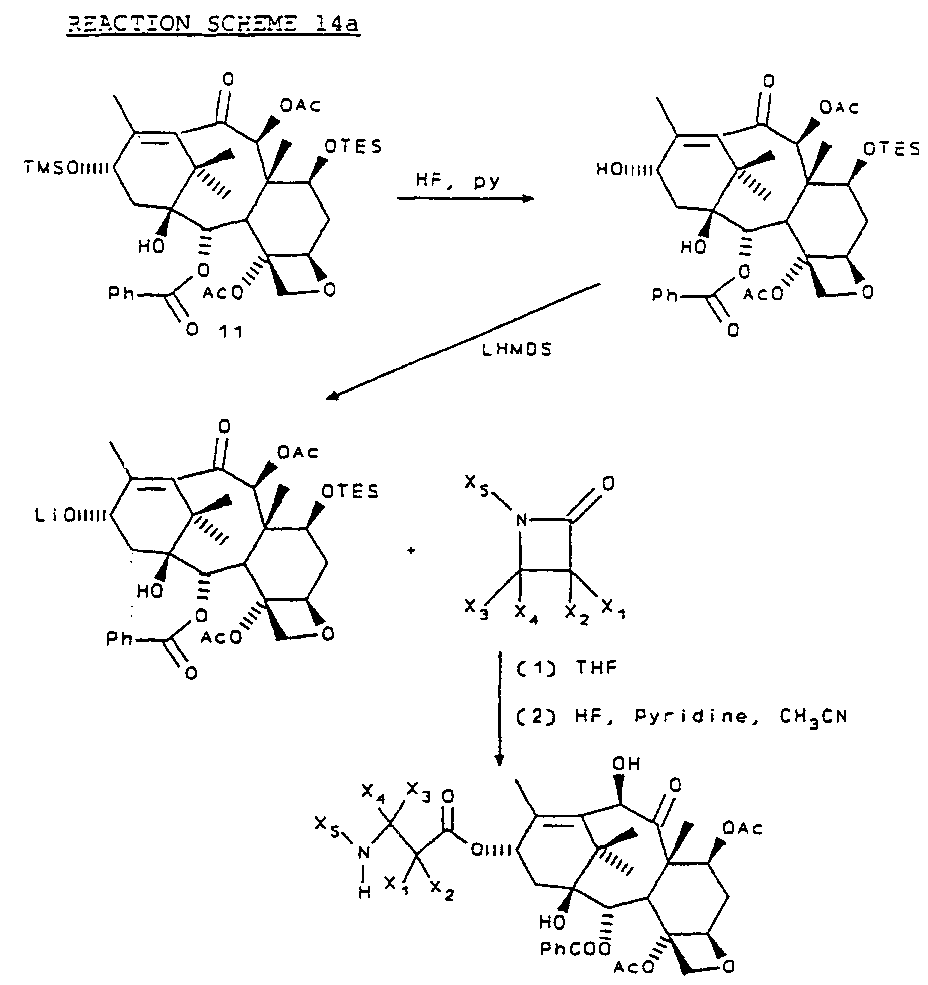

Examples of possible meanings of X₁, X₂, X₃, X₄ and X₅ are given below.

As shown in Reaction Scheme 14, Baccatin III may be converted into 7-fluoro baccatin III by treatment with FAR at room temperature in THF solution. Other baccatin derivatives with a free C7 hydroxyl group behave similarly. Alternatively, 7-chloro baccatin III can be prepared by treatment of baccatin III with methane sulfonyl chloride and triethylamine in methylene chloride solution containing an excess of triethylamine hydrochloride.

Taxanes having C7 acyloxy substituents can be prepared as set forth in Reaction Scheme 14a, 7,13-protected 10-oxo-derivative 11 is converted to its corresponding C13 alkoxide by selectively removing the C13 protecting group and replacing it with a metal such as lithium. The alkoxide is then reacted with a β-lactam or other side chain precursor. Subsequent hydrolysis of the C7 protecting groups causes a migration of the C7 hydroxy substituent to C10, migration of the C10 oxo substituent to C9, and migration of the C9 acyloxy substituent to C7.

A wide variety of tricyclic taxanes are naturally occurring, and through manipulations analogous to those described herein, an appropriate side chain can be attached to the C13 oxygen of these substances. Alternatively, as shown in Reaction Scheme 15, 7-O-triethylsilyl baccatin III can be converted to a tricyclic taxane through the action of trimethyloxonium tetrafluoroborate in methylene chloride solution. The product diol then reacts with lead tetraacetate to provide the corresponding C4 ketone.

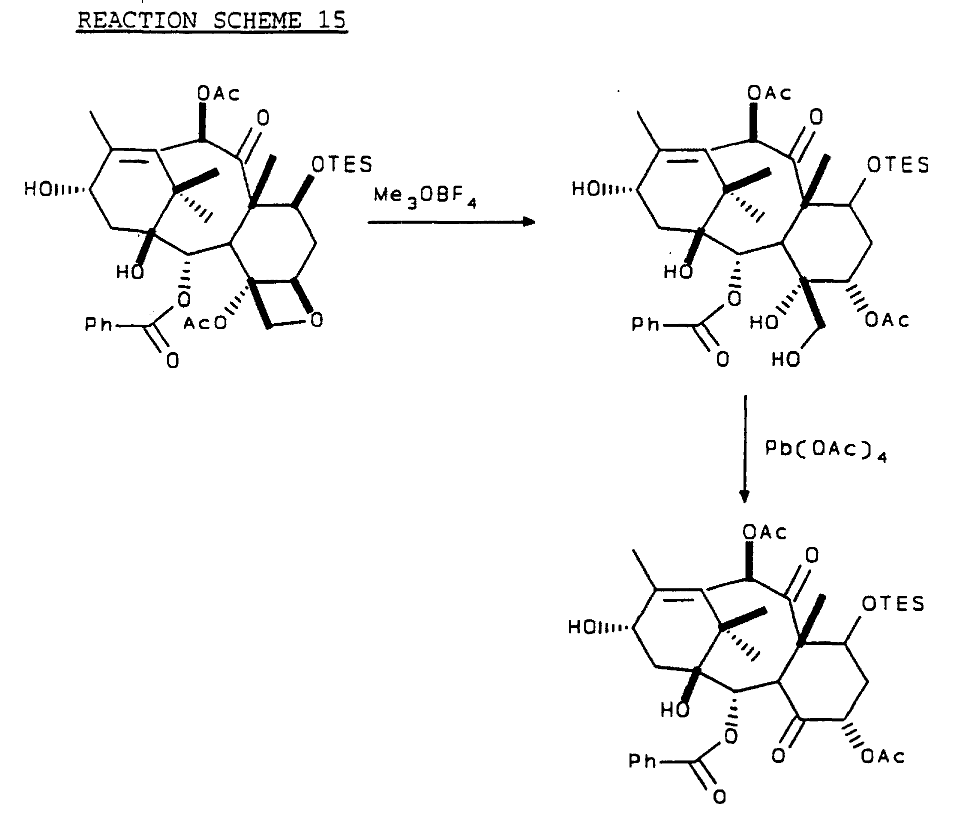

Recently a hydroxylated taxane (14-hydroxy-10-deacetylbaccatin III) has been discovered in an extract of yew needles (C&EN, p 36-37, April 12, 1993). Derivatives of this hydroxylated taxane having the various C2, C4, etc. functional groups described above may also be prepared by using this hydroxylated taxane. In addition, the C14 hydroxy group together with the C1 hydroxy group of 10-DAB can be converted to a 1,2-carbonate as described in C&EN or it may be converted to a variety of esters or other functional groups as otherwise described herein in connection with the C2, C4, C7, C9, C10 and C13 substituents.

Synthesis of tetracyclic taxanes having a C13 side-chain and different substituents at C2 and/or C4 can readily be prepared from baccatin III and 10-DAB derivatives having different substituents at C2 and/or C4 using presently known methods. For instance, a suitable side chain may be attached to a baccatin III or 10-DAB derivative as set forth in U.S. Patent Nos. 4,924,011 and 4,924,012 or by the reaction of a β-lactam and a suitably protected baccatin III or 10-desacetylbaccatin III derivative as illustrated in Reaction Scheme 14a wherein X₁ - X₅ are as follows:
X₁ is -OX₆, -SX₇, or -NX₈X₉;
X₂ is hydrogen, alkyl, alkenyl, alkynyl, aryl, or heteroaryl;
X₃ and X₄ are independently hydrogen, alkyl, alkenyl, alkynyl, aryl, or heteroaryl;
X₅ is -COX₁₀, -COOX₁₀, -COSX₁₀, -CONX₈X₁₀, or -SO₂X₁₁;
X₆ is hydrogen, alkyl, alkenyl, alkynyl, aryl, heteroaryl, hydroxy protecting group, or a functional group which increases the water solubility of the taxane derivative;
X₇ is alkyl, alkenyl, alkynyl, aryl, heteroaryl, or sulfhydryl protecting group;
X₈ is hydrogen, alkyl, alkenyl, alkynyl, aryl, heteroaryl, or heterosubstituted alkyl, alkenyl, alkynyl, aryl or heteroaryl;
X₉ is an amino protecting group;
X₁₀ is alkyl, alkenyl, alkynyl, aryl, heteroaryl, or heterosubstituted alkyl, alkenyl alkynyl, aryl or heteroaryl;
X₁₁ is alkyl, alkenyl, alkynyl, aryl, heteroaryl, -OX₁₀, or -NX₈X₁₄; and
X₁₄ is hydrogen, alkyl, alkenyl, alkynyl, aryl, or heteroaryl.

The following examples are provided to more fully illustrate the invention.

### EXAMPLE 1

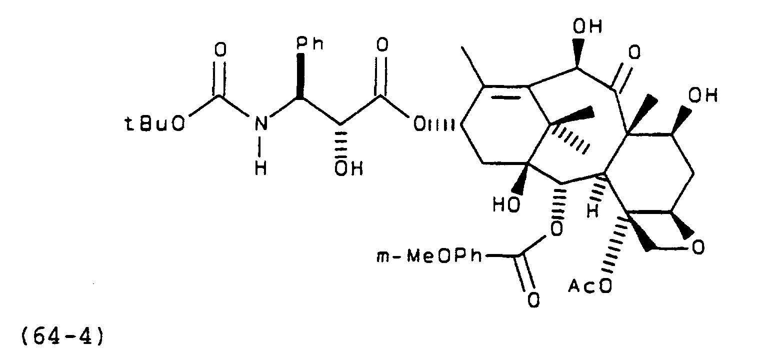

### Preparation of 2-desbenzoyl-2-(3-methoxybenzoyl)-10-deacetyl-N-desbenzoyl-N-(t-butoxycarbonyl) taxol.

To a solution of 2-desbenzoyl-2-(3-methoxybenzoyl)-10-deacetyl-7,10-bis(triethylsilyl) baccatin III (48.2 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 70.8 mg of a mixture containing (2'R,3'S)- 2',7,10-tris(triethylsilyl)-2-desbenzoyl-2-(3-methoxybenzoyl)-10-deacetyl-N-debenzoyl-N-(t-butoxycarbonyl) taxol and a very small amount of the (2'S,3'R) isomer.

To a solution of 70.8 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 50.3 mg of material which was purified by recrystallization to give 43.1 mg (86%) of 2-desbenzoyl-2-(3-methoxybenzoyl)-10-deacetyl-N-debenzoyl-N-(t-butoxycarbonyl) taxol.
m.p.162-164 °C; [α]²⁵_{Na} -61.6° (c 0.790, CHCl₃).
¹H NMR (CDCl₃, 300 MHz) δ 7.67 (m, 2H, methoxybenzoate, *ortho),* 7,36 (m, 6H, aromatic), 7.15 (m, 1H, methoxybenzoate), 6.19 (m, 1H, H13), 5.65 (d, *J* = 6.9 Hz, 1H, H2β), 5.50 (m, 1H, NH), 5.21 (m, 2H, H3', H10), 4.95 (dd, *J* = 7.8, 1.8 Hz, 1H, H5), 4.60 (m, 1H, H2'), 4.33 (d, *J* = 8.7 Hz, 1H, H20α), 4.23 (m, 1H, H7), 4.17 (d, *J* = 8.7 Hz, 1H, H20β), 3.89 (d, J= 6.9 Hz, 1H, H3), 3.86 (s, 3H, methoxy), 3.56 (m, 1H, 2'OH), 2.55 (m, 1H, H6α), 2.34 (s, 3H, 4Ac), 2.23 (m, 2H, H14), 1.83 (s, 3H, Me18), 1.79 (m, 1H, H6β) 1.73 (s, 3H, Me19), 1.32 (s, 9H, t-butyl), 1.22 (s, 3H, Me17), 1.11 (s, 3H, Me16).

### EXAMPLE 2

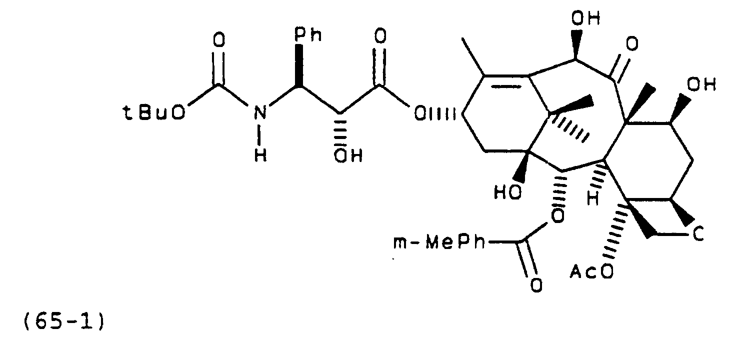

### Preparation of 2-desbenzoyl-2-(3-methylbenzoyl)-10-deacetyl-N-desbenzoyl-N-(t-butoxycarbonyl) taxol.

To a solution of 2-desbenzoyl-2-(3-methylbenzoyl)-10-deacetyl-7,10-bis(triethylsilyl) baccatin III (47.2⁺ mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 70.0 mg of a mixture containing (2'R, 3'S)- 2',7,10-tris(triethylsilyl)-2-desbenzoyl-2-(3-methyl-benzoyl)-10-deacetyl-N-desbenzoyl-N-(t-butoxycarbonyl) taxol and a very small amount of the (2'S,3'R) isomer.

To a solution of 70.0 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 49.3 mg of material which was purified by recrystallization to give 41.9 mg (85%) of 2-desbenzoyl-2-(3-methylbenzoyl)-10-deacetyl-N-desbenzoyl-N-(t-butoxycarbonyl) taxol.
m.p.169-171 °C; [α]²⁵ _{Na}-60.4° (c 0.510, CHCl₃).
¹H NMR (CDCl₃, 300 MHz) δ 7.91 (m, 2H, benzoate), 7,38 (m, 7H, aromatic), 6.21 (m, 1H, H13), 5.65 (d, *J* = 7.2 Hz, 1H, H2β), 5.42 (m, 1H, NH), 5.26 (m, 1H, H3'), 5.20 (d, *J* = 1.2 Hz, 1H, H10), 4.94 (m, 1H, H5), 4.61 (m, 1H, H2'), 4.31 (d, *J* = 8.7 Hz, 1H, H20α), 4.24 (m, 1H, H7), 4.17 (d, *J* = 8.7 Hz, 1H, H20β), 3.91 (d, J= 7.2 Hz, 1H, H3), 3.37 (m, 1H, 2'OH), 2.57 (m, 1H, H6α), 2.43 (s, 3H, 4Ac), 2.26 (m, 2H, H14), 2.17 (s, 3H, methylbenzoate), 1.84 (s, 3H, Me18), 1.79 (m, 1H, H6β), 1.74 (s, 3H, Me19), 1.33 (s, 9H, t-butyl), 1.22 (s, 3H, Me17), 1.12 (s, 3H, Me16).

### EXAMPLE 3

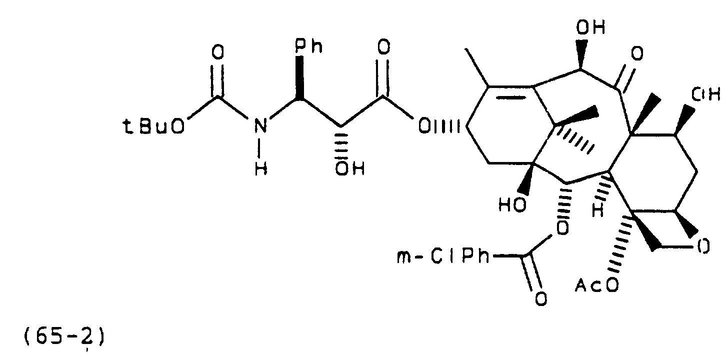

### Preparation of 2-desbenzoyl-2-(3-chlorobenzoyl)-10-deacetyl-N-desbenzoyl-N-(t-butoxycarbonyl) taxol.

To a solution of 2-desbenzoyl-2-(3-chlorobenzoyl)-10-deacetyl-7,10-bis(triethylsilyl) baccatin III (48.4 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 71 mg of a mixture containing (2'R,3'S)-2',7,10-tris(triethylsilyl)-2-desbenzoyl-2-(3-chlorobenzoyl)-10-deacetyl-N-desbenzoyl-N-(t-butoxycarbonyl) taxol and a very small amount of the (2'S,3'R) isomer.

To a solution of 71 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 50.5 mg of material which was purified by recrystallization to give 40.4 mg (80%) of 2-desbenzoyl-2-(3-chlorobenzoyl)-10-deacetyl-N-desbenzoyl-N-(t-butoxycarbonyl) taxol.
m.p.149-150 °C; [α]²⁵ _{Na}-53.3° (c 0.510, CHCl₃).
¹H NMR (CDCl₃, 300 MHz) δ 8.11 (br s, 1H, chlorobenzoate *ortho),* 7.98 (d, *J* = 7.5 Hz, 1H, chlorobenzoate *ortho*), 7.59 (m, 1H, chlorobenzoate), 7.45 (t, *J* = 7.5 Hz, 1H, chlorobenzoate), 7.38 (m, 5H, aromatic), 6.18 (m, 1H, H13), 5.62 (d, *J* = 7.2 Hz, 1H, H2β), 5.41 (m, 1H, H3'), 5.24 (m, 1H, NH), 5.20 (d, *J* = 1.0 Hz, 1H, H10), 4.95 (dd, *J* = 9.3, 1.2 Hz, 1H, H5), 4.59 (m, 1H, H2'), 4.30 (d, *J* = 8.4 Hz, 1H, H20α), 4.23 (m, 1H, H7), 4.15 (d, *J* = 8.4 Hz, 1H, H20β), 3.91 (d, *J*= 7.2 Hz, 1H, H3), 3.35 (m, 1H, 2'OH), 2.58 (m, 1H, H6α), 2.36 (s, 3H, 4Ac), 2.24 (m, 2H, H14), 1.84 (s, 3H, Me18), 1.79 (m, 1H, H6β), 1.75 (s, 3H, Me19), 1.34 (s, 9H, t-butyl), 1.23 (s, 3H, Me17), 1.12 (s, 3H, Me16).

### EXAMPLE 4

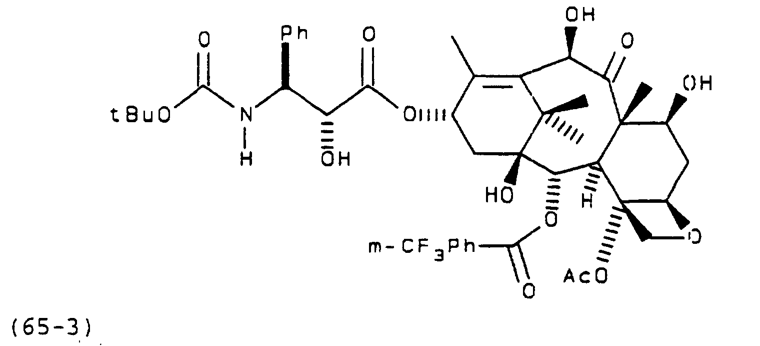

### Preparation of 2-desbenzoyl-2-(3-trifluoromethylbenzoyl)-10-deacetyl-N-desbenzoyl-N-(t-butoxycarbonyl) taxol.

To a solution of 2-desbenzoyl-2-(3-trifluoromethylbenzoyl)-10-deacetyl-7,10-bis(triethylsilyl) baccatin III (50.4 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 73.0 mg of a mixture containing (2'R,3'S)-2',7,10-tris(triethylsilyl)-2-desbenzoyl-2-(3-trifluoromethylbenzoyl)-10-deacetyl-N-desbenzoyl-N-(t-butoxycarbonyl) taxol and a very small amount of the (2'S,3'R) isomer.

To a solution of 73.0 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 52.6 mg of material which was purified by recrystallization to give 41.0 mg (78%) of 2-desbenzoyl-2-(3-trifluoromethylbenzoyl)-10-deacetyl-N-desbenzoyl-N-(t-butoxycarbonyl) taxol.
m.p.140-142 °C; [α]²⁵ _{Na} - 50.4° (c 1.055, CHCl₃).
¹H NMR (CDCl₃, 300 MHz) δ 8.43 (s, 1H, benzoate, *ortho*), 8.29 (d, *J* = 7.8 Hz, 1H, benzoate *ortho*), 7.88 (d, *J* = 7.8 Hz, 1H, benzoate), 7.66 (t, *J* = 7.8 Hz, 1H, benzoate), 7,38 (m, 5H, aromatic), 6.17 (m, 1H, H13), 5.65 (d, *J* = 7.2 Hz, 1H, H2β), 5.38 (m, 1H, NH), 5.23 (m, 1H, H3'), 5.21 (d, *J* = 1.8 Hz, 1H, H10), 4.95 (m, 1H, H5), 4.58 (m, 1H, H2'), 4.27 (d, *J* = 8.7 Hz, 1H, H20α), 4.21 (m, 1H, H7), 4.15 (d, *J* = 8.7 Hz, 1H, H20β), 3.93 (d, J= 7.2 Hz, 1H, H3), 3.35 (m, 1H, 2'OH), 2.59 (m, 1H, H6α), 2.33 (s, 3H, 4Ac), 2.23 (m, 2H, H14), 1.85 (s, 3H, Me18), 1.79 (m, 1H, H6β), 1.76 (s, 3H, Me19), 1.32 (s, 9H, t-butyl), 1.22 (s, 3H, Me17), 1.11 (s, 3H, Me16).

### EXAMPLE 5

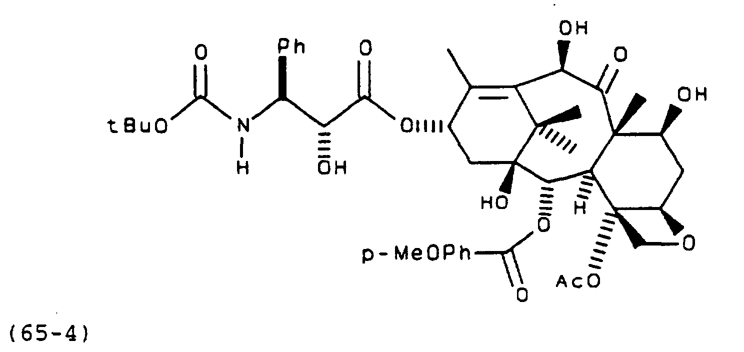

### Preparation of 2-desbenzoyl-2-(4-methoxybenzoyl)-10-deacetyl-N-desbenzoyl-N-(t-butoxycarbonyl) taxol.

To a solution of 2-desbenzoyl-2-(4-methoxybenzoyl)-10-deacetyl-7,10-bis(triethylsilyl) baccatin III (48.2 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 71 mg of a mixture containing (2'R,3'S)- 2',7,10-tris(triethylsilyl)-2-desbenzoyl-2-(4-methoxybenzoyl)-10-deacetyl-N-desbenzoyl-N-(t-butoxycarbonyl) taxol and a very small amount of the (2'S,3'R) isomer.

To a solution of 71 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 50.3 mg of material which was purified by recrystallization to give 45.2 mg (90%) of 2-desbenzoyl-2-(4-methoxybenzoyl)-10-deacetyl-N-desbenzoyl-N-(t-butoxycarbonyl) taxol.
m.p.160-162 °C; [α]²⁵ _{Na} -47.6° (c 0.290, CHCl₃).
¹H NMR (CDCl₃, 300 MHz) δ 8.05 (dd, *J* = 9.0, 2H, methoxybenzoate, *ortho*), 7,38 (m, 5H, aromatic), 6.96 (dd, *J* = 9.0, 2H, methoxybenzoate, *meta*), 6.23 (m, 1H, H13), 5.64 (d, *J* = 7.2 Hz, 1H, H2β), 5.42 (m, 1H, H3'), 5.27 (m, 1H, NH), 5.19 (d, *J* = 1.2 Hz, 1H, H10), 4.93 (dd, *J* = 7.8, 1.8 Hz, 1H, H5), 4.62 (m, 1H, H2'), 4.31 (d, *J* = 9.0 Hz, 1H, H20α), 4.24 (m, 1H, H7), 4.19 (d, *J* = 9.0 Hz, 1H, H20β), 3.89 (d, *J*= 7.2 Hz, 1H, H3), 3.65 (s, 3H, methoxy), 3.32 (m, 1H, 2'OH), 2.58 (m, 1H, H6α), 2.37 (s, 3H, 4Ac), 2.26 (m, 2H, H14), 1.85 (s, 3H, Me18), 1.78 (m, 1H, H6β), 1.75 (s, 3H, Me19), 1.34 (s, 9H, t-butyl), 1.23 (s, 3H, Me17), 1.12 (s, 3H, Me16).

### EXAMPLE 6

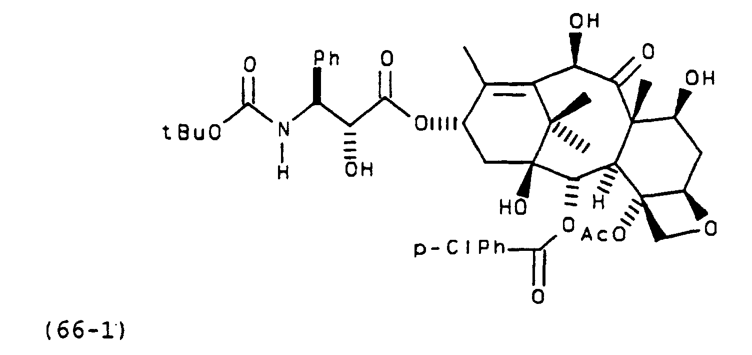

### Preparation of 2-desbenzoyl-2-(4-chlorobenzoyl)-10-deacetyl-N-desbenzoyl-N-(t-butoxycarbonyl) taxol.

To a solution of 2-desbenzoyl-2-(4-chlorobenzoyl)-10-deacetyl-7,10-bis(triethylsilyl) baccatin III (48.4 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis-*1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 71 mg of a mixture containing (2'R,3'S)-2',7,10-tris(triethylsilyl)-2-desbenzoyl-2-(4-chlorobenzoyl)-10-deacetyl-N-desbenzoyl-N-(t-butoxycarbonyl) taxol and a very small amount of the (2'S,3'R) isomer.

To a solution of 71 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 51 mg of material which was purified by recrystallization to give 37.9 mg (75%) of 2-desbenzoyl-2-(4-chlorobenzoyl)-10-deacetyl-N-desbenzoyl-N-(t-butoxycarbonyl) taxol.
m.p.160-161 °C; [α]²⁵ _{Na} -46.0° (c 0.104, CHCl₃).
¹H NMR (CDCl₃, 300 MHz) δ 8.03 (d, *J* = 8.7 Hz, 2H, chlorobenzoate *ortho),* 7.48 (d, *J* = 8.7 Hz, 2H, chlorobenzoate *meta*), 7.38 (m, 5H, aromatic), 6.23 (m, 1H, H13), 5.64 (d, *J* = 7.2 Hz, 1H, H2β), 5.45 (m, 1H, H3'), 5.26 (m, 1H, NH), 5.20 (d, *J* = 1.2 Hz, 1H, H10), 4.93 (d, *J* = 7.8 Hz, 1H, H5), 4.63 (m, 1H, H2'), 4.28 (d, J= 8.2 Hz, 1H, H20α), 4.22 (m, 1H, H7), 4.15 (d, *J* = 8.2 Hz, 1H, H20β), 3.90 (d, J= 7.2 Hz, 1H, H3), 3.36 (m, 1H, 2'OH), 2.58 (m, 1H, H6α), 2.37 (s, 3H, 4Ac), 2.25 (m, 2H, H14), 1.85 (s, 3H, Me18), 1.80 (m, 1H, H6β), 1.75 (s, 3H, Me19), 1.32 (s, 9H, t-butyl), 1.23 (s, 3H, Me17), 1.11 (s, 3H, Me16).

### EXAMPLE 7

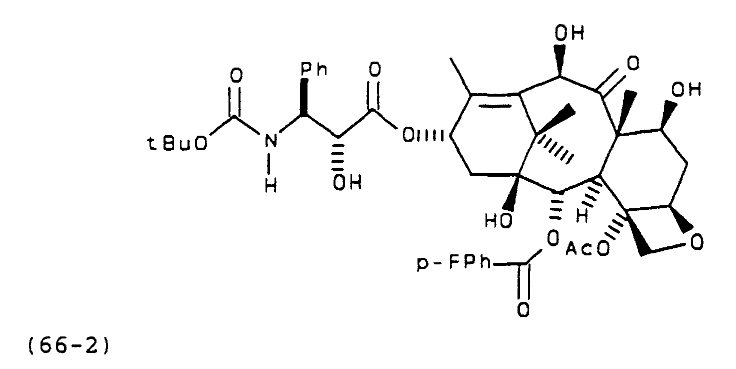

### Preparation of 2-desbenzoyl-2-(4-fluorobenzoyl)-10-deacetyl-N-desbenzoyl-N-(t-butoxycarbonyl) taxol.

To a solution of 2-desbenzoyl-2-(4-fluorobenzoyl)-10-deacetyl-7,10-bis(triethylsilyl) baccatin III (47.5 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 70 mg of a mixture containing (2'R,3'S)- 2',7,10-tris(triethylsilyl)-2-desbenzoyl-2-(4-fluorobenzoyl)-10-deacetyl-N-desbenzoyl-N-(t-butoxycarbonyl) taxol and a very small amount of the (2'S,3'R) isomer.

To a solution of 70 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 49.5 mg of material which was purified by recrystallization to give 42.0 mg (85%) of 2-desbenzoyl-2-(4-fluorobenzoyl)-10-deacetyl-N-desbenzoyl-N-(t-butoxycarbonyl) taxol.
m.p. 158-160 °C; [α]²⁵ _{Na} -47.6° (c 0.290, CHCl₃).
¹H NMR (CDCl₃, 300 MHz) δ 8.13 (m, 23, fluorobenzoate *ortho*), 7.38 (m, 5H, aromatic), 7.17 (m, 2H, fluorobenzoate), 6.23 (m, 1H, H13), 5.64 (d, *J* = 7.2 Hz, 1H, H2β), 5.41 (d, J=9.9 Hz, 1H, H3'), 5.26 (m, 1H, NH), 5.20 (d, *J* = 1.2 Hz, 1H, H10), 4.93 (dd, *J* = 9.9, 2.1 Hz, 1H, H5), 4.63 (m, 1H, H2'), 4.28 (d, *J*= 8.2 Hz, 1H, H20α), 4.24 (m, 1H, H7), 4.17 (d, *J* = 8.2 Hz, 1H, H20β), 3.91 (d, J= 7.2 Hz, 1H, H3), 3.32 (m, 1H, 2'OH), 2.58 (m, 1H, H6α), 2.37 (s, 3H, 4Ac), 2.25 (m, 2H, H14), 1.85 (s, 3H, Me18), 1.80 (m, 1H, H6β), 1.75 (s, 3H, Me19), 1.33 (s, 9H, t-butyl), 1.25 (s, 3H, Me17), 1.12 (s, 3H, Me16).

### EXAMPLE 8

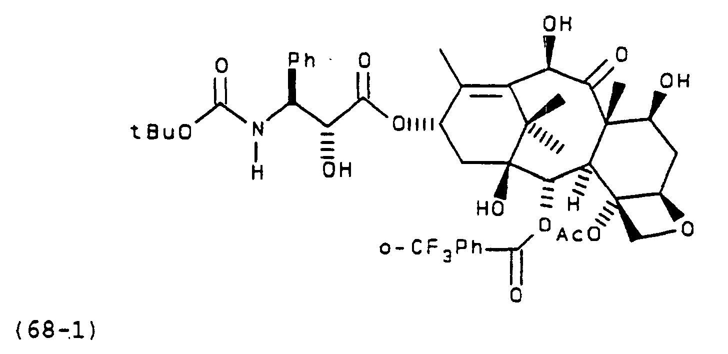

### Preparation of N-desbenzoyl-N-(t-butoxycarbonyl)-2-desbenzoyl-2-(2-trifluoromethylbenzoyl)-10-desacetyl taxol.

To a solution of 2-desbenzoyl-2-(2-trifluoromethylbenzoyl)-10-deacetyl-7,10-(bis)-O-triethylsilyl baccatin III (50.4 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 73.0 mg of a mixture containing (2'R,3'S)-2',7,10-(tris)-O-triethylsilyl-N-desbenzoyl-N-(t-butoxycarbonyl)-2-desbenzoyl-2-(2-trifluoromethylbenzoyl)-10-desacetyl taxol and a very small amount of the (2'S,3'R) isomer.

To a solution of 73.0 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 52.6 mg of material which was purified by recrystallization to give 39.4 mg (75%) of N-desbenzoyl-N-(t-butoxycarbohyl)-2-desbenzoyl-2-(2-trifluoromethylbenzoyl)-10-desacetyl taxol.
m.p.121-123 °C; [α]²⁵ _{Na}-34.2° (c 0.760, CHCl₃).
¹H NMR (CDCl₃, 300 MHz) δ 8.10 (m, 1H, benzoate, *ortho*), 7.82 (d, *J* = 7.5 Hz, 1H, benzoate), 7.70 (m, 2H, benzoate), 7,35 (m, 5H, aromatic), 6.24 (m, 1H, H13), 5.64 (d, *J* = 7.2 Hz, 1H, H2β), 5.46 (m, 1H, NH), 5.28 (m, 1H, H3'), 5.19 (d, *J* = 1.8 Hz, 1H, H10), 4.89 (dd, *J* = 8.7, 1.2 Hz, 1H, H5), 4.63 (m, 1H, H2'), 4.26 (d, *J* = 8.1 Hz, 1H, H20α), 4.17 (m, 2H, H7, H20β), 3.90 (d, J= 7.2 Hz, 1H, H3), 3.35 (m, 1H, 2'OH), 2.56 (m, 1H, H6α), 2.39 (m, 2H, H14), 2.24 (s, 3H, 4Ac), 1.87 (s, 3H, Me18), 1.84 (m, 1H, H6β), 1.76 (s, 3H, Me19), 1.38 (s, 9H, t-butyl), 1.24 (s, 3H, Me17), 1.11 (s, 3H, Me16).

### EXAMPLE 9

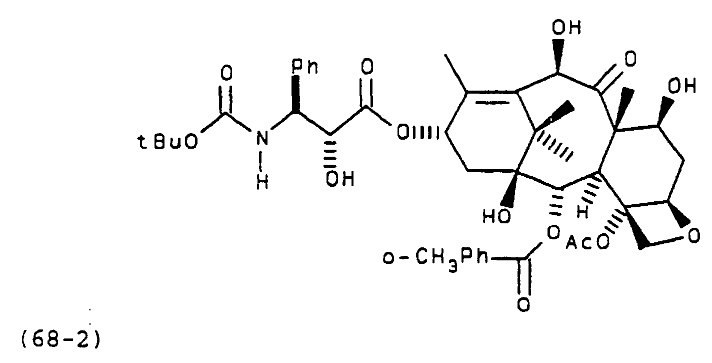

### Preparation of N-desbenzoyl-N-(t-butoxycarbonyl)-2-desbenzoyl-2-(2-methylbenzoyl)-10-desacetyl taxol.

To a solution of 2-desbenzoyl-2-(2-methylbenzoyl)-10-desacetyl-7,10-(bis)-O-triethylsilyl baccatin III (47.2 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 70.0 mg of a mixture containing (2'R,3'S)- 2',7,10-(tris)-O-triethylsilyl-N-desbenzoyl-N-(t-butoxycarbonyl)-2-desbenzoyl-2-(2-methylbenzoyl)-10-desacetyl taxol and a very small amount of the (2'S,3'R) isomer.

To a solution of 70.0 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 49.3 mg of material which was purified by recrystallization to give 44.4 mg (90%) of 2-desbenzoyl-2-(2-methylbenzoyl)-10-deacetyl-N-desbenzoyl-N-(t-butoxycarbonyl) taxol.
m.p.129-131 °C; [α]²⁵ _{Na} -50.8° (c 0.750, CHCl₃).
¹H NMR (CDCl₃, 300 MHz) δ 8.05 (m, 1H, benzoate), 7,38 (m, 8H, aromatic), 6.21 (m, 1H, H13), 5.65 (d, *J* = 6.6 Hz, 1H, H2β), 5.46 (m, 1H, NH), 5.24 (m, 1H, H3'), 5.20 (d, *J* = 0.9 Hz, 1H, H10), 4.91 (dd, *J* = 9.3, 1.5 Hz, 1H, H5), 4.60 (br s, 1H, H2'), 4.25 (d, *J* = 8.1 Hz, 1H, H20α), 4.24 (m, 1H, H7), 4.17 (d, *J* = 8.1 Hz, 1H, H20β), 3.88 (d, J= 6.6 Hz, 1H, H3), 3.37 (m, 1H, 2'OH), 2.63 (s, 3H, methylbenzoate), 2.57 (m, 1H, H6α), 2.30 (s, 3H, 4Ac), 2.58 (m, 2H, H14), 1.83 (s, 3H, Me18), 1.79 (m, 1H, H6β), 1.75 (s, 3H, Me19), 1.37 (s, 9H, t-butyl), 1.24 (s, 3H, Me17), 1.13 (s, 3H, Me16).

### EXAMPLE 10

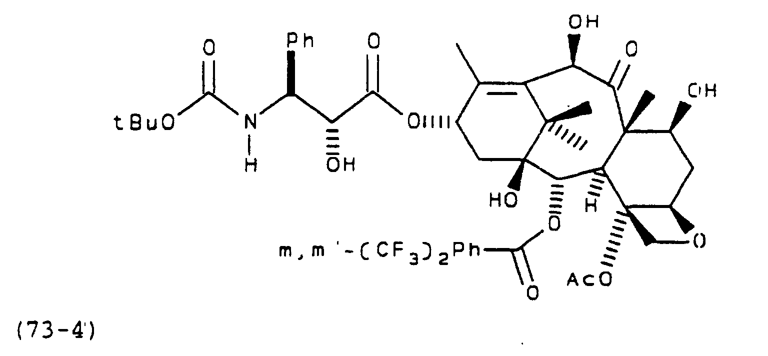

### Preparation of N-desbenzoyl-N-(t-butoxycarbonyl)-2-desbenzoyl-2-(3,5-bis(trifluoromethyl)benzoyl)-10-desacetyl taxol.

To a solution of 2-desbenzoyl-2-(3,5-bis(trifluoromethyl)benzoyl)-7,10-(bis)-O-triethylsilyl-10-desacetyl baccatin III (51.3 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give' 73.9 mg of a mixture containing (2'R,3'S)-2',7,10-(tris)-O-triethylsilyl-N-desbenzoyl-N-(t-butoxycarbonyl)-2-desbenzoyl-2-(3,5-bis(trifluoromethyl)benzoyl)-10-desacetyl taxol and a very small amount of the (2'S,3'R) isomer.

To a solution of 73.9 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 53.4 mg of material which was purified by recrystallization to give 49.1 mg (92%) of N-desbenzoyl-N-(t-butoxycarbonyl)-2-desbenzoyl-2-(3,5-bis(trifluoromethyl)-benzoyl)-10-desacetyl taxol.
m.p.141-143 °C; [α]²⁵ _{Na} -43.6° (c 0.730, CHCl₃).
¹H NMR (CDCl₃, 300 MHz) δ 8.59 (s, 2H, benzoate, ortho), 8.12 (s, 1H, benzoate *para*), 7.37 (m, 5H, aromatic), 6.14 (m, 1H, H13), 5.64 (d, *J* = 7.2 Hz, 1H, H2β), 5.36 (m, 1H, NH); 5.21 (d, *J* = 1.2 Hz, 1H, H10), 5.18 (m, 1H, H3'), 4.97 (dd, *J* = 9.6, 2.1 Hz, 1H, H5), 4.58 (m, 1H, H2'), 4.19 (m, 3H, H20, H7), 3.95 (d, J= 7.2 Hz, 1H, H3), 3.39 (m, 1H, 2'OH), 2.59 (m, 1H, H6α), 2.30 (s, 3H, 4Ac), 2.25 (m, 2H, H14), 1.85 (s, 3H, Me18), 1.79 (m, 1H, H6β), 1.75 (s, 3H, Me19), 1.32 (s, 9H, t-butyl), 1.22 (s, 3H, Me17), 1.13 (s, 3H, Me16).

### EXAMPLE 11

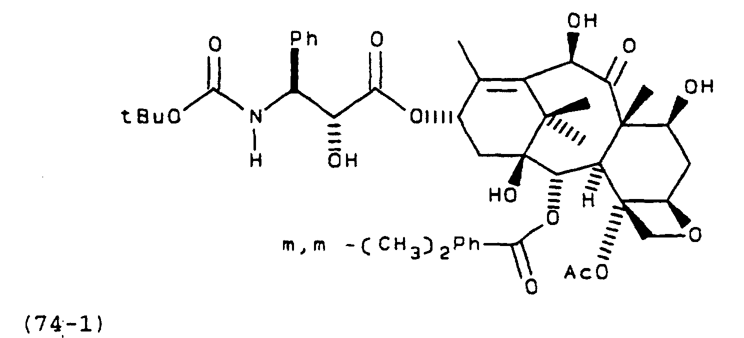

### Preparation of N-desbenzoyl-N-(t-butoxycarbonyl)-2-desbenzoyl-2-(3,5-dimethylbenzoyl)-10-desacetoxy taxol.

To a solution of 2-desbenzoyl-2-(3,5-dimethylbenzoyl)-7,10-(bis)-O-triethylsilyl-10-desacetyl baccatin III (48.1 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel to give 70.1 mg of a mixture containing (2'R,3'S)-2',7,10-(tris)-O-triethylsilyl-N-desbenzoyl-N-(t-butoxycarbonyl)-2-desbenzoyl-2-(3,5-dimethylbenzoyl)-10-desacetoxy taxol and a very small amount of the (2'S;3'R) isomer.

To a solution of 70.1 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitiored between saturated aqueous sodium bicarbonate and ethyl acetate. Evaporation of the ethyl acetate sclution gave 50.2 mg of material which was purified by recrystallization to give 45.1 mg (90%) of N-desbenzoyl-N-(t-butoxycarbonyl)-2-desbenzoyl-2-(3,5-dimethylbenzoyl)-10- desacetoxy taxol.
m.p.198-200 °C; [α]²⁵ _{Na} - 49.0° (c 0.965, CHCl₃).
¹H NMR (CDCl₃, 300 MHz) δ 7.72 (s, 2H, benzoate, *ortho*), 7,37 (m, 5H, aromatic), 7.23 (s, 1H, benzoate, *para),* 6.21 (m, 1H, H13), 5.64 (d, *J* = 7.2 Hz, 1H, H2β), 5.45 (m, 1H, NH), 5.25 (m, 1H, H3'), 5.20 (d, *J* = 1.8 Hz, 1H, H10), 4.94 (dd, *J* = 9.3, 1.2 Hz, 1H, H5), 4.61 (m, 1H, H2'), 4.32 (d, *J* = 8.1 Hz, 1H, H20α), 4.21 (m, 1H, H7), 4.16 (d, *J* = 8.7 Hz, 1H, H20β), 3.89 (d, J= 7.2 Hz, 1H, H3), 3.39 (m, 1H, 2'OH), 2.58 (m, 1H, H6α), 2.38 (s, 6H, dimethylbenzoate), 2.36 (s, 3H, 4Ac), 2.27 (m, 2H, H14), 1.88 (m, 1H, H6β), 1.83 (s, 3H, Me18), 1.74 (s, 3H, Me19), 1.33 (s, 9H, t-butyl), 1.22 (s, 3H, Me17), 1.12 (s, 3H, Me16).

### EXAMPLE 12

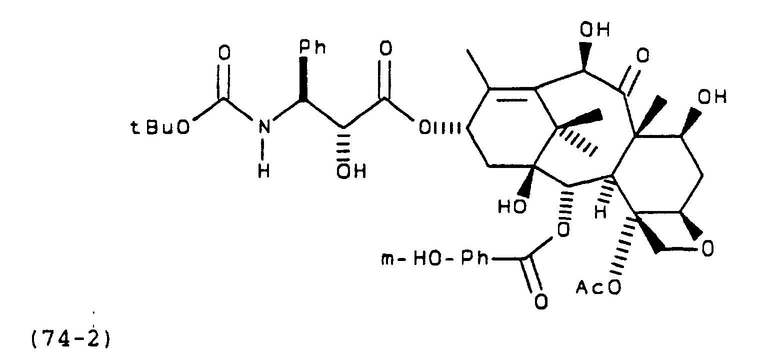

### Preparation of N-desbenzoyl-N-(t-butoxycarbonyl)-2-desbenzoyl-2-(3-hydroxybenzoyl)-10-desacetyl taxol.

To a solution of 2-desbenzoyl-2-(3-triethylsilyloxybenzoyl)-7,10-(bis)-O-triethylsilyl-10-desacetyl baccatin III (54.1 mg, 0.060 mmol) in 0.5 mL of THF at -45 °C was added dropwise 0.066 mL of a 1.00 M solution of LiN(SiMe₃)₂ in hexane. After 0.5 h at -45 °C, a solution of *cis*-1-(t-butoxycarbonyl)-3-triethylsilyloxy-4-phenylazetidin-2-one (90 mg, 0.240 mmol) in 0.5 mL of THF was added dropwise to the mixture. The solution was warmed to 0 °C and kept at that temperature for 1 h before 0.5 mL of a 10% solution of ACOH in THF was added. The mixture was partitioned between saturated aqueous NaHCO₃ and 60/40 ethyl acetate/hexane. Evaporation of the organic layer gave a residue which was purified by filtration through silica gel to give 76.7 mg of a mixture containing (2'R,3'S)-2',7,10-tris(triethylsilyl)-N-debenzoyl-N-(t-butoxycarbonyl)-2-desbenzoyl-2-(3-triethylsilyloxybenzoyl)-10-desacetyl taxol and a very small amount of the (2'S,3'R) isomer.

To a solution of 76.7 mg of the mixture obtained from the previous reaction in 4 mL of acetonitrile and 0.19 mL of pyridine at 0 °C was added 0.52 mL of 48% aqueous HF. The mixture was stirred at 0 °C for 3 h, then at 25 °C for 13 h, and partitioned between saturated aqueous sodium bicarbonate and ethyl acetate. Evaporation of the ethyl acetate solution gave 49.4 mg of material which was purified by recrystallization to give 43.4 mg (88%) of N-desbenzoyl-N-(t-butoxycarbonyl)-2-desbenzoyl-2-(3-hydroxybenzoyl)-10-desacetyl taxol.
m.p.153-155 °C [α]²⁵ _{Na} -45.0° (c 0.560, CHCl₃).
¹H NMR (CDCl₃, 300 MHz) δ 7.36 (m, 9H, aromatic), 7.10 (m, 1H, OH), 6.38 (m, 1H, H13), 5.60 (d, *J* = 9.9 Hz, NH), 5.53. (d, *J* = 7.5 Hz, 1H, H2β), 5.37 (m, 1H, H3'), 5.18 (d, *J* = 1.2 Hz, 1H, H10), 4.90 (dd, *J* = 9.9, 2.4 Hz, 1H, H5), 4.75 (m, 1H, H2'), 4.29 (d, *J* = 8.4 Hz, 1H, H20α), 4.24 (m, 2H, H7, H20β), 3.93 (d, J= 7.5 Hz, 1H, H3), 3.29 (m, 1H, 2'OH), 2.56 (m, 1H, H6α), 2.36 (s, 3H, 4Ac), 2.27 (m, 2H, H14), 1.91 (s, 3H, Me18), 1.85 (m, 1H, H6β), 1.76 (s, 3H, Me19), 1.33 (s, 9H, t-butyl), 1.24 (s, 3H, Me17), 1.08 (s, 3H, Me16).

### EXAMPLE 13

Compounds 64-4, 65-1, 65-2, 65-3, 65-4, 66-1, 66-2, 68-1, 68-2, 73-4, 74-1 and 74-2 of Examples 1-12 were evaluated in in vitro cytotoxicity activity against human colon carcinoma cells HCT-116. Cytotoxicity was assessed in HCT116 cells by XTT (2,3-bis(2-methoxy-4-nitro-5-sulfophenyl)-5-[(phenylamino)carbonyl]-2H-tetrazolium hydroxide) assay (Scudiero et al, "Evaluation of a soluble tetrazolium/formazan assay for cell growth and drug sensitivity in culture using human and other tumor cell lines", Cancer Res. 48:4827-4833, 1988). Cells were plated at 4000 cells/well in 96 well microtiter plates and 24 hours later drugs were added and serial diluted. The cells were incubated at 37°C for 72 hours at which time the tetrazolium dye, XTT, was added. A dehydrogenase enzyme in live cells reduces the XTT to a form that absorbs light at 450 nm which can be quantitated spectrophotometrically. The greater the absorbance the greater the number of live cells. The results are expressed as an IC₅₀ which is the drug concentration required to inhibit cell proliferation (i.e. absorbance at 450 nm) to 50% of that of untreated control cells.

All compounds had an IC₅₀ less than 0.5 and all except compound 65-4 (Example 5) had an IC₅₀ less than 0.1 indicating that they are cytotoxically active.

### EXAMPLE 14

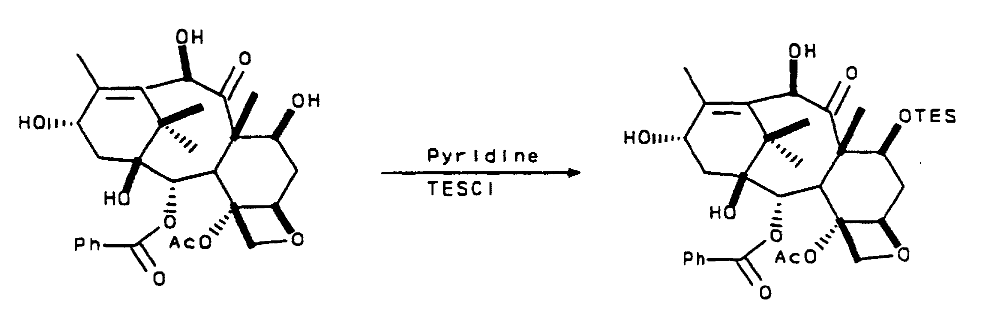

### Protection of 10-deacetyl Baccatin (III) at C7, C10, and C13

**7-O-Triethylsilyl-10-deacetyl baccatin (III).** To a solution of 10-deacetyl baccatin (III) (1.5 g, 2.8 mmol) in 100 mL of pyridine was added 4.7 mL (10 eq) of triethylsilyl chloride (TESC1) and the mixture was stirred for 24 h at 25°C. The reaction mixture was diluted with EtOAc (800 mL) and washed with H₂O (2 x 200 mL) and 10% aqueous CuSO₄ until all pyridine was removed. The organic layer was washed with brine (50.0 mL), dried over anhydrous Na₂SO₄, filtered, and concentrated to give crude product (1.92 g). Plug filtration from 20% EtOAc in hexane to 50% EtOAc in hexane gave 7-O-triethylsilyl-10-deacetyl baccatin (III) (1.78 g, 97.7%). m.p. 257-258°C, [α]²⁵ _{Na} -23.8° (c 0.5, CHCl₃), ¹H NMR (CDCl₃, 300 MHz), δ 8.10 (d, J = 7.1 Hz, 2H, benzoate ortho), 7.63-7.45 (m, 3H, aromatic), 5.60 (d, J = 7.2 Hz, 1H, H2), 5.17 (d, J = 1.7 Hz, 1H, H10), 4.95 (dd, J = 1.7, 9.9 Hz, 1H, H5), 4.88 (m, 1H, H13), 4.41 (dd, J = 6.6, 10.4 Hz, 1H, H7), 4.31 (d, J = 8.2 Hz, 1H, H20α), 4.16 (d, J = 8.2 Hz, 1H, H20β), 3.95 (d, J = 7.1 Hz, 1H, H3), 2.49 (m, 1H, H6α), 2.28 (s, 3H, 4Ac), 2.10-2.09 (m, 2H, H14α, H14β), 2.08 (s, 3H, Me18), 1.90 (m, 1H, H6β), 1.73 (s, 3H, Me19), 1.19 (s, 3H, Me17), 1.08 (s, 3H, Me16), 1.02-0.93 (m, 9H, SiCH₂CH₃), 0.59-0.51 (m, 6H, SiCH₂CH₃).

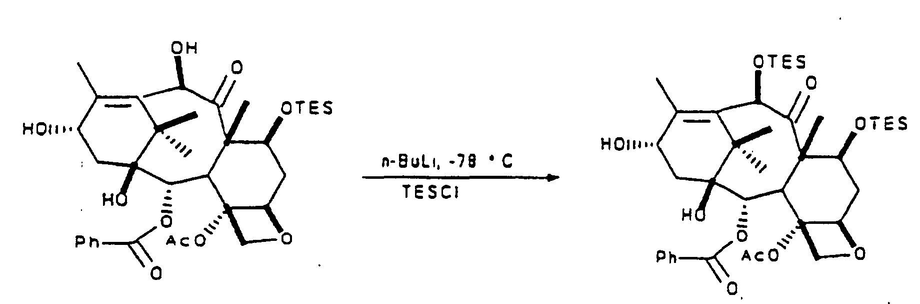

**7,10-Bis-O-triethylsilyl-10-deacetyl baccatin (III).** To a solution of 7-O-triethylsilyl-10-deacetyl baccatin (III) (1.0 g, 1.55 mmol) in 20 mL of THF at -78°C under N₂ was added 1.04 mL of a 1.64 M solution of n-butyllithium (1.1 equiv) in hexane. The mixture was stirred for 30 min at -78°C and 0.31 mL (1.2 equiv) of TESC1 was added dropwise. The mixture was stirred for 1h at -78°C and 10 mL of saturated aqueous NaHCO₃ was added. The solution was diluted with EtOAc (80.0 mL). The organic phase was washed with brine (15.0 mL), dried over anhydrous Na₂SO₄, filtered and concentrated under reduced pressure to give a crude solid (1.45 g). Flash chromatography from 25% EtOAc in hexane to 50% EtOAc in hexane gave 7,10-bis-O-triethylsilyl-10-dcacetyl baccatin (III) (0.63 g, 53.6%) and recovered 7-O-triethylsilyl-10-deacetyl baccatin (III) (0.35 g, 35.0%). m.p. 184-186° C, [α]²⁵ _{Na} -46.0° (c 0.5, CHCl₃), ¹H NMR (CDCl₃, 300 MHz) δ 8.10 (d, J = 6.6 Hz, 2H, benzoate ortho), 7.6-7.4 (m, 3H, aromatic), 5.61 (d, J = 7.1 Hz, 1H, H2), 5.21 (s, 1H, H10), 4.93 (dd, J = 1.7, 9.3 Hz, 1H, H5), 4.82 (m, 1H, H13), 4.42 (dd, J = 6.6, 10.4 Hz, 1H, H7), 4.27 (d, J = 8.2 Hz, 1H, H20α), 4.14 (d, J = 8.2 Hz, 1H, H20β), 3.91 (d, J = 6.6 Hz, 1H, H3), 2.53 (m, 1H, H6α), 2.27 (s, 3H, 4Ac), 2.25 (m, 2H, H14α, H14β), 2.03 (s, 3H, Me18), 1.85 (m, 1H, H6β), 1.64 (s, 3H, Me19), 1.18 (s, 3H, Me17), 1.04 (s, 3H, Me16), 1.02-0.85 (m, 18H, SiCH₂CH₃), 0.69-0.58 (m, 12H, SiCH₂CH₃).

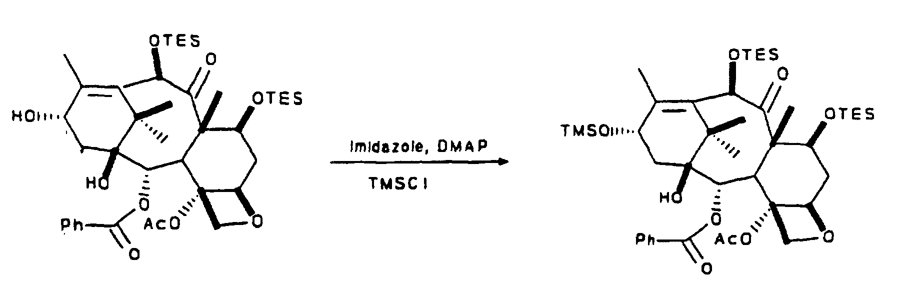

**13-O-Trimethylsilyl-7,10-bis-O-triethylsilyl-10-deacetyl baccatin (III).** To a solution of 0.5 g (0.66 mmol) of 7,10-bis-O-triethylsilyl-10-deacetyl baccatin (III), 90 mg (2 eq) of imidazole, 40 mg (0.5 eq) of *p*-dimethylaminopyridine (DMAP) in 15 mL of CH₂Cl₂ at 0° C was added 0.17 mL (2 eq) of trimethylsilyl chloride (TMSCl). The solution was stirred at 0° C for 30 min and 1.0 mL of methanol was added. The mixture was diluted with H₂O (10.0 mL) and EtOAc (50.0 mL) and the organic layer was separated, washed with brine (10.0 mL), dried over anhydrous Na₂SO₄, filtered and concentrated under reduced pressure to afford a crude solid (0.58 g). Plug filtration with 10% EtOAc in hexane gave 13-O-trimethylsilyl-7,10-bis-O-triethylsilyl-10-deacetyl baccatin (III) (0.53 g, 96.5%). m.p. 213-215° C, [α]²⁵ _{Na} -43.0° (c 0.5, CHCl₃), ¹H NMR (CDCl₃, 300 MHz), δ 8.10 (d, J = 7.1 Hz, 2H, benzoate ortho), 7.6-7.4 (m, 3H, aromatic), 5.62 (d, J = 7.1 Hz, 1H, H2), 5.19 (s, 1H, H10), 4.94 (dd, J = 1.8, 8.8 Hz, 1H, H5), 4.86 (m, 1H, H13), 4.41 (dd, J = 6.6, 10.4 Hz, 1H, H7), 4.28 (d, J = 8.2 Hz, 1H, H20α), 4.12 (d, J = 8.2 Hz, 1H, H20β), 3.86 (d, J = 7.14 Hz, 1H, H3), 2.51 (m, 1H, H6α), 2.26 (s, 3H, 4Ac), 2.22-2.03 (m, 2H, H14α, H14β), 1.93 (s, 3H. Me18), 1.84 (m, 1H. H6β), 1.64 (s, 3H, Me19), 1.19 (s, 3H, Me17), 1.12 (s, 3H, Me16), 1.02-0.93 (m, 18H, SiCH₂CH₃), 0.69-0.56 (m, 12H, SiCH₂CH₃), 0.17 (s, 9H, SiCH₃).

### EXAMPLE 15

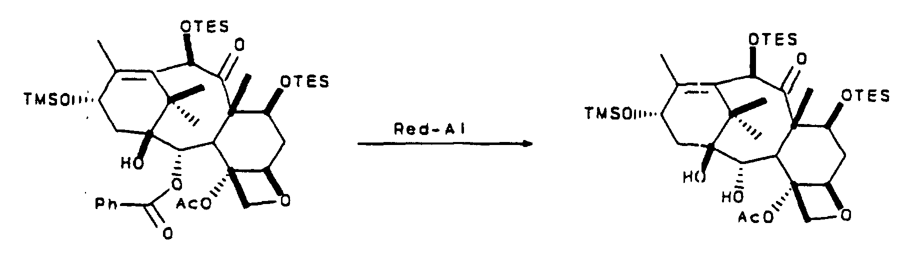

### Preparation of Taxol Analogs With Various Substituents At C-2

### a. 13-O-Trimethylsilyl-7,10-bis-O-triethylsilyl-2-debenzoyl-2-acetyl-10-deacetyl baccatin (III).

**13-O-Trimethylsilyl-7,10-bis-O-triethylsilyl-2-debenzoyl-10-deacetyl baccatin (III).** To a solution of 13-O-trimethylsilyl-7,10-bis-O-triethylsilyl10-deacetyl baccatin (III) (0.1 g, 0.12 mmol) in THF (6.0 mL) at 0° C was added dropwise 60 µL of a 1.0 M solution of Red-Al in toluene. The resulting mixture was stirred at 0° C for 1 h and 3.0 mL of saturated aqueous NaHCO₃ was added. The solution was filtered and the solid was rinsed with EtOAc. The filtrate was concentrated under reduced pressure and diluted with EtOAc (50.0 mL). The organic layer was separated and washed with brine (5.0 mL). The combined organic layer was dried over anhydrous Na₂SO₄, filtered, and concentrated under reduced pressure to give a crude solid (0.14 g). Flash chromatography with 30% EtOAc in hexane gave pure 13-O-trimethylsilyl-7,10-bis-O-triethylsilyl-2-debenzoyl-10-deacetyl baccatin (III) (84.5 mg, 96.6%). m.p. 73-74° C, [α]²⁵ _{Na} -24.0° (c 0.5, CHCl₃), ¹H NMR (CDCl₃, 300 MHz), δ 5.11 (s, 1H, H10), 4.94 (dd, J = 1.7, 9.3 Hz, 1H, H5), 4.87 (m, 1H, H13), 4.62 (d, J = 9.3 Hz, 1H, H20α), 4.54 (d, J = 8.8 Hz, 1H, H20β), 4.35 (dd, J = 6.6, 10.4 Hz, 1H, H7), 3.86 (m, 1H, H2), 3.47 (d, J = 6.6 Hz, 1H. H3), 2.51 (m, 1H, H6α,), 2.14 (s, 3H, 4Ac), 2.02-1.83 (m, 3H, H14α, H14β, H6β), 1.60 (s, 3H, Me18), 1.60 (s, 3H, Me19), 1.14 (s, 3H, Me17), 1.07 (s, 3H, Me16). 0.99-0.92 (m, 18H. SiCH₂CH₃), 0.66-0.55 (m, 12H, SiCH₂CH₃), 0.13 (s, 9H. SiCH₂).

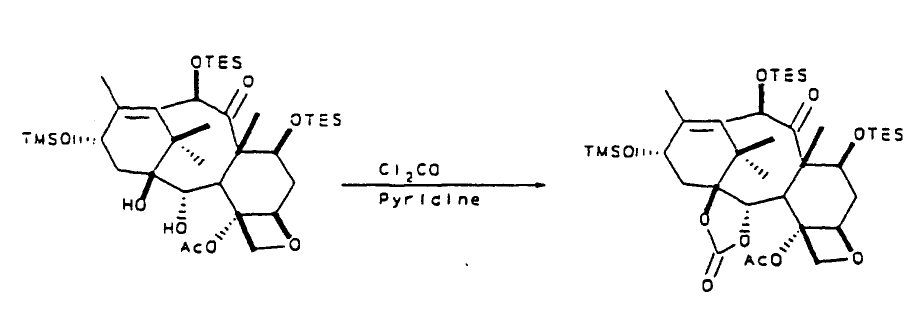

**13-O-Trimethylsilyl-7,10-bis-O-triethylsilyl-2-debenzoyl-10-deacetyl baccatin (III) 1,2-carbonate.** To a solution of 13-O-trimethylsilyl-7,10-bis-O-triethylsilyl-2-debenzoyl-10-deacetyl baccatin (III) (20.0 mg, 0.027 mmol) in CH₂Cl₂ (4.0 mL.) and pyridine (0.8 mL) at -78° C was added 80 µL of a 3.4 M solution of COCl₂ in benzene (10 eq). The mixture was warmed to -10° C (ice-acetone) and kept for 30 min at -10° C. Saturated aqueous NaHCO₃ (5.0 mL) was added and the mixture was extracted with EtOAc (3 x 10 mL). The organic layer was washed with aqueous 10% CuSO₄ until all pyridine disappeared then brine (5.0 mL). The combined organic layer was dried over anhydrous Na₂SO₄, filtered and concentrated under reduced pressure to give a crude solid (22.5 mg). Plug filtration with 20% EtOAc in hexane gave pure 13-O-trimethylsilyl-7,10-bis-O-triethylsilyl-2-debenzoyl-10-deacetyl baccatin (III) 1,2-carbonate (20.5 mg, 99.0%). m.p. 144-146° C, [α]²⁵ _{Na} -27.5° (c 0.5, CHCl₃), ¹H NMR (CDCl₃, 300 MHz), δ 5.15 (s, 1H, H10), 4.90 (m, 2H, H5, H13), 4.58 (d, J = 8.9 Hz, 1H, H20α), 4.44 (d, J = 8.6 Hz, 1H, H20β), 4.43 (d, J = 5.4 Hz, 1H, H2), 4.37 (dd, J = 6.6, 10.4 Hz, 1H, H7), 3.43 (d, J = 5.6 Hz, 1H, H3), 2.56 (m, 1H, H6α). 2.37 (m, 1H, 14α), 2.14 (s, 3H. 4Ac), 2.13 (m, 1H, H14β), 1.92 (s, 3H, Me18), 1.84 (m, 1H, H6β), 1.64 (s, 3H, Me19), 1.22 (s, 3H, Me17), 1.17 (s, 3H, Me16), 0.99-0.85 (m, 18H, SiCH₂CH₃), 0.66-0.55 (m, 12H, SiCH₂CH₃), 0.17 (s, 9H, SiCH₃).

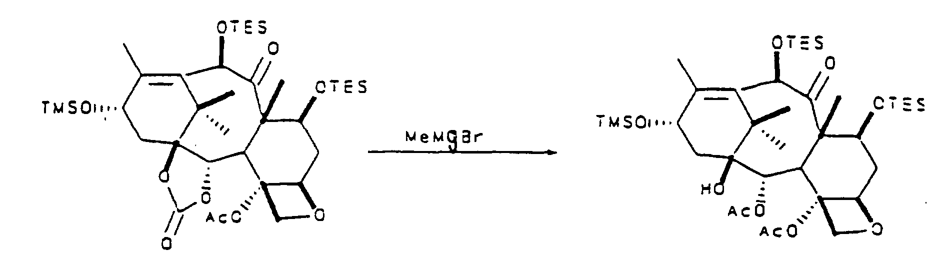

**13-O-trimethylsilyl-7,10-bis-O-triethylsilyl-2-debenzoyl-2-acetyt-10-deacetyl baccatin (III).** To a solution of 13-O-trimethylsilyl-7,10-bis-O-triethylsilyl-2-debenzoyl-10-deacetyl baccatin (III) 1,2-carbonate (10.0 mg, 0.014 mmol) in THF (0.5 mL) at 0° C was added 40 µL of a 3.4 M solution (10 eq) of MeMgBr in ether. The solution was stirred for 1 h at 0° C under N₂ and saturated aqueous NaHCO₃ was added (1.0 mL). The mixture was extracted with EtOAc (3 x 5.0 mL) and the organic layer was washed with brine (5.0 mL), dried over anhydrous Na₂SO₄, filtered and concentrated under reduced pressure to give a crude solid (11.3 mg). Flash chromatography with 20% EtOAc in hexane gave pure 13-O-trimethylsilyl-7,10-bis-O-triethylsilyl-2-debenzoyl-2acetyl-10-deacetyl baccatin (III) (9.8 mg, 95.9%). m.p. 201-203° C, [α] ²⁵ _{Na} -38.9° (c 0.5, CHCl₃), ¹H NMR (CDCl₃, 300 MHz), δ 5.34 (d, J = 7.2 Hz, 1H, H2), 5.15 (s, 1H, H10), 4.93 (dd, J = 2.8, 9.3 Hz, 1H, H5), 4.83 (m, 1H, H13), 4.43 (d, J = 7.7 Hz, 1H, H20α), 4.38 (d, J = 7.1 Hz, 1H, H20β), 4.18 (dd, J = 6.1, 11.6 Hz, 1H, H7), 3.73 (d, J = 6.6 Hz, 1H, H3), 2.54 (m, 1H, H6α, 2.20-2.03 (m, 2H, H14α, H14β), 2.15 (s, 3H, 4Ac), 2.07 (s, 3H, 2Ac), 1.96 (m, 1H, H6β), 1.89 (s, 3H, Me18), 1.58 (s, 3H, Me19), 1.12 (s, 3H, Me17), 1.00 (s, 3H. Me16), 0.99-0.91 (m, 18H, SiCH₂CH₃), 0.67-0.56 (m, 12H, SiCH₂CH₃), 0.16 (s, 9H. SiCH₃).

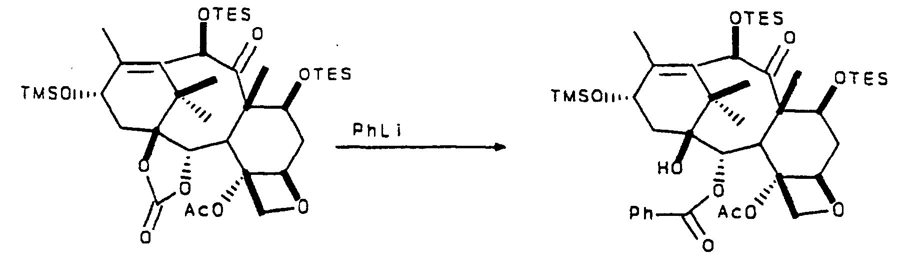

### b. 13-O-Trimethylsilyl-7,10-bis-O-triethylsilyl-2-debenzoyl-10-deacetyl baccatin (III)

To a solution of 13-O-trimethylsilyl-7,10-bis-O-triethylsilyl-2-debenzoyl-10-deacetyl baccatin (III) 1,2-carbonate (10.0 mg, 0.014 mmol) in THF (0.5 mL) at -45° C was added 78 µL of a 1.8 M solution of phenyllithium (10 eq) in 30% ether/70% cyclohexane. The solution was stirred for 1 h at -45° C under N₂ and saturated aqueous NaHCO was added (1.0 mL). The mixture was extracted with EtOAc (3 x 5.0 mL). The organic layer was washed with brine (5.0 mL), dried over anhydrous Na₂SO₄, filtered and concentrated under reduced pressure to give a crude solid (12.5 mg). Flash chromatography with 10% EtOAc in hexane gave pure 13-O-trimethylsilyl-7,10-bis-O-triethylsilyl-2-debenzoyl-10-deacetyl baccatin (III) (10.8 mg, 94.5%).

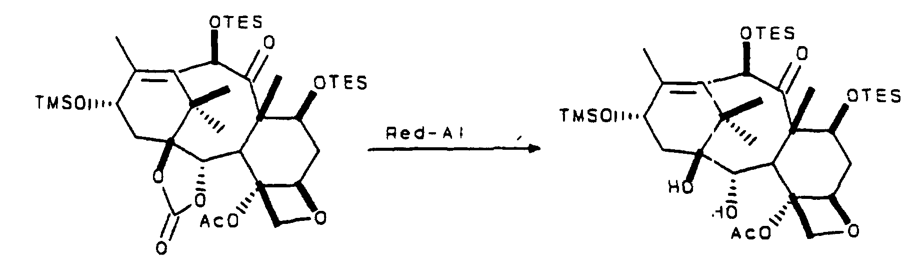

### c. 13-O-Trimethylsilyl-7,10-bis-O-triethylsilyl-2-debenzoyl-10-deacetyl baccatin (III)

To a stirred solution of 13-O-trimethylsilyl-7,10-bis-O-triethylsilyl-2-debenzoyl-10-deacetyl baccatin (III) 1,2-carbonate (6.0 mg, 0.0082 mmol) in THF (0.5 mL) at 0° C was added 60 µL of a 0.068 M solution (5 eq) of Red-Al in toluene. The resuting solution was stirred for 1 h at 0° C under N₂, 1.0 mL of saturated aqueous NaHCO₃ was added, and the mixture was extracted with EtOAc (2 x 10.0 mL). The organic layer was washed with brine (5.0 mL), dried over anhydrous Na₂SO₄, filtered and concentrated under reduced pressure to give a crude solid (6.75 mg). Flash chromatography with 30% EtOAc in hexane gave 13-O-trimethylsilyl-7,10-bis-O-triethylsilyl-2-debenzoyl-10-deacetyl baccatin (III) (4.3 mg, 71.5%) and 13-O-trimethylsilyl-7,10-bis-O-triethylsilyl-2-debenzoyl-2--formyl-10-deacetyl baccatin (III) (1.5 mg, 24.5%).

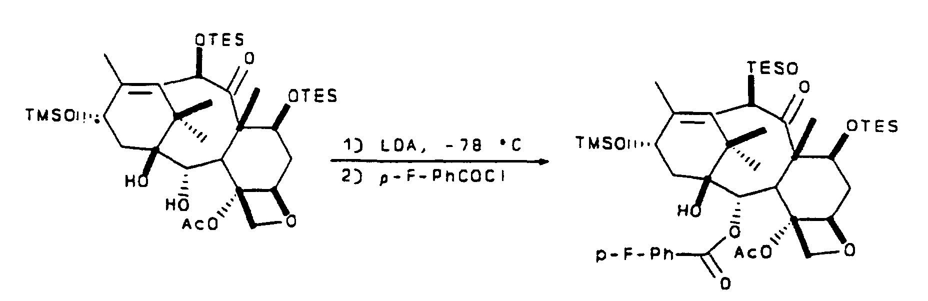

### d. 13-O-Trimethylsilyl-7,10-bis-O-triethylsilyl-2-debenzoyl-2-p-fluorobenzoyl-10-deacetyl baccatin (III)

To a solution of 13-O-trimethylsilyl-7,10-bis-O-triethylsilyl-2-debenzoyl-10-deacetyl baccatin (III) (40.0 mg, 0.054 mmol) in THF (1.0 mL) at -78° C under N₂ was added dropwise 320 µL of a 0.328 M solution (2 eq) of LDA in THF. The mixture was stirred for 30 min at -78° C and a solution of 26 µL (4 eq) of *p*-fluorobenzoyl chloride in 100 µL of THF was added. After 1h diisopropylamine (100 µL) was added and the mixture was warmed to 25° C. After 10 min the mixture was diluted with aqueous NaHCO₃(5.0 mL) and extracted with EtOAc (2 x 10.0 mL). The organic layer was washed with brine (5.0 mL), dried over anhydrous Na₂SO₄, filtered and concentrated under reduced pressure to give a crude solid (67.5 mg). Flash chromatography with 10% EtOAc in hexane gave 13-O-trimethylsilyl-7,10-bis-O-triethylsilyl-2-debenzoyl-2-*p*-fluorobenzoyl-10-deacetyl baccatin (III) (36.9 mg, 80.2%). m.p. 216-218° C, [α]²⁵ _{Na} -45.6° (c 0.5, CHCl₃), ¹H NMR (CDCl₃, 300 MHz) δ 8.10 (m, 2H, aromatic), 7.18-7.12 (m, 2H, aromatic), 5.60 (d, J = 7.2 Hz, 1H, H2), 5.19 (s, 1H, H10), 4.94 (dd, J = 1.7, 9.9 Hz, 1H, H5), 4.86 (m, 1H, H13), 4.41 (dd, J = 6.9, 10.4 Hz, 1H, H7), 4.26 (d, J = 8.2 Hz, 1H, H20α), 4.11 (d, J = 8.2 Hz, 1H, H20β), 3.86 (d, J = 6.6 Hz, 1H, H3), 2.51 (m, 1H, H6α), 2.25 (s, 3H, 4Ac), 2.11 (m, 2H, H14α, H14β), 2.04 (s, 3H, Me18), 1.88 (m, 1H, H6β), 1.64 (s, 3H, Me19), 1.18 (s, 3H, Me17), 1.12 (s, 3H, Me16), 1.02-0.92 (m, 18H, SiCH₂CH₃), 0.69-0.54 (m, 12H, SiCH₂CH₃), 0.17 (s, 9H, SiCH₃).

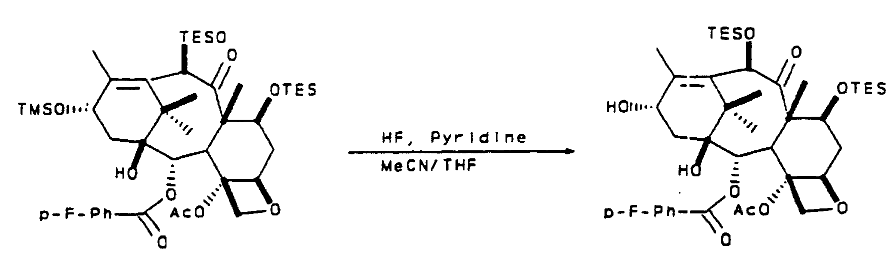

### e. 7,10-Bis-O-triethylsilyl-2-debenzoyl-2-p-fluorobenzoyl-10-deacetyl baccatin (III)

To a solution of 13-O-trimethylsilyl-7,10-bis-O-triethylsilyl-2-debenzoyl-2-*p*-fluorobenzoyl-10-dcacetyl baccatin (III) (30.0 mg, (1.035 mmol) in 2.25 mL of acetonitrile and 2.25 mL of THF in a polyethylene vial was added dropwise 48 µL of pyridine and 75 µL of 48% aqueous HF. The reaction mixture was stirred at 25° C for 12 h and then diluted with EtOAc (20.0 mL). Saturated aqueous NaHCO₃ was added until gas evolution ceased. The organic layer was separated, washed with brine (3.0 mL), dried over anhydrous Na₂SO₄, filtered and concentrated under reduced pressure to give a crude solid (36.2 mg). Flash chromatography with 25% EtOAc in hexane gave 7,10-bis-O-triethylsilyl-2-debenzoyl-2-*p*-fluorobenzoyl-10-deacetyl baccatin (III) (21.5 mg, 78.8%) and 10-O-triethylsilyl-2-debenzoyl-2--*p*-fluorobenzoyl-10-deacetyl baccatin (III) (3.8 mg, 15.9%). m.p. 186-188° C, [α]²⁵ _{Na} -48.2° (c 0.5, CHCl₃), ¹H NMR (CDCl₃, 300 MHz) δ 8.11 (m, 2H, aromatic), 7.26-7.11 (m, 2H, aromatic), 5.59 (d, J = 6.6 Hz, 1H, H2), 5.21 (s, 1H, H10), 4.94 (dd, J = 1.7, 9.34 Hz, 1H, H5), 4.84 (m, 1H, H13), 4.42 (dd, J = 6.6, 10.4 Hz, 1H, H7), 4.26 (d, J = 8.24 Hz, 1H, H20α), 4.14 (d, J = 8.25 Hz, 1H, H20β), 3.90 (d, J = 6.6 Hz, 1H, H3), 2.54 (m, 1H, H6α), 2.26 (s, 3H, 4Ac), 2.05 (m, 2H, H14α, H14β), 2.02 (s, 3H, Me18), 1.88 (m, 1H, H6β), 1.65 (s, 3H, Me19), 1.18 (s, 3H, Me17), 1.05 (s, 3H, Me16), 1.02-0.92 (m, 18H, SiCH₂CH₃), 0.69-0.53 (m, 12H, SiCH₂CH₃).

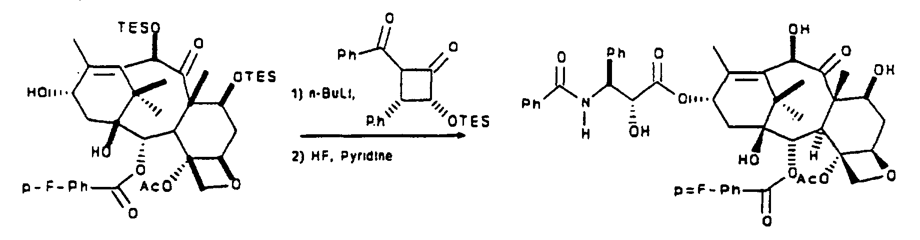

### f. 2-Debenzoyl-2-p-fluorobenzoyl taxol.

To a solution of 7,10-bis-O-triethylsilyl-2-debenzoyl-2-*p*-fluoro-benzoyl-10-deacetyl baccatin (III) (20.0 mg, 0.026 mmol) in 1.0 mL of THF at -45° C was added dropwise 16 µL of a 1.64 M solution of n-butyllithium in hexane. After 0.5 h at -45° C, a solution of (±) cis-1-benzoyl-3-triethyl-silyloxy-4-phenyl azetidin-2-one (50.0 mg, 0.13 mmol) in THF (0.5 mL) was added dropwise to the mixture. The solution was warmed to 0° C and kept at that temperature for 1 h and 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel with 20% EtOAc in hexane to give a crude solid (32.5 mg). To a soludon of this solid in 1.6 mL of acetonitrile and 79 µL of pyridine at 0° C was added 240 µL of 48% aqueous HF. The mixture was stirred at 0° C for 3 h, then at 25° C for 13 h, and partitioned between saturated aqueous sodium bicarbonate and ethyl acetate. Evaporation of the ethyl acetate solution gave a crude solid (24.4 mg) which was purified by flash chromatography with 70% EtOAc in hexane to give 2-debenzoyl-2-*p*-fluorobenzoyl taxol (15.2 mg, 70.4%). m.p. 180-183° C, [α]²⁵ _{Na} -56.9° (c 0.5, CHCl₃), ¹H NMR (CDCl₃, 300 MHz) δ 8.15 (m, 2H, aromatic), 7.73 (m, 2H, aromatic), 7.52-7.34 (m, 8H, aromatic), 7.20 (m, 2H, aromatic), 7.07 (d, J = 9.3 Hz, 1H, NH), 6.22 (dd, J = 8.8, 8.8 Hz, 1H, H13), 5.79 (dd, J = 8.8, 2.7 Hz, 1H, H3'), 5.64 (d, J = 7.1 Hz, 1H, H2β), 5.17 (s, 1H, H10), 4.94 (dd, J = 9.3, 1.7 Hz, 1H, H5), 4.79 (m, 1H, H2'), 4.29-4.15 (m, 3H, H7, H20α, H20β), 3.90 (d, J = 7.1 Hz, 1H, H3), 3.56 (d, J = 5.0 Hz, 1H, 2'OH), 2.58 (m, 1H, H6α), 2.38 (s, 3H, 4Ac), 2.28 (m, 2H, H14α, H14β), 1.82 (m, 1H, H6β), 1.79 (s, 3H, Me18), 1.74 (s, 3H, Me19), 1.20 (s, 3H, Me17), 1.10 (s, 3H, Me16).

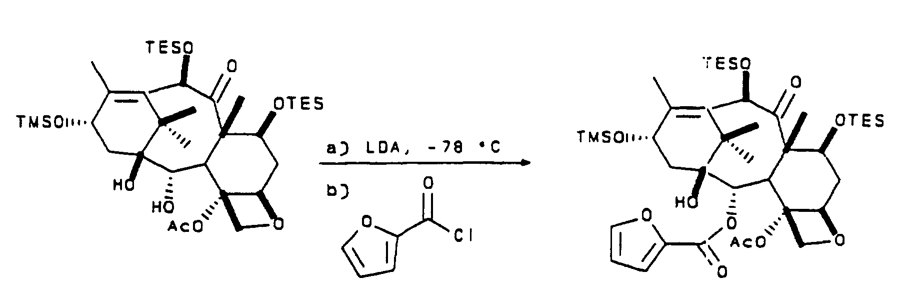

### g. 13-O-Trimethylsilyl-7,10-bis-O-triethylsilyl-2-debenzoyl-2-(2-furoyl)-10-deacetyl baccatin (III)

To a solution of 13-O-trimethylsilyl-7,10-bis-O-triethylsilyl-2-debenzoyl-10-deacetyl baccatin (III) (40.0 mg, 0.054 mmol) in THF (1.0 mL) at -78° C under N₂ was added dropwise 320 µL of a 0.328 M solution (2 eq) of LDA in THF. The mixture was stirred for 30 min at -78° C and a solution of 26 µL (4 eq) of *p*-fluorobenzoyl chloride in 100 µL of THF was added. After 1h diisopropylamine (100 µL) was added and the mixture was warmed to 25° C. After 10 min the mixture was diluted with aqueous NaHCO₃ (5.0 mL) and extracted with EtOAc (2 x 10.0 mL). The organic layer was washed with brine (5.0 mL), dried over anhydrous Na₂SO₄, filtered and concentrated under reduced pressure to give a crude solid (64.2 mg). Flash chromatography with 15% EtOAc in hexane gave 13-O-trimethylsilyl-7,10--bis-O-triethylsilyl-2-debenzoyl-2-(2-furoyl)-10-deacetyl baccatin (III) (33.9 mg, 76.3%). m.p. 208-210° C, [α]²⁵ _{Na} -49.6° (c 0.5, CHCl₃), ¹H NMR (CDCl₃, 300 MHz) δ 7.62 (m, 1H, furoyl), 7.20 (m, 1H, furoyl), 6.50 (m, 1H, furoyl), 5.52 (d, J = 7.1 Hz, 1H, H2), 5.18 (s, 1H, H10), 4.95 (dd, J = 1.6, 9.4 Hz, 1H, H5), 4.85 (m, 1H, H13), 4.41 (dd, J = 6.9, 10.4 Hz, 1H, H7), 4.38 (d, J = 8.8 Hz, 1H, H20α), 4.15 (d, J = 8.2 Hz, 1H, H20β), 3.82 (d, J = 6.6 Hz, 1H, H3), 2.51 (m, 1H, H6α), 2.22 (s, 3H, 4Ac), 2.10 (m, 2H, H14α, H14β), 1.92 (s, 3H, Me18), 1.89 (m, 1H, H6β), 1.64 (s, 3H, Me19), 1.17 (s, 3H, Me17), 1.12 (s, 3H, Me16), 1.01-0.93 (m, 18H, SiCH₂CH₃), 0.69-0.52 (m, 12H, SiCH₂CH₃), 0.16 (s, 9H, SiCH₃).

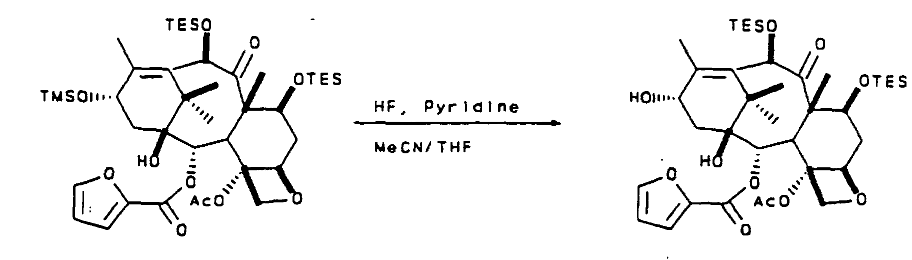

### h. 7,10-Bis-O-triethylsilyl-2-debenzoyl-2-(2-furoyl)-10-deacetyl baccatin (III)

To a solution of 13-O-trimethylsilyl-7,10-bis-O-triethylsilyl-2-debenzoyl-2-(2-furoyl)-10-deacetyl baccatin (III) (30.0 mg, 0.036 mmol) in 2.25 mL of acetonitrile and 2.25 mL of THF in a polyethylene vial was added dropwise 48 µL of pyridine and 75 µL of 48% aqueous HF. The reaction mixture was stirred at 25° C for 12 h and then diluted with EtOAc (20.0 mL). Saturated aqueous NaHCO₃ was added until gas evolution ceased. The organic layer was separated, washed with brine (3.0 mL), dried over anhydrous Na₂SO₄, filtered and concentrated under reduced pressure to give a crude solid (33.4 mg). Flash chromatography with 30% EtOAc in hexane gave 7,10-bis-O-triethylsilyl-2-debenzoyl-2-(2-furoyl)-10-deacetyl baccatin (III) (21.3 mg, 78.8%) and 10-O-triethylsilyl-2-debenzoyl-2-(2-furoyl)-10-deacetyl baccatin (III) (4.9 mg, 21.4%). m.p. 179-181° C, [α]²⁵ _{Na} -45.6° (c 0.5, CHCl₃), ¹H NMR (CDCl₃, 300 MHz) δ 7.62 (m, 1H, furoyl), 7.21 (m, 1H, furoyl), 6.53 (m, 1H, furoyl), 5.51 (d, J = 7.1 Hz, 1H, H2), 5.20 (s, 1H, H10), 4.94 (dd, J = 1.7, 9.3 Hz, 1H, H5), 4.82 (m, 1H, H13), 4.43-4.37 (m, 2H, H7, H20α), 4.18 (d, J = 8.2 Hz, 1H, H20β), 3.87 (d, J = 7.2 Hz, 1H, H3), 2.52 (m, 1H, H6α), 2.23 (s, 3H, 4Ac), 2.10 (m, 2H, H14α, H14β), 2.01 (s, 3H, Me18), 1.88 (m, 1H, H6β), 1.64(s, 3H, Me19), 1.17 (s, 3H, Me17), 1.04 (s, 3H, Me16), 1.02-0.92 (m, 18H, SiCH₂CH₃), 0.69-0.54 (m, 12H, SiCH₂CH₃).

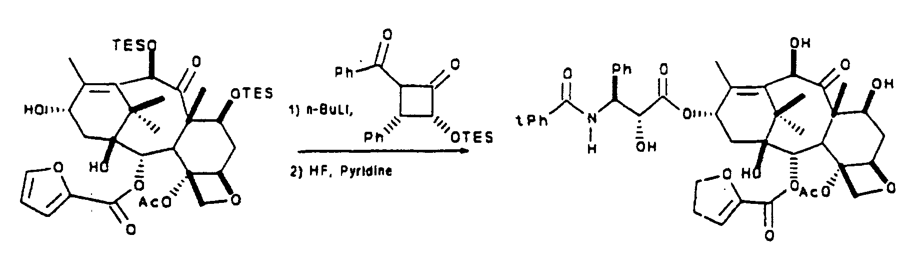

### i. 2-Debenzoyl-2-(2-furoyl) taxol.

To a solution of 7,10-bis-O-triethylsilyl-2-debenzoyl-2-(2-furoyl)-10-deacetyl baccatin (III) (20 mg, 0.027 mmol) in 1.0 mL of THF at -45° C was added dropwise 16 µL of a 1.64 M solution of n-butyllithium in hexane. After 0.5 h at -45° C, a solution of (±) cis-1-benzoyl-3-triethylsilyloxy-4-phenyl azetidin-2-one (50.0 mg, 0.13 mmol) in THF (0.5 mL) was added dropwise to the mixture. The solution was warmed to 0° C and kept at that temperature for 1 h and 1 mL of a 10% solution of AcOH in THF was added. The mixture was partitioned between saturated aqueous NaHCO₃ and 60/40 ethyl acetate/hexane. Evaporation of the organic layer gave a residue which was purified by filtration through silica gel with 20% EtOAc in hexane to give a crude solid (31.7 mg). To a solution of this solid in 1.6 mL of acetonitrile and 79 µL of pyridine at 0° C was added 240 µL of 48% aqueous HF. The mixture was stirred at 0° C for 3 h, then at 25° C for 13 h, and partitioned between saturated aqueous sodium bicarbonate and ethyl acetate. Evaporation of the ethyl acetate solution gave a crude solid (24.4 mg) which was purified by flash chromatography with 70% EtOAc in hexane to give 2-debenzoyl-2- (2-furoyl) taxol (14.9 mg, 68.8%). m.p. 176-179° C, [α]²⁵ _{Na} -43.1° (c 0.5, CHCl₃), ¹H NMR (CDCl₃, 300 MHz) δ 7.76-7.32 (m, 12H, aromatic) 7.08 (d, J = 8.8 Hz, 1H, NH), 6.60 (m, 1H, furoyl), 6.20 (dd, J = 8.8, 8.8 Hz, 1H, H13), 5.78 (dd, J = 8.8, 2.8 Hz, 1H, H3'), 5.56 (d, J = 7.1 Hz, 1H, H2β), 5.16 (s, 1H, H10), 4.93 (dd, J = 9.3, 1.7 Hz, 1H, H5), 4.78 (m, 1H, H2'), 4.40 (d, J = 8.3 Hz, 1H, H20α), 4.24 (d, J = 8.2 Hz, 1H, 20β), 4.19 (m, 1H, H7), 3.86 (d, J = 7.1 Hz, 1H, H3), 3.57 (d, J = 5.0 Hz, 1H, 2'OH), 2.56 (m, 1H, H6α), 2.35 (s, 3H, 4Ac), 2.24 (m, 2H, H14α, H14β), 1.83 (m, 1H, H6β), 1.76 (s, 3H, Me18), 1.73 (s, 3H, Me19), 1.19 (s, 3H, Me17), 1.08 (s, 3H, Me16).

### EXAMPLE 16

### Preparation of Taxol Analogs With Various Substituents At C-4

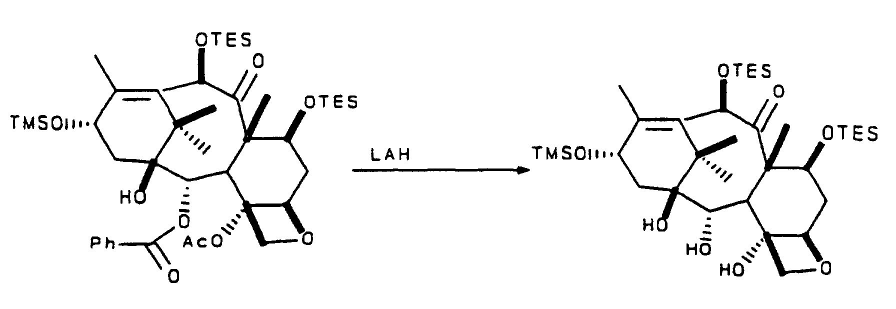

### a. 13-O-Trimethylsilyl-7,10-bis-O-triethylsilyl-2-debenzoyl-4,10-bisdeacetyl baccatin (III).

To a solution of 13-O-trimethylsilyl-7,10-bis-O-triethylsilyl-10-deacetyl baccatin (III) (0.1 g, 0.012 mmol) in ether (4.0 mL) at -10 °C was added dropwise 320 µL of a 1.0 M solution of lithium aluminum hydride (LAH) in ether. The resulting mixture was slowly warmed from -10 °C to 0 °C over a 2 h period and 3.0 mL of saturated aqueous NaHCO₃ was added. The solution was filtered and the solid was rinsed with EtOAc. The filtrate was concentrated under reduced pressure and diluted with EtOAc (50.0 mL). The organic layer was separated and washed with brine (5.0 mL). The combined organic layer was dried over anhydrous Na₂SO₄, filtered, and concentrated under reduced pressure to give a crude oil (0.15 g). Flash chromatography with 30% EtOAc in hexane gave pure 13-O-trimethylsilyl-7,10-bis-O-triethylsilyl-2-debenzoyl-4,10-bisdeacetyl baccatin (III) (72.0 mg, 85.8%) as a colorless oil. [α]²⁵ _{Na} -25.5° (c 0.5, CHCl₃), ¹H NMR (CDCl₃, 300 MHz), δ 5.15 (s, 1H, H10), 4.73 (dd, J = 1.8, 8.9 Hz, 1H, H5), 4.65 (m, 1H, H13), 4.53 (d, J = 9.3 Hz, 1H, H20α), 4.39 (d, J = 8.2 Hz, 1H, H20β), 4.00 (dd, J = 6.0, 11.5 Hz, 1H, H7), 3.76 (m, 1H, 2H), 3.44 (d, J = 11.0 Hz, 20H), 3.27 (d, J = 6.0 Hz, 1H, H3), 2.45 (m, 2H, H6α, H14α), 2.08-1.93 (m, 2H, H6β, H14β), 1.84 (s, 3H, Me18), 1.53 (s, 3H, Me19), 1.09 (s, 3H, Me17), 1.05 (s, 3H, Me16), 1.01-0.91 (m, 18H, SiCH₂CH₃), 0.66-0.53 (m, 12H, SiCH₂CH₃), 0.23 (s, 9H, SiCH₃).

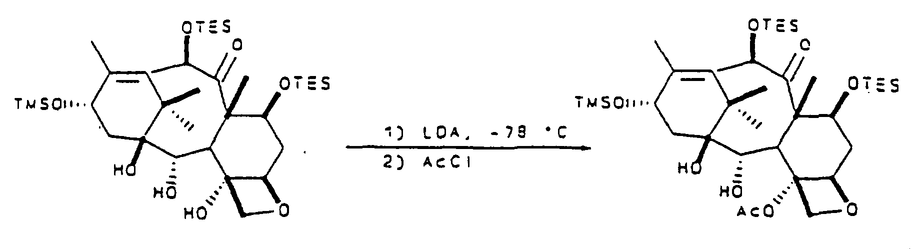

### b. 13-O-Trimethylsilyl-7,10-bis-O-triethylsilyl-2-debenzoyl-10-deacetyl baccatin (III)

To a solution of 13-O-trimethylsilyl-7,10-bis-O-triethylsilyl-2-debenzoyl-4,10-bisdeacetyl baccatin (III) (10.0 mg, 0.0143 mmol) in THF (1.0 mL) at -78 °C was added 440 µL of a 0.328 M solution (10 eq) of lithium diisopropyl amide (LDA) in THF under N₂. The solution was stirred for 30 min at -78 °C and 200 µL of a 1.4 M solution of acetyl chloride in THF (20 eq) was added. The mixture was stirred for 1h at -78 °C, saturated aqueous NaHCO₃ (2.0 mL) was added, and the mixture was extracted with EtOAc (2 x 10 mL). The organic layer was washed with brine (5.0 mL), dried over anhydrous Na₂SO₄, filtered and concentrated to give a crude oil (12.7 mg). Flash chromatography from 25% EtOAc to 50% EtOAc in hexane gave 13-O-trimethylsilyl-7,10-bis-O-triethylsilyl-2-debenzoyl-10-deacetyl baccatin (III) (6.1 mg, 57.5%), 13-O-trimethylsilyl-7,10-bis-O-triethylsilyl-2-debenzoyl-2-acetyl-4,10-bisdeaceryl baccatin (III) (1.89 mg, 17.9%), recovered 13-O-trimethylsilyl-7,10-bis-O-triethylsilyl-2-debenzoyl-4,10-bisdeacetyl baccatin (III) (1.2 mg, 12.0%) and 13-O-trimethylsilyl-7,10-bis-O-triethylsilyl-2-debenzoyl-2-acetyl-4,10-bisdeaceryl baccatin (III) (< 1.0 mg).

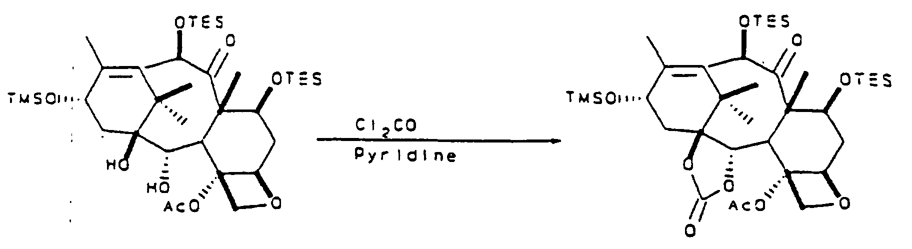

### c. 13-O-Trimethylsilyl-7,10-bis-O-triethylsilyl-2-debenzoyl- 4,10-bisdeacetyl baccatin (III) 1,2-carbonate

To a solution of 13-O-trimethylsilyl-7,10-bis-O-triethylsilyl-2-debenzoyl-4,10-bisdeacetyl baccatin (III) (20.0 mg, 0.029mmol) in CH₂Cl₂(4.0 mL) and pyridine (0.8 mL) at -78 °C was added 80 µL of a 3.4 M solution of COCl₂ in benzene (10 eq). The mixture was warmed to -10 °C (ice-acetone) and kept for 30 min at -10 °C. Saturated aqueous NaHCO₃(5.0 mL) was added and the mixture was extracted with EtOAc (3 x 10 mL). The organic layer was washed with aqueous 10% CuSO₄ then brine (5.0 mL), dried over anhydrous Na₂SO₄, filtered and concentrated under reduced pressure to give a crude solid (21.9 mg). Plug filtration with 20% EtOAc in hexane gave pure 13-O-trimethylsilyl-7,10-bis-O-triethylsilyl-2-debenzoyl-4,10-bisdeacetyl baccatin (III) 1,2-carbonate (20.8 mg, 98.9%). m.p. 147-148 °C, [α]²⁵_{Na} -28.8° (c 0.5, CHCl₃), ¹H NMR (CDCl₃, 300 MHz), δ 5.21 (s, 1H, H10), 4.76 (dd, J = 2.8, 9.9 Hz, 1H, H5), 4.65 (m, 1H, H13), 4.54 (d, J = 8.8 Hz, 1H, H20α), 4.52 (d, J = 8.3 Hz, 1H, H20β), 4.32 (d, J = 5.0 Hz, 1H, H2), 4.10 (dd, 1 = 6.6, 11.0 Hz, 1H, H7), 3.10 (d, J = 5.3 Hz, 1H, H3), 2.54 (m, 3H, H6α, H14α, H14β), 1.99 (m, 1H, H6β), 1.92 (s, 3H, Me18), 1.61 (s, 3H, Me19), 1.17 (s, 3H, Me17), 1.12 (s, 3H, Me16), 1.01-0.91 (m, 18H, SiCH₂CH₃), 0.67-0.56 (m, 12H, SiCH₂CH₃), 0.23 (s, 9H, SiCH₃).

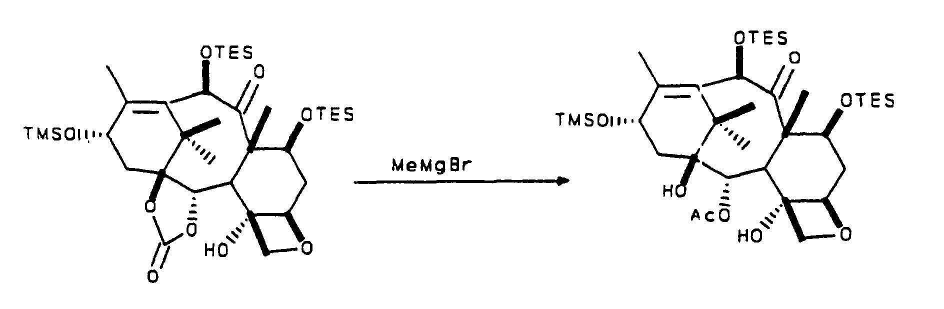

### d. 13-O-Trimethylsilyl-7,10-bis-O-triethylsilyl-2-debenzoyl-2-acetyl-4,10-bisdeacetyl baccatin (III)

To a solution of 13-O-trimethylsilyl-7,10-bis-O-triethylsilyl-2-debenzoyl-4,10-bisdeacetyl baccatin (III) (10.0 mg, 0.014 mmol) in THF (0.5 mL) at 0 °C was added 40 µL of a 3.4 M solution (10 eq) of MeMgBr in ether. The solution was stirred for 1 h at 0 °C under N₂ and saturated aqueous NaHCO₃ was added (1.0 mL). The mixture was extracted with EtOAc (3 x 5.0 mL) and the organic layer was washed with brine (5.0 mL), dried over anhydrous Na₂SO₄, filtered and concentrated under reduced pressure to give a crude solid (11.9 mg). Flash chromatography with 20% EtOAc in hexane gave pure 13-O-trimethylsilyl-7,10-bis-O-triethylsilyl-2-debenzoyl-2-acetyl-4,10-bisdeacetyl baccatin (III) (9.9 mg, 97.0%). m.p. 198-201 _C, [α] ²⁵ _{Na} -39.9° (c 0.5, CHCl₃), ¹H NMR (CDCl₃, 300 MHz), δ 5.27 (d, J = 5.5 Hz, 1H, H2), 5.22 (s, 1H, H10), 4.71 (m, 1H, H13), 4.58 (dd, J = 2.8, 9.3 Hz, 1H, H5), 4.41 (d, J = 7.7 Hz, 1H, H20α), 4.35 (d, J = 7.1 Hz, 1H, H20β), 4.01 (dd, J = 6.1, 11.6 Hz, 1H, H7), 3.74 (s, 1H, 40H), 3.47 (d, J = 5.5 Hz, 1H, H3), 2.45 (m, 1H, H6α, 2.24-2.04 (m, 2H, H14α H14β), 2.06 (s, 3H, 2Ac), 1.96 (m, 1H, H6β), 1.88 (s, 3H, Me18), 1.46 (s, 3H, Me19), 1.14 (s, 3H, Me17), 1.02 (s, 3H, Me16), 0.99-0.91 (m, 18H, SiCH₂CH₃), 0.67-0.56 (m, 12H, SiCH₂CH₃), 0.24 (s, 9H, SiCH₃).

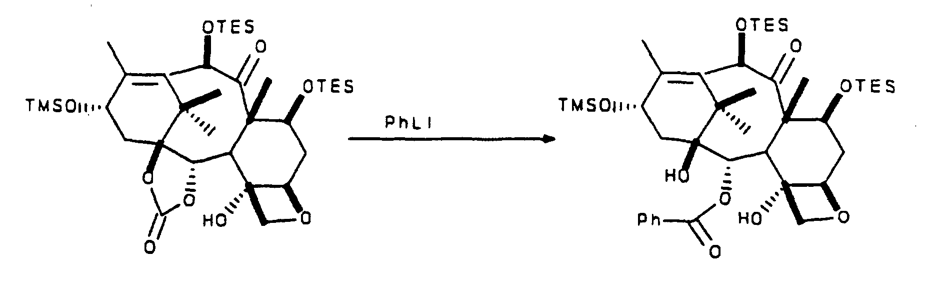

### e. 13-O-Trimethylsilyl-7,10-bis-O-triethylsilyl-4,10-bisdeacetyl baccatin (III)

To a solution of 13-O-trimethylsilyl-7,10-his-O-triethylsilyl-2-debenzoyl-4,10-bisdeacetyl baccatin (III) 1,2-carbonate (10.0 mg, 0.014 mmol) in THF (0.5 mL) at -45 °C was added 78 µL of a 1.8 M solution of phenyllithium (10 eq) in 30% ether/70% cyclohexane. The solution was stirred for 1 h at -45 °C under N₂ and saturated aqueous NaHCO was added (1.0 mL). The mixture was extracted with EtOAc (3 x 5.0 mL). The organic layer was washed with brine (5.0 mL), dried over anhydrous Na₂SO₄, filtered and concentrated under reduced pressure to give a crude solid (12.8 mg). Flash chromatography with 50% EtOAc in hexane gave pure 13-O-trimethylsilyl-7,10-bis-O-triethylsilyl-4,10-bisdeacetyl baccatin (III) (9.9 mg, 98.5%). m.p. 177-179 °C, [α]²⁵ _{Na} -44.5° (c 0.3, CHCl₃), ¹H NMR (CDCl₃, 300 MHz) δ 8.05 (d, J = 7.2 Hz, 2H, benzoate ortho), 7.60-7.37 (m, 3H, aromatic), 5.61 (d, J = 6.1 Hz, 1H, H2), 5.25 (s, 1H, H10), 4.74 (m, 1H, H13), 4.57 (dd, J = 1.7, 9.3 Hz, 1H, H5), 4.38 (d, J = 8.2 Hz, 1H, H20α), 4.12 (d, J = 8.2 Hz, 1H, H20b), 4.05 (dd, J = 6.1, 12.1 Hz, 1H, H7), 3.69 (d, J = 6.0 Hz, 1H, H3), 2.55 (m, 1H, H6a), 2.42 (m, 2H, H14α, H14β), 2.04 (s, 3H, Me18), 1.99 (m, 1H, H6β), 1.64 (s, 3H, Me19), 1.20 (s, 3H, Me17), 1.04 (s, 3H, Me16), 1.01-0.90 (m, 18H, SiCH₂CH₃), 0.70-0.56 (m, 12H, SiCH₂CH₃).

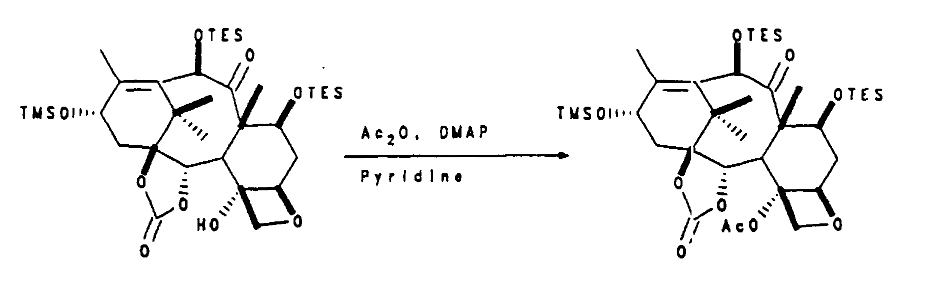

### f. 13-O-Trimethylsilyl-7,10-bis-O-triethylsilyl-2-debenzoyl-10-deacetyl baccatin (III) 1,2-carbonate

To a stirred solution of 13-O-trimethylsilyl-7,10-bis-O-triethylsilyl-2-debenzoyl-4,10-bisdeacetyl baccatin (III) 1,2-carbonate (8.0 mg, 0.011 mmol) in pyridine (0.5 mL) was added Ac₂O (100 µL) and DMAP (50 mg). The solution was heated at reflux under N₂ for 12 h, cooled to room temperature, and 15.0 mL of EtOAc was added. The organic layer was washed with 10% aqueous CuSO₄ and brine (5.0 mL), dried over anhydrous Na₂SO₄, filtered and concentrated under reduced pressure to give a crude solid (22.0 mg). Flash chromatography with 20% EtOAc in hexane gave 13-O-trimethylsilyl-7,10-bis-O-triethylsilyl-2-debenzoyl-10-deacetyl baccatin (III) 1,2-carbonate (4.4 mg, 53.0%) and 13-O-acetyl-7,10-bis-O-triethylsilyl-2--debenzoyl-10-deacetyl baccatin (III) 1,2-carbonate (2.3 mg, 27.8%).

### EXAMPLE 17

### 10-Desacetoxybaccatin III:

To a solution of baccatin III (20 mg; 0.034 mmol) in THF (0.09 mL) at 0°C under nitrogen was added a solution of Sml₂ (0.1 M; 0.9 mL; 0.09 mmol) in THF. After stirring 45 minutes at 0°C the flask was opened to the air, and the reaction mixture diluted with ethyl acetate (10 mL). The mixture was poured into aqueous HCl (0.2N; 25 mL), extracted with ethyl acetate, and the extract was washed successively with saturated aqueous NaHCO₃ and brine, dried over Na₂SO₄ and evaporated. The product was isolated by flash chromatography (SiO₂; 80% ethyl acetate-hexanes) affording 16.6 mg (92%) of 10-desacetoxybaccatin III which was recrystallized from CHCl₃-hexanes. mp 230-232 °C. [α]²⁵ _{c} = -103.6 (c=0.00195, CHCl₃). IR (cm⁻¹): 3100, 2970, 2950, 2900, 1750, 1710, 1460, 1370, 1320, 1270, 1255, 1110, 980, 890, 760, 700. ¹H-nmr (500 MHz, CDCl₃) δ 8.11 (dd ; 2H; J=8.4, 1.2 Hz; o-Bz); 7.61 (dt; 1H; J=7.5,1.2 Hz; p-Bz); 7.48 (br t; 2H; J=7.8 Hz; m-Bz); 5.66 (br d; 1 H; J=6.9 Hz; H-2β); 4.98 (br dd; 1H; J=9.4,2; H-5a); 4.83 (br; 1H; w1/2 19 Hz; H-13β); 4.34 (dt; 1H; J=11.2, 7.8Hz; H-7α); 4.31 (br d; 1H;J=8.4 Hz; H-20α); 4.17 (br d; 1H; J=6.9, Hz; H-3α); 4.15 (dd; 1H; J=8.4, 1Hz; H-20β); 3.84 (d; 1H; J=15.6 Hz; H-10α); 3.46 (ddd; 1H; J=15.6,3.7,1.6 Hz; H-10β); 2.64 (ddd; 1H; J=14.4,9.4,6.9 Hz; H-6α); 2.29 (s; 3H; 4-OAc); 2.28 (m; 2H; H-14α and H-14β); 1.95 (t; 3H; J=1.6 Hz; 18-Me); 1.94 (d, 1H; J=6.8 Hz; 13-OH); 1.79 (ddd; 1H; J=14.4, 11.2, 2.1 Hz; H-6β); 1.64 (s; 3H; 19-Me); 1.58 (s; 1H; 1-OH); 1.38 (d; 1H; J=7.8 Hz; 7-OH); 1.13 (s, 3H; 16-Me); 1.06 (s, 3H; 17-Me).

### EXAMPLE 18

### 7-Triethylsilyl-10-desacetoxybaccatin III:

To a stirred solution of 10-desacetoxybaccatin III (10.0 mg; 0.019 mmol) in anhydrous pyridine (0.05 mL) at room temperature and under nitrogen, triethylchlorosilane (15 L; 0.09 mmol) was added and the resulting mixture was stirred at room temperature for 48 h. After diluting with ethyl acetate (5 mL) the mixture was poured into saturated aqueous NaHCO₃ (25 mL) and extracted with ethyl acetate. The extract was washed successively with water, 10% aqueous CuSO₄ and brine, dried over Na₂SO₄ and evaporated. The product was purified by flash chromatography (SiO₂; 40% EA-hexanes) affording 11.1 mg (91%) of 7-triethylsilyl-10-desacetoxybaccatin III.

### EXAMPLE 19

### 10-Desacetoxytaxol:

To a stirred solution of taxol (35 mg; 0.041 mmol) in THF (0.1 mL) at 0 °C under nitrogen was added a solution of Sml₂ (0.1 M; 1.0 mL; 0.10 mmol) in THF. After stirring 45 minutes at 0 °C the flask was opened to the air and the reaction mixture diluted with ethyl acetate (10 mL). The mixture was poured into aqueous HCl (0.2N; 25 mL), extracted with ethyl acetate, and the extract was washed successively with saturated aqueous NaHCO₃ and brine, dried over Na₂SO₄ and evaporated. The product was isolated by flash chromatography (SiO₂; 80% ethyl acetate-hexanes) affording 29.4 mg (90%) of 10-desacetoxytaxol.

## Claims

1. A process for the preparation of a derivative or analog of baccatin III or 10-desacetyl baccatin III having a C2 substituent other than benzoate and/or a C4 substituent other than acetate from a derivative or analog of baccatin III or 10-desacetyl baccatin III which comprises a C2 benzoate and/or C4 acetate substituent, the process comprising selectively reducing the C2 benzoate substituent and/or the C4 acetate substituent with an aluminate to the corresponding hydroxy group(s) in a non-aqueous system and acylating the C2 hydroxy substituent and/or the C4 hydroxy substituent to convert the C2 hydroxy substituent to R₃₁COO- and/or convert the C4 hydroxy substituent to R₃₀COO- wherein R₃₁ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

2. A process according to claim 1, wherein the derivative or analog of baccatin III or 10-desacetyl derivative has the formula 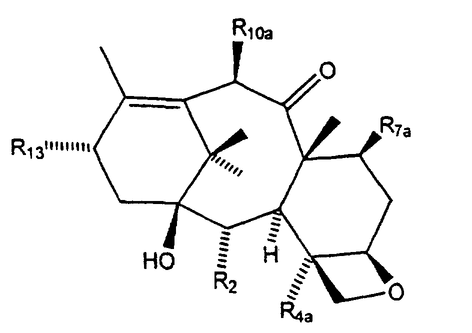 wherein
R₁₀ₐ is H, -OH, protected hydroxy, or -OCOR₂₉ₐ;
R₁₃ is -OH or protected hydroxy;
R₇ₐ is H, -OH or protected hydroxy;
R₂ is -OCOR₃₁;
R₄ₐ is -OCOR₃₀; and
R₂₉ₐ, R₃₀ and R₃₁ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

3. A process according to claim 1 or claim 2, wherein the C2 benzoate substituent and/or the C4 acetate substituent of the derivative of baccatin III or 10-desacetyl baccatin III is or are selectively reduced with lithium aluminum hydride or sodium bis (2-methoxyethoxy) aluminum hydride.

4. A process according to any one of claims 1 to 3, wherein the derivative or analog of baccatin III or 10-desacetyl derivative has the formula 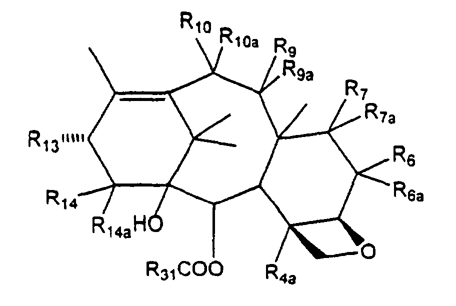 wherein
R₄ₐ is hydrogen, optionally substituted alkyl, optionally substituted alkenyl, optionally substituted alkynyl, optionally substituted aryl, optionally substituted heteroaryl, cyano, hydroxy, or -OCOR₃₀;
R₆ is hydrogen, optionally substituted alkyl, optionally substituted alkenyl, optionally substituted alkynyl, optionally substituted aryl, or optionally substituted heteroaryl, hydroxy, protected hydroxy or together with R₆ₐ forms an oxo;
R₆ₐ is hydrogen, optionally substituted alkyl, optionally substituted alkenyl, optionally substituted alkynyl, optionally substituted aryl, or optionally substituted heteroaryl, hydroxy, protected hydroxy or together with R₆ forms an oxo;
R₇ is hydrogen or together with R₇ₐ forms an oxo;
R₇ₐ is hydrogen, halogen, protected hydroxy, -OR₂₈, or together with R₇ forms an oxo;
R₉ is hydrogen or together with R₉ₐ forms an oxo;
R₉ₐ is hydrogen, hydroxy, protected hydroxy, or together with R₉ forms an oxo;
R₁₀ is hydrogen or together with R₁₀ₐ forms an oxo;
R₁₀ₐ is hydrogen, hydroxy, protected hydroxy, or together with R₁₀ forms an oxo;
R₁₃ is hydroxy or protected hydroxy;
R₁₄ is hydrogen, optionally substituted alkyl, optionally substituted alkenyl, optionally substituted alkynyl, optionally substituted aryl, or optionally substituted heteroaryl;
R₁₄ₐ is hydrogen, optionally substituted alkyl, optionally substituted alkenyl, optionally substituted alkynyl, optionally substituted aryl, or optionally substituted heteroaryl, hydroxy, protected hydroxy or together with R₁ forms a carbonate;
R₂₈ is hydrogen, hydroxy protecting group or a functional group which increases the solubility of the taxane derivative; and
R₃₀ and R₃₁ are independently hydrogen, optionally substituted alkyl, optionally substituted alkenyl, optionally substituted alkynyl, optionally substituted monocyclic aryl or optionally substituted monocyclic heteroaryl.

5. A process according to any one of claims 1 to 4, wherein the derivative or analog of baccatin III or 10-desacetyl baccatin III is prepared from a C1 hydroxy C2 benzoate derivative of baccatin III or 10-desacetyl baccatin III, the C1 hydroxy C2 benzoate derivative is reduced with sodium bis (2-methoxyethoxy) aluminum hydride to form a 1,2-diol derivative, the 1,2-diol is reacted with Cl₂CO to form a 1,2-carbonate, and the 1,2-carbonate is reacted with a nucleophilic reagent to convert the C2 substituent to R₃₁COO- wherein R₃₁ is as defined in claim 1.

6. A process for the preparation of a derivative or analog of baccatin III or 10-desacetyl baccatin III from a 1,2 carbonate derivative of baccatin III or 10-desacetyl baccatin III, the process comprising reacting the 1,2 carbonate with a nucleophilic reagent to convert the C2 substituent to R₃₁COO- wherein R₃₁ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl and the 1,2 carbonate is a compound having the formula 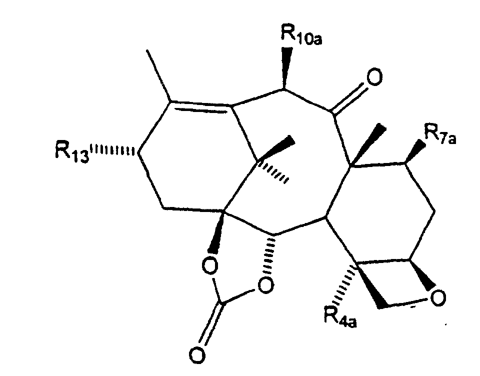 wherein
R₄ₐ is -OH or R₃₀COO- ;
R₇ₐ is protected hydroxy;
R₁₀ₐ is H, -OH, protected hydroxy, or -OCOR₂₉;
R₁₃ is -OH or protected hydroxy;
R₂₉ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl; and
R₃₀ is H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

7. A compound having the formula 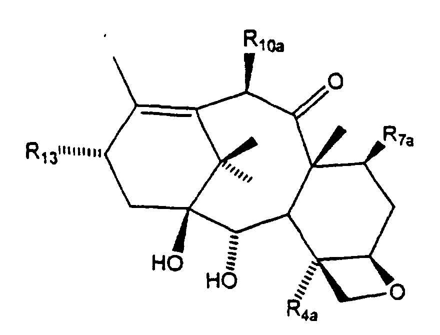 wherein
R₁₀ₐ is H, -OH, protected hydroxy, or -OCOR₂₉;
R₁₃ is -OH or protected hydroxy;
R₄ₐ is R₃₀COO-;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl with the exception that, when R₁₀ₐ and R₁₃ both represent -OH and R₄ₐ is acetoxy, then R₇ₐ is not triethylsilyloxy.

8. A compound having the formula 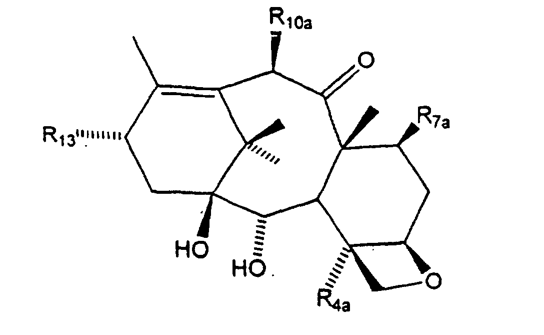 wherein
R₁₀ₐ is H, -OH, protected hydroxy, or -OCOR₂₉;
R₁₃ is -OH or protected hydroxy;
R₄ₐ is -OH or R₃₀COO- ;
R₇ₐ is H; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

9. A compound having the formula 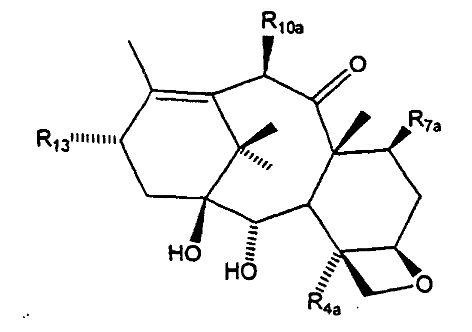 wherein
R₁₀ₐ is H or -OCOR₂₉;
R₁₃ is -OH or protected hydroxy;
R₄ₐ is -OH or R₃₀COO- ;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

10. A compound having the formula 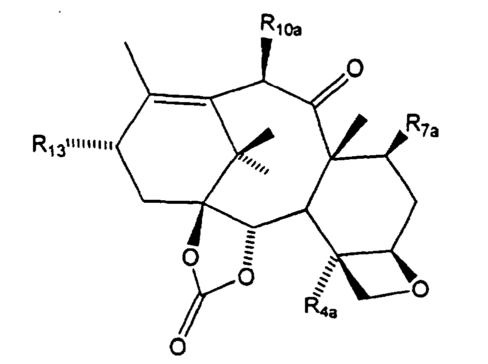 wherein
R₁₀ₐ is H, -OH, protected hydroxy, or -OCOR₂₉;
R₁₃ is -OH or protected hydroxy;
R₄ₐ is -OH or R₃₀COO-;
R₇ₐ is H, -OH or protected hydroxy; and
R₂₉ and R₃₀ are independently H, optionally substituted C₁-C₆ alkyl, optionally substituted C₂-C₆ alkenyl, optionally substituted C₂-C₆ alkynyl, optionally substituted monocyclic aryl, or optionally substituted monocyclic heteroaryl.

## Patentansprüche

1. Verfahren zur Herstellung eines Derivats oder Analogons von Baccatin III oder 10-Desacetyl-Baccatin III mit einem anderen C2-SubstituentenalsBenzoatund/odereinemanderenC4-Substituenten als Acetat aus einem Derivat oder Analogon von Baccatin III oder 10-Desacetyl-BaccatinIII,daseinenC2-Benzoat-und/oder C4-Acetat-Substituenten hat, bei dem man den C2-Benzoat-Substituenten und/oderdenC4-Acetat-Substituenten in einem nichtwässrigen System mit einem Aluminat selektiv zu der bzw. den entsprechenden Hydroxygruppe(n) reduziert und den C2-Hydroxy-Substituenten und/oder den C4-Hydroxy-Substituenten acetyliert, um den C2-Hydroxy-Substituenten zu R₃₁COO- und/oder den C4-Hydroxy-Substituenten zu R₃₀COO- umzusetzen, worin R₃₁ und R₃₀ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind.

2. Verfahren nach Anspruch 1, bei dem das Derivat oder Analogon des Baccatin III oder 10-Desacetyl-Derivats die Formel 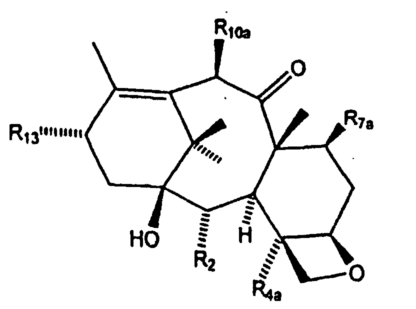 hat, worin
R₁₀ₐ Wasserstoff, -OH, geschütztes Hydroxy oder -OCOR₂₉ₐ ist,
R₁₃ -OH oder geschütztes Hydroxy ist,
R₇ₐ Wasserstoff, -OH oder geschütztes Hydroxy ist,
R₂ -OCOR₃₁ ist,
R₄ₐ -OCOR₃₀ ist, und
R₂₉ₐ, R₃₀ und R₃₁ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind.

3. Verfahren nach Anspruch 1 oder Anspruch 2, bei dem der C2-Bezoat-Substituent und/oder der C4-Acetat-Substituent des Derivats von Baccatin III oder 10-Desacetyl-Baccatin III selektiv mit Lithiumaluminiumhydrid oder Natrium-bis(2-methoxyethoxy)-aluminiumhydrid reduziert wird bzw. werden.

4. Verfahren nach einem der Ansprüche 1 bis 3, bei dem das Derivat oder Analogon des Baccatin III oder das 10-Desacetyl-Derivat die Formel 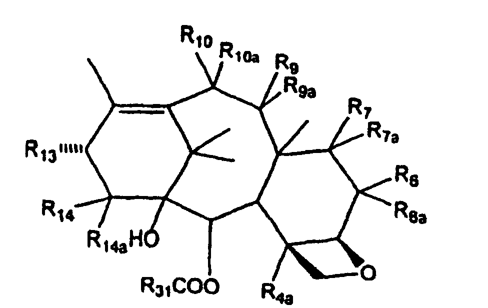 hat, worin
R₄ₐ Wasserstoff, wahlweise substituiertes Alkyl, wahlweise substituiertes Alkenyl, wahlweise substituiertes Alkynyl, wahlweise substituiertes Aryl, wahlweise substituiertes Heteroaryl, Cyano, Hydroxy oder -OCOR₁₀ ist,
R₆ Wasserstoff, wahlweise substituiertes Alkyl, wahlweise substituiertes Alkenyl, wahlweise substituiertes Alkynyl, wahlweise substituiertes Aryl oder wahlweise substituiertes Heteroaryl, Hydroxy, geschütztes Hydroxy ist oder zusammen mit R₆ₐ ein Oxo bildet,
R₆ₐ Wasserstoff, wahlweise substituiertes Alkyl, wahlweise substituiertes Alkenyl, wahlweise substituiertes Alkynyl, wahlweise substituiertes Aryl oder wahlweise substituiertes Heteroaryl, Hydroxy oder geschütztes Hydroxy ist oder zusammen mit mit R₆ ein Oxo bildet,
R₇ Wasserstoff ist oder zusammen mit R₇ₐ ein Oxo bildet,
R₇ₐ Wasserstoff, Halogen, geschütztes Hydroxy, -OR₂₈ ist oder zusammen mit R₇ ein Oxo bildet,
R₉ Wasserstoff ist oder zusammen mit R₉ₐ ein Oxo bildet,
R₉ₐ Wasserstoff, Hydroxy oder geschütztes Hydroxy ist oder zusammen mit R₉ ein Oxo bildet,
R₁₀ Wasserstoff ist oder zusammen mit R₁₀ₐ ein Oxo bildet,
R₁₀ₐ Wasserstoff, Hydroxy oder geschütztes Hydroxy ist oder zusammen mit R₁₀ ein Oxo bildet,
R₁₃ Hydroxy oder geschütztes Hydroxy ist,
R₁₄ Wasserstoff, wahlweise substituiertes Alkyl, wahlweise substituiertes Alkenyl, wahlweise substituiertes Alkynyl, wahlweise substituiertes Aryl oder wahlweise substituiertes Heteroaryl ist,
R₁₄ₐ Wasserstoff, wahlweise substituiertes Alkyl, wahlweise substituiertes Alkenyl, wahlweise substituiertes Alkynyl, wahlweise substituiertes Aryl oder wahlweise substituiertes Heteroaryl, Hydroxy, geschütztes Hydroxy ist oder zusammen mit R₁ ein Carbonat bildet,
R₂₈ Wasserstoff, eine Hydroxy-Schutzgruppe oder eine funktionelle Gruppe ist, die die Löslichkeit des Taxan-Derivats erhöht, und
R₃₀ und R₃₁ unabhängig Wasserstoff, wahlweise substituiertes Alkyl, wahlweise substituiertes Alkenyl, wahlweise substituiertes Alkynyl, wahlweise substituiertes monocyclisches Aryl oder wahlweise substituiertes monocyclisches Heteroaryl sind.

5. Verfahren nach einem der Ansprüche 1 bis 4, bei dem das Derivat oder Analogon von Baccatin III oder 10-Desacetyl-Baccatin III aus einem C1-Hydroxy-C2-Bezoat-Derivat des Baccatin III oder 10-Desacetyl-Baccatin III hergestellt wird, das C1-Hydroxy-C2-Bezoat-Derivat mit Natrium-bis(2-methoxyethoxy)-aluminiumhydrid zu einem 1,2-Diol-Derivat reduziert wird, das 1,2-Diol mit Cl₂CO zu einem 1,2-Carbonat umgesetzt wird und das 1,2-Carbonat mit einem nucleophilen Reagenz zur Reaktion gebracht wird, um den C2-Substituenten zu R₃₁COO- umzusetzen, worin R₃₁ wie in Anspruch 1 definiert ist.

6. Verfahren zur Herstellung eines Derivats oder Analogons von Baccatin III oder 10-Desacetyl-Baccatin III aus einem 1,2-Carbonat-Derivat des Baccatin III oder 10-Desacetyl-Baccatin III, bei dem man das 1,2-Carbonat mit einem nukleophilen Reagenz zur Reaktion bringt, um den C2-Substituenten zu R₃₁ COO- umzusetzen, worin R₃₁ Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes moncyclisches Aryl oder wahlweise substituiertes monocyclisches Heteroaryl ist und das 1,2-Carbonat eine Verbindung mit der Formel 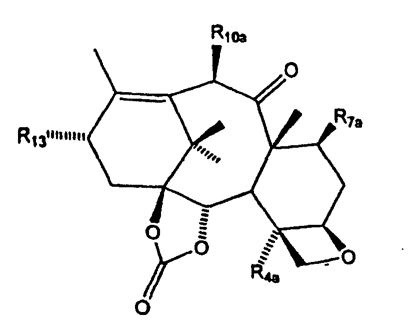 ist, worin
R₄ₐ -OH oder R₃₀COO- ist,
R₇ₐ geschütztes Hydroxy ist,
R₁₀ₐ Wasserstoff, -OH, geschütztes Hydroxy oder -OCOR₂₉ ist,
R₁₃ -OH oder geschütztes Hydroxy ist,
R₂₉ Wasserstoff, wahlweise substituiertes C₁-C₆ Alkyl, wahlweise substituiertes C₂-C₆-Akenyl, wahlweise substituiertes C₂-C₆-Alkynyl, wahlweise substituiertes monocyclisches Aryl oder wahlweise substituiertes monocyclisches Heteroaryl ist, und
R₃₀ Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl ist.

7. Verbindung mit der Formel 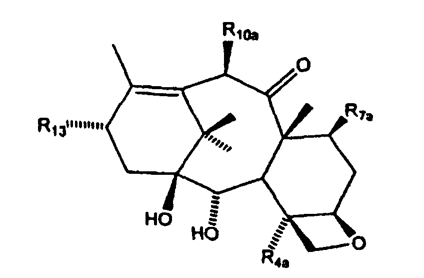 worin
R₁₀ₐ Wasserstoff, -OH, geschütztes Hydroxy oder -OCOR₂₉ ist,
R₁₃ -OH oder geschütztes Hydroxy ist,
R₄ₐ R₃₀COO- ist,
R₇ₐ Wasserstoff, -OH oder geschütztes Hydroxy ist, und
R₂₉ und R₃₀ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind mit der Ausnahme, daß R₇ₐ nicht Triethylsilyloxy ist, wenn R₁₀ₐ und R₁₃ beide -OH bedeuten und R₄ₐ Acetoxy ist.

8. Verbindung mit der Formel 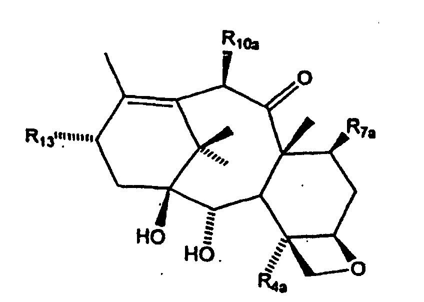 worin
R₁₀ₐ Wasserstoff, -OH, geschütztes Hydroxy oder -OCOR₂₉ ist,
R₁₃ -OH oder geschütztes Hydroxy ist,
R₄ₐ -OH oder R₃₀COO- ist,
R₇ₐ Wasserstoff ist, und
R₂₉ und R₃₀ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind.

9. Verbindung mit der Formel 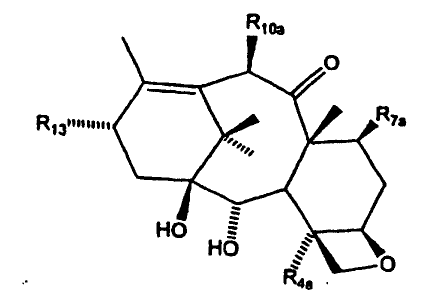 worin
R₁₀ₐ Wasserstoff oder -OCOR₂₉ ist,
R₁₃ -OH oder geschütztes Hydroxy ist,
R₄ₐ -OH oder R₃₀COO- ist,
R₇ₐ Wasserstoff, -OH oder geschütztes Hydroxy ist, und
R₂₉ und R₃₀ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind.

10. Verbindung mit der Formel 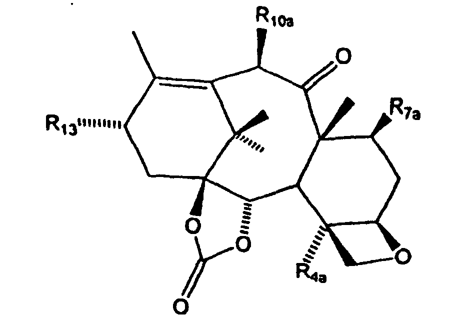 worin
R₁₀ₐ Wasserstoff, -OH, geschütztes Hydroxy oder -OCOR₂₉ ist,
R₁₃ -OH oder geschütztes Hydroxy ist,
R₄ₐ -OH oder R₃₀COO- ist,
R₇ₐ Wasserstoff, -OH oder geschütztes Hydroxy ist, und
R₂₉ und R₃₀ unabhängig Wasserstoff, wahlweise substituiertes C₁-C₆-Alkyl, wahlweise substituiertes C₂-C₆-Alkenyl, wahlweise substituiertes C₂-C₆-Alkynyl, wahlweise substituiertes monocyclisches Aryl oder wahlweise substituiertes monocyclisches Heteroaryl sind.

## Revendications

1. Procédé pour la préparation d'un dérivé ou d'un analogue de la baccatine III ou de la 10-désacétylbaccatine III ayant un substituant en C2 autre que le benzoate et/ou un substituant en C4 autre que l'acétate à partir d'un dérivé ou d'un analogue de la baccatine III ou de la 10-désacétylbaccatine III qui comprend un substituant benzoate en C2 et/ou un substituant acétate en C4, le procédé comprenant une réduction sélective du substituant benzoate en C2 et/ou du substituant acétate en C4 avec un aluminate en groupe(s) hydroxy correspondant(s) dans un système non aqueux et une acylation du substituant hydroxy en C2 et/ou du substituant hydroxy en C4 pour convertir le substituant hydroxy en C2 en R₃₁COO- et/ou convertir le substituant hydroxy en C4 en R₃₀COO- où R₃₁ et R₃₀ sont indépendamment l'un de l'autre H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.

2. Procédé selon la revendication 1, dans lequel le dérivé ou l'analogue de la baccatine III ou du dérivé 10-désacétyle a la formule 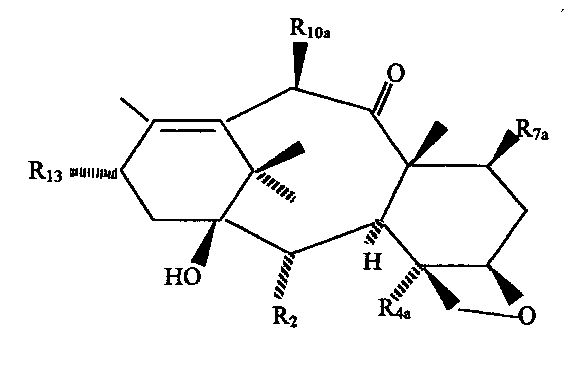 dans laquelle
R₁₀ₐ est H, -OH, un hydroxy protégé, ou -OCOR₂₉ₐ;
R₁₃ est -OH ou un hydroxy protégé ;
R₇ₐ est H, -OH ou un hydroxy protégé ;
R₂ est -OCOR₃₁;
R₄ₐ est -OCOR₃₀; et
R₂₉ₐ, R₃₀ et R₃₁ sont indépendamment les uns des autres H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.

3. Procédé selon la revendication 1 ou la revendication 2, dans lequel le substituant benzoate en C2 et/ou le substituant acétate en C4 du dérivé de la baccatine III ou de la 10-désacétylbaccatine III est ou sont réduit(s) sélectivement avec de l'hydrure d'aluminium et de lithium ou l'hydrure de sodium et de bis(2-méthoxyéthoxy) aluminium.

4. Procédé selon l'une quelconque des revendications 1 à 3, dans lequel le dérivé ou l'analogue de la baccatine III ou du dérivé 10-désacétyle a la formule 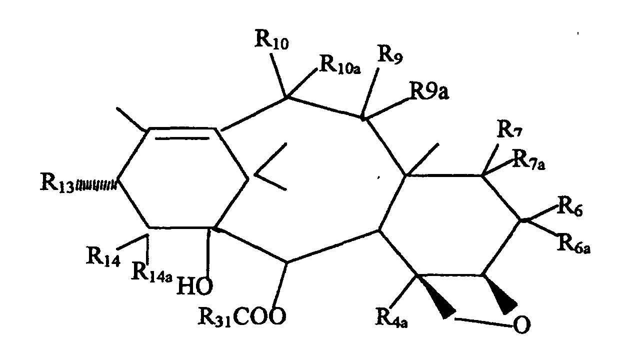 dans laquelle
R₄ₐ est un hydrogène, un alkyle éventuellement substitué, un alcényle éventuellement substitué, un alcynyle éventuellement substitué, un aryle éventuellement substitué, un hétéroaryle éventuellement substitué, un cyano, un hydroxy, ou -OCOR₃₀;
R₆ est un hydrogène, un alkyle éventuellement substitué, un alcényle éventuellement substitué, un alcynyle éventuellement substitué, un aryle éventuellement substitué, ou un hétéroaryle éventuellement substitué, un hydroxy, un hydroxy protégé ou forme un oxo avec R₆ₐ ;
R₆ₐ est un hydrogène, un alkyle éventuellement substitué, un alcényle éventuellement substitué, un alcynyle éventuellement substitué, un aryle éventuellement substitué, ou un hétéroaryle éventuellement substitué, un hydroxy, un hydroxy protégé ou forme un oxo avec R₆;
R₇ est un hydrogène ou forme un oxo avec R₇ₐ;
R₇ₐ est un hydrogène, un halogène, un hydroxy protégé, -OR₂₈, ou forme un oxo avec R₇ ;
R₉ est un hydrogène ou forme un oxo avec R₉ₐ ;
R₉ₐ est un hydrogène, un hydroxy, un hydroxy protégé ou forme un oxo avec R₉ ;
R₁₀ est un hydrogène ou forme un oxo avec R₁₀ₐ ;
R₁₀ₐ est un hydrogène, un hydroxy, un hydroxy protégé ou forme un oxo avec R₁₀ ;
R₁₃ est un hydroxy ou un hydroxy protégé ;
R₁₄ est un hydrogène, un alkyle éventuellement substitué, un alcényle éventuellement substitué, un alcynyle éventuellement substitué, un aryle éventuellement substitué, ou un hétéroaryle éventuellement substitué ;
R₁₄ₐ est un hydrogène, un alkyle éventuellement substitué, un alcényle éventuellement substitué, un alcynyle éventuellement substitué, un aryle éventuellement substitué, ou un hétéroaryle éventuellement substitué, un hydroxy, un hydroxy protégé ou forme un carbonate avec R₁ ;
R₂₈ est un hydrogène, un groupe protégeant les hydroxy ou un groupe fonctionnel qui augmente la solubilité du dérivé taxane ; et
R₃₀ et R₃₁ sont indépendamment l'un de l'autre un hydrogène, un alkyle éventuellement substitué, un alcényle éventuellement substitué, un alcynyle éventuellement substitué, un aryle monocyclique éventuellement substitué, ou un hétéroaryle monocyclique éventuellement substitué.

5. Procédé selon l'une quelconque des revendications 1 à 4, dans lequel le dérivé ou l'analogue de la baccatine III ou de la 10-désacétylbaccatine III est préparé à partir d'un dérivé hydroxy en C1 et benzoate en C2 de la baccatine III ou de la 10-désacétylbaccatine III, le dérivé hydroxy en C1 et benzoate en C2 est réduit avec de l'hydrure de sodium et de bis(2-méthoxyéthoxy)aluminium pour former un dérivé 1,2-diol, on fait réagir le 1,2-diol avec Cl₂CO pour former un 1,2-carbonate, et on fait réagir le 1,2-carbonate avec un réactif nucléophile pour convertir le substituant en C2 en R₃₁COO- dans lequel R₃₁ est défini comme dans la revendication 1.

6. Procédé pour la préparation d'un dérivé ou d'un analogue de la baccatine III ou de la 10-désacétylbaccatine III à partir d'un dérivé 1,2 carbonate de la baccatine III ou de la 10-désacétyl baccatine III, le procédé comprenant la réaction du 1,2 carbonate avec un réactif nucléophile pour convertir le substituant en C2 en R₃₁COO- dans lequel R₃₁ est H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué et le 1,2 carbonate est un composé ayant la formule 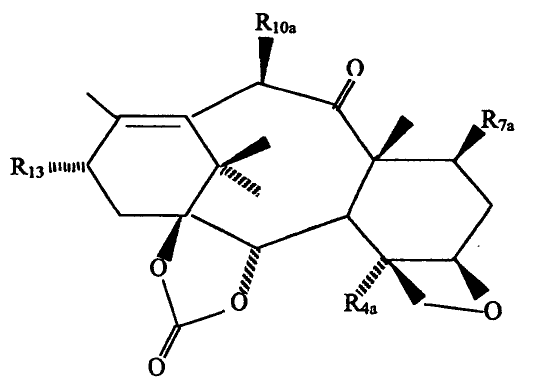 dans laquelle
R₄ₐ est un -OH ou R₃₀COO- ;
R₇ₐ est un hydroxy protégé ;
R₁₀ₐ est H, -OH, un hydroxy protégé, ou -OCOR₂₉;
R₁₃ est -OH ou un hydroxy protégé ;
R₂₉ est H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué ; et
R₃₀ est H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.

7. Composé ayant la formule 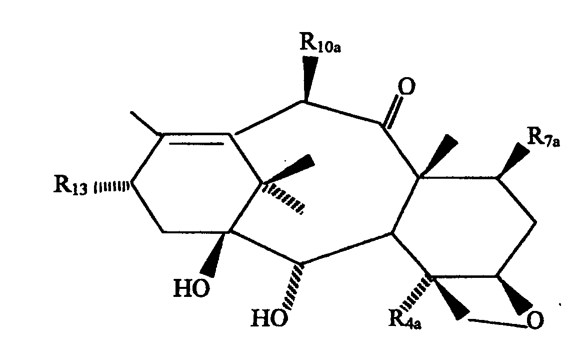 dans laquelle
R₁₀ₐ est H, -OH, un hydroxy protégé, ou -OCOR₂₉ ;
R₁₃ est -OH ou un hydroxy protégé ;
R₄ₐ est R₃₀COO-;
R₇ₐ est H, -OH ou un hydroxy protégé ; et
R₂₉ et R₃₀ sont indépendamment l'un de l'autre H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué sauf que, si R₁₀ₐ et R₁₃ représentent tous deux -OH et que R₁₄ est un acétoxy, alors R₇ₐ n'est pas un triéthylsilyloxy.

8. Composé ayant la formule 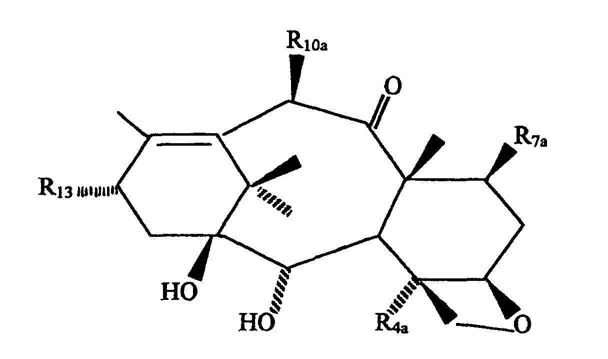 dans laquelle
R₁₀ₐ est H, -OH, un hydroxy protégé, ou -OCOR₂₉;
R₁₃ est -OH ou un hydroxy protégé ;
R₄ₐ est -OH ou R₃₀COO- ;
R₇ₐ est H ; et
R₂₉ et R₃₀ sont indépendamment l'un de l'autre H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.

9. Composé ayant la formule 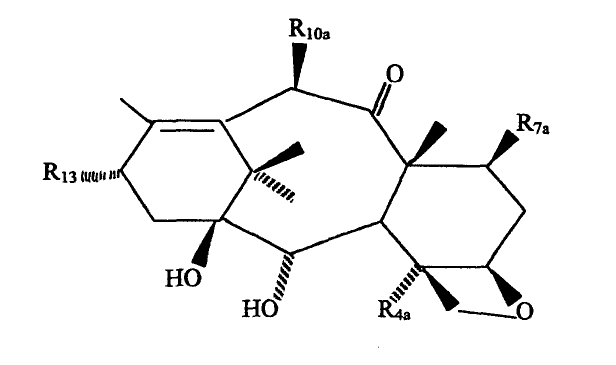 dans laquelle
R₁₀ₐ est H, ou -OCOR₂₉ ;
R₁₃ est -OH ou un hydroxy protégé ;
R₄ₐ est -OH ou R₃₀COO- ;
R₇ₐ est H, -OH ou un hydroxy protégé ; et
R₂₉ et R₃₀ sont indépendamment l'un de l'autre H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.

10. Composé ayant la formule 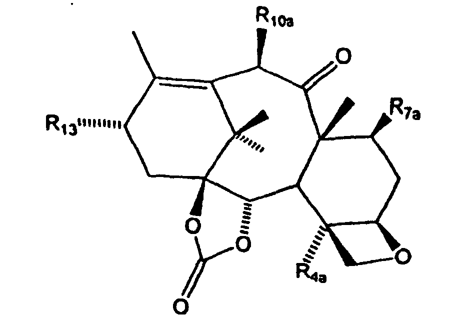 Dans laquelle
R₁₀ₐ est H, -OH, un hydroxy protégé, ou -OCOR₂₉ ;
R₁₃ est -OH ou un hydroxy protégé ;
R₄ₐ est -OH ou R₃₀COO- ;
R₇ₐ est H, -OH ou un hydroxy protégé ; et
R₂₉ et R₃₀ sont indépendamment l'un de l'autre H, un alkyle en C₁-C₆ éventuellement substitué, un alcényle en C₂-C₆ éventuellement substitué, un alcynyle en C₂-C₆ éventuellement substitué, un aryle monocyclique éventuellement substitué, ou un hétéroaryle monocyclique éventuellement substitué.
